# EUROPÄISCHE PATENTSCHRIFT

(11) **EP 3 964 166 B1**
(45) Veröffentlichungstag und Bekanntmachung des Hinweises auf die Patenterteilung: **18.03.2026**
(21) Anmeldenummer: 20194459.2
(22) Anmeldetag: 03.09.2020
(51) Int. Cl.: A61C 15/02, A46B 5/00, A46B 15/00, A61C 15/04, A61B 17/24

(54) **MUNDHYGIENEMITTEL UND HERSTELLUNGSVERFAHREN DAFÜR**
ORAL HYGIENE PRODUCT AND MANUFACTURING PROCESS THEREFOR
PRODUIT D'HYGIÈNE BUCCALE ET LEUR PROCÉDÉ DE FABRICATION

(43) Veröffentlichungstag der Anmeldung: 09.03.2022
(73) Patentinhaber: Trisa Holding AG, 6234 Triengen (CH)
(72) Erfinder: Fischer, Herbert, 5057 Reitnau (CH)
(74) Vertreter: Daub, Thomas

(56) Entgegenhaltungen:
- CN-C- 1 129 403
- CN-U- 207 011 911
- JP-A- 2018 143 644
- US-A- 3 609 789
- US-B1- 6 336 242

## Beschreibung

### Stand der Technik

Die Erfindung betrifft ein Mundhygienemittel.

Es ist bereits ein Mundhygienemittel wie eine Interdentalbürste, ein Flosser oder eine Zahnbürste oder anderes Mundhygienemittel mit zumindest einer Anwendungseinheit und mit zumindest einer mit der Anwendungseinheit verbundenen Griffeinheit, welche zumindest einen materiellen Griffkörper aufweist, vorgeschlagen worden.

Aus der DE 20 2020 000 613 und der WO 2014/169398 A1 sind bereits Mundhygienemittel bekannt.

Ferner sind aus der US 6 336 242 B1, US 3 609 789 A, CN 207 011 911 U, CN 1 129 403 C und JP 2018 143644 A bereits Mundhygienemittel mit einer Anwendungseinheit und mit einer mit der Anwendungseinheit verbundenen Griffeinheit bekannt, welche zumindest einen materiellen Griffkörper aufweist, wobei der materielle Griffkörper der Griffeinheit oder die Anwendungseinheit zumindest teilweise aus einem Papierwerkstoff besteht.

Die Aufgabe der Erfindung besteht insbesondere darin, eine gattungsgemäße Vorrichtung mit verbesserten Eigenschaften hinsichtlich einer Herstellbarkeit, eines Komforts sowie hinsichtlich der Ökologie bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung geht aus von einem Mundhygienemittel, wie eine Interdentalbürste, einem Flosser oder einer Zahnbürste, oder anderes Mundhygienemittel mit zumindest einer Anwendungseinheit und mit zumindest einer mit der Anwendungseinheit verbundenen Griffeinheit, welche zumindest einen materiellen Griffkörper aufweist, wobei der materielle Griffkörper der Griffeinheit oder der materielle Griffkörper der Griffeinheit und die Anwendungseinheit zumindest teilweise, insbesondere zumindest zu einem Großteil, aus einem Papierwerkstoff besteht.

Vorzugsweise besteht der Papierwerkstoff insbesondere aus Fasern, wobei insbesondere verschiedene Verarbeitungsmöglichkeiten bestehen. Der Papierwerkstoff kann zudem behandelt sein. Der Papierwerkstoff kann beschichtet sein, wie insbesondere lackiert, beispielsweise mit einem Lack zu einer Verbesserung der Verbindung beim Verbinden, und/oder getränkt. Der Papierwerkstoff kann insbesondere sowohl in flacher Form, in Block-Form oder in flüssiger bis Pappe-förmiger Form und/oder Konsistenz vorliegen, wobei ausgehend von der Form verschiedene Verarbeitungen möglich sind. In flacher Form kann der Papierwerkstoff gerollt, geschichtet, gepresst, laminiert, gerillt, geprägt, gefaltet, gestanzt und/oder grainiert, also insbesondere aufgeraut oder gekörnt, werden. Zu einem Verbinden kann der Papierwerkstoff geklebt, wobei allenfalls Vorbereitungsschritte wie Grainieren oder dergleichen nötig sind, verschweißt werden, wie insbesondere mittels Ultraschall-Schweißen, Siegeln (heiß oder kalt) oder Laminieren, etc. Zu einem Verbinden können auf dem Papierwerkstoff Spezialschichten nötig sein, um die Verbindung zu schaffen. Verbindungen können mit einem Papierwerkstoff oder anderen in dieser Schrift genannten Materialien hergestellt werden. In Block-Form kann der Papierwerkstoff mechanisch bearbeitet, beispielsweise gefräst werden. In flüssiger Form kann der Papierwerkstoff gegossen oder spritzgegossen werden, wie insbesondere mittels Papierspritzguss oder Papierguss. Hierbei wird der Papierwerkstoff insbesondere analog zu einem Spitzgussprozess in einer Kavität geformt. Anschließend wird das gegossene Teil ausgehärtet bzw. getrocknet. Beim Papierspritzguss ist insbesondere auch ein Anspritzen an andere Teile bzw. Materialien, beispielsweise Kunststoff mit Papier umspritzt, denkbar. Alternativ kann ein Kunststoffteil auch an einem Papierwerkstoff angespritzt werden. Insgesamt kann der Produktaufbau des Körperpflegeprodukts verschiedener Art sein, wie insbesondere vollständig aus einem Papierwerkstoff oder in einer Hybridtechnik, beispielsweise aus Papierwerkstoff mit Kunststoff, insbesondere beispielsweise mit einem Wechselkopf und/oder mit einer Griffeinheit aus Papier und einer Anwendungseinheit aus Kunststoff. Weitere Materialkombinationen mit Papier können wie erwähnt Kunststoff und/oder Metall und/oder organische Materialien wie Holz, Bambus und/oder anorganische Materialien wie Stein, Glas etc. sein.

Durch die erfindungsgemäße Ausgestaltung des Körperpflegeprodukts können vorteilhafte Eigenschaften hinsichtlich einer Ergonomie sowie einer Herstellbarkeit des Körperpflegeprodukts bereitgestellt werden. Es kann insbesondere ein vorteilhaft ökologisches Körperpflegeprodukt bereitgestellt werden. Es kann insbesondere eine vorteilhaft einfache Entsorgung des Körperpflegeprodukts erreicht werden.

Das Körperpflegeprodukt ist von einem Mundhygienemittel gebildet. Unter einem "Mundhygienemittel" soll insbesondere eine Zahnbürste und/oder ein Interdentalreiniger, insbesondere eine Interdentalbürste, und/oder ein Flosser und/oder ein Zungenreiniger und/oder ein Zahnstocher verstanden werden. Vorteilhaft ist das Mundhygienemittel als eine Zahnbürste, insbesondere eine Handzahnbürste, als eine Interdentalbürste und/oder als ein Flosser ausgebildet. Alternativ kann das Mundhygienemittel von einer elektrischen Zahnbürste gebildet sein. Das Mundhygienemittel kann hierbei bei einer Ausbildung als Zahnbürste insbesondere eine Einwegzahnbürste, eine Mehrwegzahnbürste oder auch eine Wechselkopfzahnbürste sein. Das Mundhygienemittel könnte jedoch auch allgemein von einem Bürstenprodukt gebildet sein. Unter einem "Körperpflegeprodukt" soll insbesondere eine Mundhygienebürste und/oder eine Kosmetikbürste und/oder eine Haarbürste und/oder eine Haushaltsbürste verstanden werden. Als Mundhygienebürsten sind beispielsweise manuelle Zahnbürsten wie Mehrwegzahnbürsten, Wechselkopfzahnbürsten, Einwegzahnbürsten oder Single-Tuft-Bürsten, elektrische Zahnbürsten, wie auch Hybridzahnbürsten, Interdentalreiniger, insbesondere mit eingedrehten Borsten, in gespritzter Form oder als Flosser, Zungenreiniger und/oder Zahnseide denkbar. Als Körperpflegprodukte insbesondere Kosmetikbürsten sind beispielsweise Mascarabürsten, Nagellackpinsel, Gesichtsbürsten, Applikatoren, insbesondere auch Haarfärbe-Applikatoren, Massagegeräte, Make-up-Pinsel, Rasierpinsel und/oder Nassrasierer oder andere Körperpflegeprodukte denkbar. Als Haushaltsbürsten sind beispielsweise Abwaschbürsten, Bodenwischer und/oder Besen denkbar.

Bei einer Ausbildung als Zahnbürste ist insbesondere denkbar, dass die Anwendungseinheit aus einem Grundkörper und einem Borstenplättchen, welches mit Borsten und/oder alternativen Reinigungselementen besetzt ist, zusammengesetzt ist. Vorzugsweise besteht das Körperpflegeprodukt aus einer Anwendungseinheit mit Borsten und der Griffeinheit, wobei die Anwendungseinheit insbesondere einen Halsteil aufweist, der die Anwendungseinheit mit der Griffeinheit verbindet. Alle Teilelemente können aus mindestens einer Hart- und/oder einer oder mehreren Weichkomponente/-n bestehen.

Das Körperpflegeprodukt weist insbesondere eine Längsachse auf, die vorteilhaft zumindest im Wesentlichen parallel zu einer Haupterstreckungsrichtung des Körperpflegeprodukts angeordnet ist. Bevorzugt verläuft die Längsachse zumindest abschnittsweise innerhalb des Körperpflegeprodukts und insbesondere durch dessen Schwerpunkt. Insbesondere ist die Längsachse des Körperpflegeprodukts eine Zentralachse des Körperpflegeprodukts und/oder eine Zentralachse der Griffeinheit. Unter einer "Zentralachse" eines Objekts soll dabei insbesondere eine gedachte Achse verstanden werden, die innerhalb des Objekts parallel zu einer Haupterstreckungsrichtung des Objekts verläuft und das Objekt an höchstens zwei Punkten schneidet. Unter "zumindest im Wesentlichen parallel" soll hier insbesondere eine Ausrichtung einer Richtung relativ zu einer Bezugsrichtung, insbesondere in einer Ebene, verstanden werden, wobei die Richtung gegenüber der Bezugsrichtung eine Abweichung insbesondere kleiner als 8°, vorteilhaft kleiner als 5° und besonders vorteilhaft kleiner als 2° aufweist. Unter einer "Haupterstreckungsrichtung" eines Objekts soll dabei insbesondere eine Richtung verstanden werden, welche parallel zu einer längsten Kante eines kleinsten gedachten Quaders verläuft, welcher das Objekt gerade noch vollständig umschließt. Unter einer "Haupterstreckung" eines Objekts soll in diesem Zusammenhang insbesondere eine Erstreckung einer längsten Kante eines kleinsten gedachten Quaders, welcher das Objekt gerade noch vollständig umschließt, verstanden werden.

Vorteilhaft weist die Anwendungseinheit zumindest einen Reinigungsbereich auf, der zu einer Zahnreinigungsanwendung, insbesondere in einem Mundraum des Benutzers, vorgesehen ist. Vorzugsweise umfasst der Reinigungsbereich zumindest eine Reinigungseinheit. Die Reinigungseinheit kann einen Bürstenkopf, vorteilhaft einen Zahnbürstenkopf, bevorzugt mit mehreren Borsten und/oder Borstenbündeln und/oder gespritzten Reinigungselementen bzw. gespritzten Borsten und/oder weichelastischen Reinigungselementen, umfassen. Alternativ oder zusätzlich kann die Reinigungseinheit auch als eine Interdentalbürste und/oder als ein Single Tuft (z.B. einzelnes großes Borstenbündel) und/oder als ein mit Zahnseide und/oder mit einem Zahnzwischenraumband, bevorzugt einem Zahnzwischenraumtape, bespannter Bogen, insbesondere als ein Flosser, oder dergleichen ausgebildet sein. Ferner weist die Anwendungseinheit vorteilhaft zumindest das Hals- und/oder Bügelelement auf, welches bevorzugt mit dem Reinigungsbereich, insbesondere unmittelbar und/oder einstückig, verbunden ist. Unter "einstückig" soll insbesondere zumindest stoffschlüssig verbunden, beispielsweise durch einen Verbindungsprozess wie Schweißprozess, einen Klebeprozess, einen Anspritzprozess und/oder einen anderen, dem Fachmann als sinnvoll erscheinenden Prozess, und/oder vorteilhaft in einem Stück geformt verstanden werden, wie beispielsweise durch eine Herstellung aus einem Guss und/oder durch eine Herstellung in einem Ein- oder Mehrkomponentenspritzverfahren und vorteilhaft aus einem einzelnen Rohling. Unter "vorgesehen" soll insbesondere speziell ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Vorzugsweise weist die Griffeinheit zumindest ein Griffelement auf, das vorteilhaft zu einem Halten mit einer Hand vorgesehen ist. Es ist denkbar, dass das Griffelement zumindest bereichsweise tailliert und/oder mit einem konkaven Oberflächenbereich und/oder mit einem konvexen Oberflächenbereich ausgebildet ist. Dies erlaubt vorteilhaft einen sicheren Halt und optimiert die Ergonomie. Besonders bevorzugt ist das Griffelement länglich ausgebildet, wobei vorteilhaft eine Längsachse des Griffelements der Längsachse des Körperpflegeprodukts entspricht. Das Griffelement ist zumindest teilweise, insbesondere vollständig, aus mindestens einem Papierwerkstoff ausgebildet. Alternativ oder zusätzlich kann das Griffelement und/oder die Griffeinheit zumindest teilweise aus einer Hartkomponente und/oder aus einer oder mehreren Weichkomponenten ausgebildet sein. Vorzugsweise ist der materielle Griffkörper zumindest zu einem Großteil, insbesondere vollständig, aus mindestens einem Papierwerkstoff ausgebildet. Insbesondere umfasst das Griffelement vorteilhaft zumindest einen Daumengriffbereich und/oder zumindest einen Handgriffbereich. Vorteilhaft ist der Daumengriffbereich auf der Vorderseite des Körperpflegeprodukts und insbesondere auf einer Vorderseite des Griffelements angeordnet. Es ist denkbar, dass der Daumengriffbereich und/oder der Handgriffbereich zumindest ein Element und/oder eine Oberflächenstrukturierung aus mindestens einem Papierwerkstoff, aus mindestens einer Weichkomponente und/oder mindestens einer Hartkomponente aufweisen.

Die für die Herstellung eingesetzten Kunststoffe aus Hart- und/oder Weichkomponenten welche mittels Spritzguss verarbeitet werden, können insbesondere wie folgt eingeteilt werden:
- Konventionelle Materialien: Materialien, im Wesentlichen Neumaterialien, die größtenteils erdölbasiert sind.
- Nachhaltige Materialien: wie nachfolgend aufgezählt und später beschrieben vorzugsweise biobasiert, abbaubar und/oder recycliert.
   ∘ Biobasierte Materialien: Material, welches insbesondere zu mehr als 60 %, vorzugsweise zu mehr als 80 % und besonders bevorzugt zu 100 % aus nachwachsenden Rohstoffen hergestellt ist. Eine weitere mögliche zusätzliche Eigenschaft biobasierter Materialien ist insbesondere, dass die biobasierten Materialien biologisch abbaubar sind. Vorzugsweise basieren die Materialien nicht auf Nahrungsmitteln wie insbesondere Mais etc..
   ∘ Biologisch abbaubare Materialien: Material, das gemäß den gängigen Normen biologisch abbaubar ist. Hierzu zählen insbesondere die Kompostierbarkeit (industriell oder nicht industriell). Dabei können Materialien aus nachwachsenden Rohstoffen diese Eigenschaft tragen.
   ∘ Recyclierte Materialien: Materialien, die aus einem Recycling-Prozess stammen, wie beispielsweise Post Consumer Recycled Materialien, Ocean Waste Plastic oder Social Plastic.

Die eingesetzten Materialien können recyclierbare Materialien sein. Für recyclierbare Materialien besteht nach dem Gebrauch vorteilhaft eine Recyclingmöglichkeit.

Im Rahmen dieser Offenbarung kommen nahezu beliebige Hartkomponenten und Weichkomponenten infrage, die der Fachmann zweckgemäß geeignet kombinieren und/oder auswählen wird. Als Hartkomponente kommen beispielsweise Styrolpolymerisate wie Styrolacrylnitril (SAN), Polystyrol (PS), Acrylnitrylbutadienstryrol (ABS), Styrolmethylmethacrylate (SMMA), Styrolbutadien (SB) oder dergleichen infrage. Ferner kann eine Hartkomponente Polyolefine wie Polypropylen (PP), Polyethylen (PE) oder dergleichen umfassen, insbesondere auch in Form von High-Density-Polyethylen (HDPE) oder Low-Density-Polyethylen (LDPE). Zudem kommen Polyester wie beispielsweise Polyethylenterephthalat (PET), insbesondere in Form von säuremodifiziertem Polyethylenterephthalat (PETA) oder glykolmodifiziertem Polyethylenterephthalat (PETG), Polybutylenterephthalat (PBT), säuremodifiziertes Polycyclohexylendimethylenterephthalat (PCT-A), glykolmodifiziertes Polycyclohexylendimethylenterephthalat (PCT-G) oder dergleichen infrage. Weiterhin ist eine Verwendung von Cellulosederivaten wie beispielsweise Celluloseacetat (CA), Celluloseacetobutyrat (CAB), Cellulosepropionat (CP), Celluloseacetatphthalat (CAP), Cellulosebutyrat (CB) oder dergleichen denkbar. Ferner kann eine Hartkomponente beispielsweise Polyamide (PA) wie PA 6.6, PA 6.10, PA 6.12 oder dergleichen, Polymethylmethacrylat (PMMA), Polycarbonat (PC), Polyoxymethylen (POM), Polyvinylchlorid (PVC), Polyurethan (PUR), Polyamid (PA) oder dergleichen mehr umfassen. Insbesondere Polyethylen (PE) und/oder Polyurethan (PU) können als Hartkomponente und/oder als Weichkomponente eingesetzt werden. Insbesondere weist eine Hartkomponente ein Elastizitätsmodul von wenigstens 1000 N/mm² und vorteilhaft von wenigstens 1300 N/mm² und/oder von höchstens 2400 N/mm² und vorteilhaft von höchstens 1800 N/mm² auf. Als Hartkomponente wird bevorzugt Polypropylen (PP) eingesetzt. Mindestens gewisse unter der Hartkomponente genannte Materialien können nachhaltige Materialien sein. Insbesondere Materialien mit Celluloseanteil sind zumindest teilweise biobasiert.

Vorteilhaft werden Hartkomponenten für stabile und/oder strukturtragende Elemente, insbesondere in dem Griffelement und/oder in einem Trägerelement der Anwendungseinheit und/oder einer Befestigungseinheit oder dergleichen eingesetzt. Vorteilhaft ist denkbar, dass eine verwendete Hartkomponente und eine verwendete Weichkomponente unterschiedliche Farben aufweisen, sodass Oberflächenstrukturen, Beschriftungen, Motive und dergleichen mittels geeigneter Gestaltung von Grundkörper und Weichelement realisierbar sind.

Als Weichkomponenten kommen beispielsweise thermoplastische Styrol-Elastometer (TPE-S) wie etwa ein Styrol-Ethylen-Butylen-Styrol-Copolymer (SEBS), ein StyrolButadien-Styrol-Copolymer (SBS) oder dergleichen infrage. Zudem ist eine Verwendung thermoplastischer Polyurethan-Elastomere (TPE-U), thermoplastischer Polyamid-Elastomere (TPE-A), thermoplastischer Polyolefin-Elastomere (TPE-O), thermoplastischer Polyester-Elastomere (TPE-E) oder dergleichen denkbar. Weiterhin kann eine Weichkomponente beispielsweise zumindest ein Silikon umfassen. Vorteilhaft weist eine Weichkomponente eine Shore-A-Härte von höchstens 90, vorteilhaft von höchstens 50 und besonders vorteilhaft von höchstens 30 auf. Vorzugsweise bildet zumindest eine Weichkomponente mit zumindest einer Hartkomponente, insbesondere in zumindest einem Zwei- und/oder Mehrkomponentenspritzguss, vorteilhaft mittels zumindest eines Überspritzens und/oder Umspritzens, zumindest einen Materialschluss. Die unter der Weichkomponente genannten Materialien können nachhaltige Materialien sein.

Das Körperpflegeprodukt weist bei einer Ausbildung als Zahnbürste insbesondere eine Vorderseite und eine Rückseite auf, die insbesondere einander abgewandt angeordnet sind. Vorzugsweise ist der Reinigungsbereich der Anwendungseinheit auf der Vorderseite des Körperpflegeprodukts angeordnet. Die Vorderseite ist insbesondere eine in einer Betrachtungsrichtung senkrecht zu der Längsachse des Körperpflegeprodukts und senkrecht zu der Breitenachse des Körperpflegeprodukts sichtbare Seite des Körperpflegeprodukts. Als Vorderseite des Körperpflegeprodukts ist insbesondere jene Seite der Bürste bezeichnet, auf welcher der Daumen aufgelegt wird. Die Vorderseite ist normalerweise auch jene Seite, auf welche das Borstenfeld gerichtet ist. Die Rückseite entspricht vorteilhaft einer in einer hierzu entgegengesetzten Betrachtungsrichtung sichtbaren Seite des Körperpflegeprodukts. Als Rückseite des Körperpflegeprodukts wird die dem Borstenfeld entgegengesetzte Seite der Zahnbürste bezeichnet. Als linke Seite des Körperpflegeprodukts wird insbesondere eine Seite bezeichnet, welche links liegt, wenn man senkrecht auf die Vorderseite des Körperpflegeprodukts blickt. Als rechte Seite des Körperpflegeprodukts wird insbesondere eine Seite bezeichnet, welche rechts liegt, wenn man senkrecht auf die Vorderseite des Körperpflegeprodukts blickt. Als Oberseite wird insbesondere ein Ende des Körperpflegeprodukts bezeichnet, an welchem der Reinigungsbereich angeordnet ist. Als Unterseite wird insbesondere ein der Oberseite entgegengesetztes Ende des Körperpflegeprodukts bezeichnet, welches dem Griffbereich am nächsten liegt.

Ferner wird vorgeschlagen, dass die zumindest eine Anwendungseinheit, bei einer Ausbildung des Körperpflegeprodukts als Zahnbürste, einen Bürstenkopf, insbesondere Zahnbürstenkopf, aufweist. Die Anwendungseinheit weist vorteilhaft zumindest eine Reinigungseinheit, insbesondere einen Zahnbürstenkopf, mit Borsten auf. Die Reinigungseinheit weist zudem vorteilhaft zumindest einen Borstenträger, beispielsweise einen Bürstenkopfgrundkörper, auf. Zumindest einige oder alle der Borsten sind vorteilhaft konventionell extrudierte Borsten. Borsten können hierbei insbesondere zumindest eine Hartkomponente und/oder zumindest eine Weichkomponente umfassen. Vorzugsweise sind die Borsten zumindest teilweise oder vollständig aus Polyamid (PA) und/oder aus Polyester (PBT, PET) gefertigt, wobei beliebige andere Materialien denkbar sind und auch in dieser Schrift aufgeführte nachhaltige Materialien möglich sind. Ferner ist denkbar, dass zumindest einige der Borsten am nutzungsseitigen Ende eine Zuspitzung und/oder einen veränderlichen Querschnitt aufweisen. Vorzugsweise sind die Borsten aus einem einzelnen, insbesondere auch gemischten, Material ausgebildet. Es sind aber auch Borsten mit mehreren Komponenten denkbar, die insbesondere mittels zumindest einer Koextrusion herstellbar und/oder hergestellt sein können. Die Borsten können beispielsweise mittels Extrusion, Ablängen und/oder Nachbearbeitung herstellbar und/oder hergestellt sein. Im Gegensatz zu gespritzten Borsten oder gummielastischen Massage- und Reinigungselementen, welche mittels Spritzgusses hergestellt sind, werden konventionelle Borsten extrudiert, geschnitten, bearbeitet und am Zahnbürstengriff mittels angepassten Verfahrens eingesetzt, wie beispielsweise mittels des Ankerstanz-Verfahrens oder eines ankerlosen Verfahrens.

Insbesondere kommen zylindrische oder zugespitzte Borsten mit rundem Querschnitt infrage, wobei beliebige andere Querschnitte wie beispielsweise polygonale, dreieckige, rechteckige, quadratische, elliptische, sternförmige, trapezförmige, kreuzförmige, parallelogrammförmige, rhombusförmige oder beliebige andere Querschnitte denkbar sind. Insbesondere können unterschiedliche Borsten in einem Borstenbündel, aber auch unterschiedliche Borstenbündel, insbesondere jeweils mit einer bestimmten Art von Borsten, verwendet werden. Borsten und/oder Borstenbündel können hierbei regelmäßig, aber auch unregelmäßig, angeordnet sein. Insbesondere können sich in Gruppen und/oder benachbart angeordnete Borsten und/oder Borstenbündel hinsichtlich zumindest eines Merkmals wie beispielsweise einer Länge, eines Durchmessers, eines Materials, einer Farbe, einer Materialhärte, einer Geometrie, einer Anspitzung und dergleichen, insbesondere abwechselnd, unterscheiden. Vorzugsweise weisen die Borsten einen Durchmesser, insbesondere senkrecht zu deren Längsachse, von wenigstens 0,075 mm und/oder von höchstens 0,25 mm auf. Vorteilhaft weisen die Borsten eine Querschnittsfläche, insbesondere senkrecht zu deren Längsachse, von wenigstens 0,002 mm² und/oder von höchstens 0,2 mm² auf. Im Fall von Borsten, die im Kosmetikbereich eingesetzt werden, die nicht Teil der Erfindung sind, beispielsweise Borsten eines zusätzlicher Anwendungselements, können auch dünnere Borsten und/oder Borsten mit einem kleineren Querschnitt verwendet werden, insbesondere Borsten mit einem Durchmesser, insbesondere senkrecht zu deren Längsachse, von wenigstens 0,025 mm und/oder von höchstens 0,2 mm und/oder mit einer Querschnittsfläche, insbesondere senkrecht zu deren Längsachse, von wenigstens 0,001 mm² und/oder von höchstens 0,15 mm². Im Fall von zugespitzten Borsten ist insbesondere Polyester (z.B. PBT, PET) als Material geeignet, wobei auch nachhaltige Materialien möglich sind, wobei eine Zuspitzung mechanisch und/oder chemisch erzeugt sein kann. Andere Materialien sind jedoch ebenso denkbar. Vorzugsweise sind die Borsten in Längsrichtung gerade, es sind jedoch in Längsrichtung auch gewellte und/oder gedrillte und/oder wendelförmige und/oder gedrehte Borsten denkbar sowie insbesondere auch Kombinationen unterschiedlicher Borsten. Ferner sind Borsten mit einer glatten Oberfläche denkbar, ebenso wie Borsten mit texturierter Oberfläche.

Ferner sind die Borsten, insbesondere als Borstenbündel, vorzugsweise mittels zumindest eines Ankerstanz-Verfahrens oder eines ankerlosen Verfahrens oder dergleichen verarbeitet, insbesondere an dem Borstenträger befestigt. Vorzugsweise weist der Borstenträger eine Mehrzahl von, insbesondere gebohrten und/oder im Spritzgussverfahren geformten, Borstenaufnahmen, insbesondere Löchern für Borstenbündel, auf. Im Fall eines Ankerstanzens ist beispielsweise denkbar, dass zunächst ein Grundkörper, insbesondere aus einer Hartkomponente, vorzugsweise des Bürstenkopfs, mittels eines Spritzgießens gefertigt wird, wobei vorteilhaft Sacklöcher für Borstenbündel bei dem Spritzgießen geformt werden. Selbstverständlich ist jedoch auch ein anschließendes Bohren von Sacklöchern denkbar. Vorzugsweise werden anschließend Borsten beziehungsweise Borstenbündel gefaltet und mittels zumindest eines Ankers in jeweils einem Sackloch befestigt, insbesondere mittels eines Einstanzens. Ebenso ist ein Schlingenstanzen denkbar.

Alternativ sind, wie erwähnt, auch ankerlose Verfahren denkbar, wobei vorteilhaft Borsten beziehungsweise Borstenbündel nicht gefaltet werden. Borsten beziehungsweise Borstenbündel weisen in diesem Fall im Vergleich zu einem Ankerstanzen in etwa die halbe Länge auf. Beispielsweise ist hierbei denkbar, dass die Borstenbündel zunächst vereinzelt verschmolzen und/oder deren Borstenenden insbesondere anschließend zu deren Befestigung z.B. umspritzt oder mittels Materialverdichtung des Bürstenkopfes fixiert werden können. Hierbei können vorteilhaft Borstenbündel zusammengeführt werden.

Ferner ist denkbar, dass die Anwendungseinheit aus einem Grundkörper und einem Borstenplättchen, welches mit Borsten und/oder alternativen Reinigungselementen besetzt ist, zusammengesetzt ist. Hierzu werden zunächst mittels Spritzgießens Borstenplättchen mit Durchgangslöchern gefertigt, durch welche anschließend Borsten geführt werden. Vorzugsweise werden die Borsten anschließend auf einer Rückseite verbunden, insbesondere verschmolzen, vorzugsweise miteinander und/oder mit dem entsprechenden Borstenplättchen. Auf diese Weise beborstete Borstenplättchen können sodann mit einem Grundkörper, insbesondere einem Bürstenkopf, verbunden, z.B. verschweißt und/oder verklebt werden, vorzugsweise mittels eines Ultraschallschweißens. Hierzu weist der Grundkörper, insbesondere der Bürstenkopf, insbesondere eine Ausnehmung auf, in welche das Borstenplättchen eingesetzt werden kann. Als bekanntes Herstellungsverfahren ist in diesem Zusammenhang das Anchor-Free-Tufting-Verfahren zu nennen, das insbesondere ein Zusammenführen von Borstenbündeln ermöglicht. Als Unterseite des Borstenplättchens wird insbesondere eine Seite bezeichnet, welche in eine Ausnehmung des Grundkörpers gelegt wird und in Richtung Rückseite des Körperpflegeprodukts zeigt. Entsprechend zeigt die Oberseite des Borstenplättchens in Richtung der Vorderseite des Körperpflegeprodukts.

Als weiteres Verfahren zur ankerlosen Beborstung kommt eine Fertigung, insbesondere ein Spritzgießen, eines Bürstenkopfs mit Durchgangslöchern für Borsten infrage. Borsten können anschließend durch die Durchgangslöcher geführt und auf einer Rückseite verschmolzen werden, insbesondere miteinander und/oder mit dem Bürstenkopf. Vorzugsweise erfolgt anschließend ein Überspritzen, insbesondere mit zumindest einer Weichkomponente, der verschmolzenen Bereiche und/oder des Bürstenkopfs.

Zudem ist denkbar, zunächst einen Bürstenkopf mit Sacklöchern, beispielsweise mittels Spritzgießens und/oder mittels eines Bohrens der Sacklöcher, zu fertigen. Borsten werden in diesem Fall insbesondere zu Bündeln zusammengelegt und an einem Ende verschmolzen und/oder anderweitig verbunden. Der Bürstenkopf wird anschließend erwärmt. Sodann können vorteilhaft Borstenbündel in die Sacklöcher eingeführt und mittels eines Andrückens des Bürstenkopfs verankert werden. Insbesondere verformen sich hierbei die erwärmten Sacklöcher, sodass die Borstenbündel in denselben verankert werden.

Alternativ oder zusätzlich zu gestanzten und/oder angeschweißten und/oder angeklebten Borsten sind auch angespritzte Borsten denkbar. Diese können insbesondere während eines Mehrkomponentenspritzgießens gemeinsam mit der Anwendungseinheit, der Griffeinheit und/oder der Kopplungseinheit gefertigt sein, oder nachträglich an einen Grundkörper der Anwendungseinheit angespritzt sein. Im Gegensatz zu den konventionellen Borsten werden gespritzte Borsten nicht extrudiert.

Ein weiteres mögliches Verfahren zur Beborstung des Bürstenkopfs stellt das Eindrehen dar, wobei eine zylindrische Bürste entsteht. Hierbei wird beispielsweise Borste bzw. Filament von einer Rolle zugeführt, wobei insbesondere mehrere Borsten-/Filamentstränge auf einer Rolle aufgewickelt sind. Für die Maschinenbeschickung sind jeweils mehrere Rollen vorgespannt, denn jede Borste/Filament in der Bürste entspricht einem Borsten-/Filamentstrang. Die Borsten/Filamente werden in der Breite korrekt ausgebreitet, damit sie die Breite haben, in welcher sie in die Bürste eingeführt werden. Die Borsten/Filamente werden so vorgezogen, dass sie anschließend für den nächsten Schritt freistehen, d.h. dass ein Draht darüber geführt werden kann. Anschließend wird ein Draht ab einer Rolle auf die Maschine zugeführt, d.h. abgewickelt und in den Prozess eingeführt. Der Draht wird auf eine Länge geschnitten, welche größer ist als die abgewickelte Länge der eingedrehten Bürste, das endgültige Ablängen der Borsten/Filamente erfolgt nach dem Eindrehen. Der Draht wird zu einem U gebogen, damit die offene Seite anschließend über die Borsten/Filamente geschoben werden kann, um die Borsten einzufädeln. Der Draht wird am Boden des U's gehalten. Darauffolgend wird das offene Drahtende geklemmt, damit die Borsten/Filamente zwischen den Drahtstücken halten. Die Borsten/Filamente werden auf eine Länge geschnitten, welche größer ist als die Endlänge in der Bürste, damit die Bürste anschließend, wenn die Borsten/Filamente eingedreht sind, korrekt geschnitten werden kann. Der Draht wird gedreht, sodass die Borsten/Filamente zwischen dem Draht eingeklemmt und damit fixiert werden. Nachdem die Borsten/Filamente im Draht fixiert sind, werden sie auf die korrekte Länge geschnitten und profiliert. Nachdem der Bürstenteil fertiggestellt ist, wird der überschüssige Draht abgeschnitten.

Vorzugsweise gehen Materialien gespritzter Borsten bei einem Spritzgussprozess, insbesondere einem Zwei- und/oder Mehrkomponentenspritzgießen, keinen Materialschluss mit anderen Weichkomponenten und/oder Hartkomponenten und/oder anderen Materialien des Körperpflegeprodukts ein. Bevorzugt werden gespritzte Borsten vielmehr mittels eines Formschlusses, beispielsweise mittels zumindest eines Hinterschnitts und/oder zumindest eines Durchbruchs und/oder mittels zumindest einer zumindest teilweisen Umspritzung mit Weichkomponenten und/oder Hartkomponenten verbunden, wobei insbesondere eine Schwundverbindung und/oder eine Schrumpfverbindung denkbar sind. Es ist jedoch auch eine Verbindung mittels zumindest eines Materialschlusses insbesondere mit affinen bzw. kompatiblen Kunststoff-Materialien bzw. -Komponenten denkbar.

Für sämtliche erwähnte mögliche Spritzgussprozesse ist grundsätzlich ein Ein-, Zwei- und/oder Mehrkomponentenspritzguss denkbar. Verwendete Materialien, insbesondere unterschiedliche Weichkomponenten und/oder Hartkomponenten, können hierbei, wie erwähnt, stoffschlüssig und/oder formschlüssig verbunden werden und/oder sein. Auch eine Ausbildung von gelenkigen bzw. beweglichen oder flexiblen Verbindungen mittels geeigneter Spritzgussschritte ist denkbar. Es kommen grundsätzlich beispielsweise Heißkanalverfahren, Kaltkanalverfahren und/oder Co-Injektionsverfahren in Frage.

Alternativ oder zusätzlich zu einem mit Borsten besetzten Bürstenkopf kann die Anwendungseinheit auch zumindest einen Zungenreiniger und/oder zumindest ein alternatives Reinigungs- und/oder Massageelement aufweisen. Diese können jeweils aus einer Weichkomponente, aus einer Hartkomponente oder aus einer Kombination von Weich- und Hartkomponente ausgebildet und/oder vorteilhaft mittels Spritzgießens herstellbar und/oder hergestellt sein.

Vorzugsweise sind gespritzte Borsten zumindest teilweise und vorteilhaft vollständig aus einem thermoplastischen Polyurethan-Elastomer (TPE-U) ausgebildet. Hierbei ist eine Verwendung eines modifizierten Polyurethan-Elastomers (TPE-U) denkbar, welches insbesondere bezüglich verbesserter Fließeigenschaften und/oder einer schnellen Erstarrung, insbesondere einer schnellen Kristallisation, vorteilhaft bereits bei höheren Temperaturen, modifiziert sein kann. Selbstverständlich sind aber auch andere Materialien denkbar, beispielsweise thermoplastische Polyester-Elastomere (TPE-E), thermoplastische Polyamid-Elastomere (TPE-A), Polyethylen (PE), beispielsweise in den Formen low density Polyethylen (LDPE) oder linear low density Polyethylen (LLDPE), oder dergleichen. Materialien für gespritzte Borsten weisen vorteilhaft eine Shore-D-Härte von wenigstens 0 und besonders vorteilhaft von wenigstens 30 und/oder von höchstens 100 und vorteilhaft von höchstens 80 auf. Insbesondere ist eine Shore-Härte eines Materials gespritzter Borsten vorteilhaft höher als eine Shore-Härte übriger verwendeter Weichkomponenten, beispielsweise für Griffelemente, Massageelemente, weitere Reinigungselemente oder dergleichen. Die für die Herstellung von gespritzten Borsten eingesetzten Materialien können nachhaltige Materialien sein.

Grundsätzlich ist ferner eine Verwendung wasserlöslicher Polymere denkbar, beispielsweise für Hartkomponenten, Weichkomponenten, gespritzte Borsten, Zungenreiniger oder andere Elemente des Körperpflegeprodukts.

Ebenso können für die Hartkomponente, die Weichkomponente und/oder das Material für gespritzte Borsten Biokunststoffe herangezogen werden, welche insbesondere aus nachwachsenden Rohstoffen gewonnen sein können, biologisch abbaubar, insbesondere kompostierbar, sein können und/oder aus einem recycelten und/oder recycelbaren Material bestehen können. Vorzugsweise ist das Material insbesondere aus einem Kunststoff gebildet. Vorzugsweise ist das biologisch abbaubare, insbesondere kompostierbare, und/oder recycelte und/oder recycelbare Material von einem Biokunststoff, insbesondere aus einem Kunststoff auf Basis nachwachsender Rohstoffe und/oder aus einem biologisch abbaubaren Kunststoff, gebildet. Das Material kann daher insbesondere fossil-basiert und bioabbaubar sein, wie beispielsweise PVOH, PCL, PBAT, PET oder PBS, auf nachwachsenden Rohstoffen basieren und bioabbaubar sein, wie beispielsweise PLA, PHA, Cellophane oder Stärke-Blends, oder auf nachwachsenden Rohstoffen basieren und nicht bioabbaubar sein, wie beispielsweise Ca, Bio-PE, Bio-PP, Bio-PA, Bio-PET. Es sind verschiedene, einem Fachmann als sinnvoll erscheinende Biokunststoffe denkbar, wie beispielsweise stärkebasierte Biokunststoffe, cellulosebasierte Biokunststoffe, Polyhydroxy-Alkanoate, wie insbesondere Polyhydroxybuttersäure (PHB), Polymilchsäure (PLA), aliphatische und/oder aromatische Copolyester, oder weitere Biokunststoffe wie beispielsweise Lignin-basierte Biokunststoffe. Bevorzugt kann die Anwendungseinheit bzw. die Griffeinheit zu einem Großteil aus einem biologisch abbaubaren, insbesondere kompostierbaren, und/oder einem recycelten Material bestehen. Vorzugsweise besteht das Körperpflegeprodukt nur aus einer Komponente. Insbesondere weist das Körperpflegeprodukt eine Hartkomponente auf. Der materielle Griffkörper der Anwendungseinheit bzw. Griffeinheit kann mindestens teilweise aus der Hartkomponente bestehen. Die Hartkomponente und/oder die Weichkomponente und/oder das Material für gespritzte Borsten besteht vorteilhaft aus einem Biokunststoff, welcher insbesondere aus nachwachsenden Rohstoffen gewonnen sein kann. Als Rohstoffe kommen hierbei insbesondere Mais, Hanf, Zucker, Rizinusöl, Palmöl, Kartoffeln, Weizen, Zuckerrohr, Kautschuk, Holz, die Castor-Pflanze / der Wunderbaum und dergleichen infrage. Entsprechende mögliche Grundstoffe könnten beispielsweise Cellulose, Stärke, Milchsäure (PLA), Glucose, Chitin, Chitosan oder dergleichen sein, aus denen insbesondere entsprechende Biokunststoffe synthetisiert sein können.

Ferner ist auch der Einsatz von nachhaltiger Zahnseide, wie insbesondere Zahnseide aus den oben genannten nachhaltigen Materialien wie beispielsweise nachwachsenden Materialien wie Cellulose, organischem Material wie natürliche Seide. etc., denkbar. Des Weiteren ist auch der Einsatz von den genannten nachhaltigen Materialien für Borsten/Filamente, wie beispielsweise Borsten/Filamente aus Cellulose, denkbar. Zudem ist auch der Einsatz von den genannten nachhaltigen Materialien für Kunststoff-Folien, insbesondere anstelle oder in Kombination mit einem Papierwerkstoff, wie insbesondere PLA-Folien, für Flosser oder andere Anwendungen denkbar. Folien werden insbesondere als Laminat mit dem Papierwerkstoff verwendet.

Ferner ist denkbar, dass an dem Körperpflegeprodukt Trennhilfen und oder Sollbruchstellen vorgesehen sind, an welchen das Körperpflegeprodukt zu einer Entsorgung auseinandergenommen, aufgetrennt oder separiert werden kann, damit die verschiedenen Komponenten bzw. Materialien zwecks Entsorgung mind. teilweise sortenrein getrennt werden können. Es können beispielsweise Greiflaschen, beispielsweise zwischen zu trennenden Schichten, realisiert sein, über welche die Schichten getrennt werden können. Alternativ oder zusätzlich können nicht verbundene, nicht gesiegelte oder nicht geschweißte Abschnitte zu einer Trennung vorgesehen sein. Ferner können unterstützende Perforationen vorgesehen sein, beispielsweise zu einem Trennen von Bürste und Papier, insbesondere bei Interdentalbürsten oder Flossern. Vorzugsweise sind insbesondere Trennhilfen an dem Körperpflegeprodukt vorgesehen, wie insbesondere Zuglaschen, Greiflaschen und/oder Perforationen.

Unter "zumindest zu einem Großteil" soll insbesondere verstanden werden, dass zumindest ein Hauptbestandteil eines Materials des materiellen Griffkörpers der Griffeinheit und/oder der Anwendungseinheit ein Papierwerkstoff ist. Vorzugsweise sind zumindest 50 Gew.% (Gewichtsprozent), vorzugsweise zumindest 70 Gew.% und besonders bevorzugt zumindest 90 Gew.% des materiellen Griffkörpers der Griffeinheit und/oder der Anwendungseinheit aus einem Papierwerkstoff. Besonders bevorzugt besteht der materielle Griffkörper der Griffeinheit und/oder die Anwendungseinheit vollständig aus einem Papierwerkstoff. Ferner soll in diesem Zusammenhang unter einem "Papierwerkstoff" insbesondere ein, vorzugsweise flächiger, Werkstoff verstanden werden, der im Wesentlichen aus Fasern, vorzugsweise pflanzlicher Herkunft, besteht. Der Werkstoff wird vorzugsweise durch Entwässerung einer Fasersuspension auf einem Sieb gebildet. Das entstehende Faservlies wird anschließend insbesondere verdichtet und getrocknet. Vorzugsweise ist der Werkstoff aus Zellstoff, Holzschliff und/oder Altpapier hergestellt. Der Werkstoff kann dabei einlagig oder mehrlagig ausgebildet sein. Mehrlagiger Papierwerkstoff besteht aus mehreren Lagen von Papier unterschiedlicher Dicke und/oder unterschiedlichen Eigenschaften und/oder zumindest teilweise aus unterschiedlichem Material. Die Lagen können dabei durch Zusammenkleben oder Zusammenpressen, insbesondere ohne Einsatz von Klebstoff, hergestellt werden. Die Grammatur des Papierwerkstoffs beträgt insbesondere zwischen 50 g/m² und 1000 g/m², vorzugsweise zwischen 150 g/m² und 600 g/m². Gemäß DIN 6730 kann zwischen verschiedenen Papierwerkstoffen hinsichtlich der Grammatur unterschieden werden. So handelt es sich bis 225g/mm² um Papier, während man ab 225g/mm² von Pappe spricht. Umgangssprachlich wird bis 250g/m² von Papier, von 150g/m² bis 600 g/m² von Karton und ab 500 g/m² von Pappe gesprochen. Für Karton ist beispielsweise Vollpappe oder Wellpappe denkbar. Es gibt insbesondere verschiedene Varianten von Karton, insbesondere auch auf Recyclingbasis. Vorzugsweise besteht der Papierwerkstoff, insbesondere bei Karton, besonders bevorzugt bei Vollpappe, aus Frischfaser und/oder Sekundärfaser. Die Frischfaser bzw. Primärfaser ist dabei von Neumaterial, wie beispielsweise FSC Holz, gebildet. Die Sekundärfaser ist insbesondere von einem Recycling-Papier bzw. -Karton gebildet. Alternativ oder zusätzlich sind auch noch weitere Füllstoffe wie Gras für Graspapier denkbar. Papier wird aus Faserstoffen hergestellt, die heute vor allem aus dem Rohstoff Holz gewonnen werden. Es besteht insbesondere auch die Möglichkeit von holzfreier Cellulose. Bevorzugte Basis des Papierwerkstoffs ist jedoch Cellulose bzw. Zellstoff auf Basis von Holz. Mögliche Basis-Holzarten sind beispielsweise Pappel, Buche, Fichte, Kiefer oder Eukalyptus. Alternativ kann der Papierwerkstoff auch aus Gras, Bambus oder Papyrus gewonnen werden. Die wichtigsten Faserstoffe sind Zellstoff, Holzstoffe und Altpapierstoff. Gewisses Altpapier kann aufgrund von Druckerschwärze für den Einsatz in der Mundhygiene eher weniger geeignet sein. Außer dem Faserstoff oder einer Faserstoffmischung kann der Werkstoff häufig auch Füllstoffe und weitere Zusatzstoffe enthalten. Vorzugsweise weist der Papierwerkstoff einen Recycling-Anteil von zumindest 80% auf, wobei der Papierwerkstoff einen Anteil an Primärfasern enthalten kann. Durch den Einsatz von Neumaterial können die Eigenschaften des Papierwerkstoffs in engerer Bandbreite gehalten werden und es kann u.a. eine bessere Reißfestigkeit und dadurch Stabilität erreicht werden. Es sind verschiedene, einem Fachmann als sinnvoll erscheinende Papierwerkstoffe denkbar. Zudem ist denkbar, dass der Papierwerkstoff gestrichen, das heißt beschichtet gestrichen oder ungestrichen, oder strukturiert ist, wie beispielsweise durch eine 3D Kontur. Mittels einer Strukturierung und/oder Beschichtung kann insbesondere eine Griffigkeit des Körperpflegeprodukts verbessert werden. Insbesondere kann die Strukturierung sowohl im Papierwerkstoff selbst, wie beispielsweise durch eine Oberflächenbearbeitung und/oder eine Prägung, als auch in einer Bedruckung realisiert werden. Vorzugsweise erfolgt eine Herstellung des Papierwerkstoffs frei von einem Spritzguss- oder Extrusionsprozess ausgenommen Faserguss oder Faserspritzguss, der im Vergleich zur Verarbeitung von Thermoplasten bei tieferen Temperaturen bzw. niedrigerem Druck durchgeführt wird. Die Temperatur liegt im Bereich von maximal 200°C und der Druck beträgt zwischen 10 bar und 40 bar, vorzugsweise zwischen 20 bar und 35 bar. Insbesondere ist bei der Herstellung des Papierwerkstoffs kein Schmelzprozess von Thermoplasten involviert.

Der Papierwerkstoff kann insbesondere auf verschiedene, einem Fachmann als sinnvoll erscheinende Arten bereitgestellt werden wie insbesondere in Form von Bögen, in Form von Nutzen, ab Rolle, in Block-Form oder als Granulat oder Grundstoff. Der Block kann dabei beispielsweise aus einem Kartonmaterial bestehen und beispielsweise zum mechanischen Bearbeiten wie z.B. Fräsen vorgesehen sein.

Der Papierwerkstoff kann abhängig von einer Anwendung verschiedene Dichten bzw. Stärken aufweisen. Mögliche Grammaturen sind beispielsweise ein Karton mit 330g/m², insbesondere zwei Mal einen solchen Karton verbunden z.B. geschweißt, laminiert etc., für einen Flosser oder eine Interdentalbürste. Mögliche Dichten bzw. Stärken pro Schicht können 150g/m² bis 650g/m², vorzugsweise 250 g/m² bis 500 g/m², besonders bevorzugt 300gm/2 - 400g/m2 bei Verbindung mehrerer Bögen oder Schichten. Bei Mundhygieneprodukten, bei denen flächige Schichten von Papierwerkstoffen eingesetzt werden (z.B. für Flosser oder eine Interdentalbürste), werden bevorzugt mind. 2 Schichten Papierwerkstoff eingesetzt. Es können aber auch 2, 3, 4 oder 5 Werkstoffschichten verwendet werden. Die Schichten können unterschiedliche Grammaturen und Papierwerkstoffe aufweisen. Die Schichten können Kunststofffolien aufweisen. Vorzugsweise sind die Kunststofffolien beidseitig von Schichten aus Papierwerkstoffen abgedeckt. Die Schichten können mittels Falten eines flächigen Papierwerkstoffs erzeugt werden. Es können zwei oder mehrere Faltungen erzeugt werden. Die Faltungen können sequentiell bzw. rollend sein oder die Seite jeweils alternierend wechseln (Handorgel).

Bei Mundhygieneprodukten, bei denen gerollte Schichten von Papierwerkstoffen eingesetzt werden wie z.B. bei Zahnbürsten, Interdentalbürsten, Nassrasierer etc.), wird mit dem Rollen die Stabilität im Wesentlichen über die Anzahl der gerollten Lagen bzw. Umdrehungen erreicht, sodass z.B. auch sehr dünnes Papier ab 40g/m² verwendet werden kann. Beim Rollen werden bevorzugt Dichten bzw. Stärken von 5g/m² bis 150g/m² vorzugsweise 250 g/m² 10 bis 80 g/m², besonders bevorzugt 20 g/m² - 50 g/m² eingesetzt. Je nach gewünschtem Rollendurchmesser, falls gewünscht innerem Hohlraumdurchmesser und Dichte bzw. Stärke des eingesetzten Papierwerkstoffs, wird die Anzahl gerollte Lagen bzw. Umdrehungen eingestellt. Dadurch ergeben sich die von 1 bis 150 Lagen bzw. Umdrehungen, bevorzugt 1 - 100 Lagen bzw. Umdrehungen, besonders bevorzugt 1 - 80 Lagen bzw. Umdrehungen. Auch beim Rollen können auch 2, 3, 4 oder 5 Werkstoffschichten verwendet werden. Die Schichten können unterschiedliche Grammaturen und Papierwerkstoffe aufweisen. Die Schichten können Kunststofffolien aufweisen. Vorzugsweise sind die Kunststofffolien beidseitig von Schichten aus Papierwerkstoffen abgedeckt. Das Rollen kann eine Art fortlaufende Wicklung mit einem Band aus Papierwerkstoff sein.

Vorzugsweise kann der Papierwerkstoff von einem holzfrei gestrichenen Karton gebildet sein. Insgesamt kann eine Oberfläche des Papierwerkstoffs jeweils in Abhängigkeit der Anwendung gestrichen, bedruckt, geprägt oder dergleichen sein. Für den Papierwerkstoff ist insbesondere eine hohe Reißfestigkeit und zumindest anwendungsseitig eine hohe Wasserbarriere vorteilhaft. Die Reißfestigkeit beträgt zwischen 20 N und 100 N, vorzugsweise von 30 N bis 60 N.

Der Kunststoffanteil am Gesamtgewicht des Körperpflegeprodukts beträgt weniger als 10%, vorzugsweise weniger als 5%, am meisten bevorzugt weniger als 3%. Der Kunststoffanteil setzt sich insbesondere zusammen aus Folie oder auch einem Zahnseidenfaden/-band.

Ferner kann der Papierwerkstoff insbesondere zu einer Erreichung zusätzlicher Eigenschaften zusätzlich behandelt sein. Es kann beispielsweise eine Beschichtung auf einer Oberfläche des Papierwerkstoffs in Form eines Lacks, Klebstoffs, einer physikalischen und/oder chemischen Behandlung für die Wasserresistenz oder einer Veredelung vorgesehen sein.

Des Weiteren sind Füllstoffe für den Papierwerkstoff denkbar. Es sind insbesondere unlösliche Füllstoffe und lösliche Füllstoffe denkbar. Mögliche Füllstoffe sind für antibakterielle Additive beispielsweise Silber-Partikel , Polymere für eine verbesserte Wasserfestigkeit, wie beispielsweise bei der Papierbecherherstellung, abrasive Mittel (Erhöhen des Reibungskoeffizienten), beispielsweise zur Verbesserung der Reinigungsleistung falls der Papierwerkstoff in der Reinigungseinheit eingesetzt wird oder zur Verbesserung des Haltens falls der Papierwerkstoff in der Griffeinheit eingesetzt wird, und/oder magnetische Partikel für ein magnetisches Papier, beispielsweise zur Halterung bzw. Lagerung des Körperpflegeprodukts.

Weitere Füllstoffe, die sich im Gebrauch lösen, können folgende Wirksubstanzen sein, welche in einen entsprechenden Träger eingebunden sind und freigesetzt werden:
a) Wirkstoffe mit zahnpastaähnlicher Wirkung: Sorbitol, Aromen, Hydrated Silica, Sodium Lauryl Sulfate, Sodium Monofluorophophate, Kreatin, Zink Sulfate, Triclosan, Glycerin, Sodium Saccharin, Propylene Glycol, Disodium Phosphate, Alumina, Trisodium Phosphate, Sodium Fluoride, Betaine, Titanium Dioxide, Cellulose Gum, Tetrasodium Pyrophosphate etc.
b) Wirkstoffe mit antibakterieller Wirkung: Sodium Bicarbonate, Citric Acid, Phosphric Acid, Sodium Carbonate, Potassium Carbonate, Sodium Perborate, Sodium Hexametaphosphate, Sodium Benzoate, Sodium Stéarate etc.
c) Wirkstoffe zum Anzeigen des Putzerfolges mittels Einfärben des Plaques auf der Zahnoberfläche: Glucose, Maltodextrin, Magnesium Sterate, Aroma, Saccharin, Microcrystaline Cellulose etc.
d) Wirkstoffe, die Geschmack abgeben
e) Wirkstoffe, die Geruch abgeben

Entsprechende Papierwerkstoffe sind beispielsweise bereits von Karton für Trinkbecher der Firma Storaenso bekannt, welcher foodtauglich, wasserdicht, kompostierbar und bedruckbar ist. Ferner ist von der Firma Kotkamills Karton bekannt, welcher mittels Dispersion wasserdicht gemacht wird und voll recyclier- und kompostierbar bleibt. Ferner sind bereits Kartonröhren bekannt.

Der Papierwerkstoff kann vorbehandelt, z.B. veredelt sein. Unter einer "Veredelung" soll dabei eine Oberflächenbehandlung des Papierwerkstoffs verstanden werden, die beispielsweise dazu führt, dass der Papierwerkstoff einen hochwertigeren Eindruck erzielt. Eine Veredelung kann als oder während oder nach dem Druckvorgang des Papierwerkstoffs aufgebracht werden. Eine Veredelung kann während oder nach dem Montagevorgang des Papierwerkstoffs mit anderen Elementen des Körperpflegeprodukts aufgebracht werden. Die Veredelung kann jeweils partiell an einer oder mehreren Stellen oder auch vollflächig aufgebracht/realisiert werden. Durch die Veredelung können beispielsweise metallische Effekte, optische Unterscheidungen oder auch Produktbeschriftungen realisiert werden. Durch die entsprechende Beschichtung kann auch eine Verminderung der Wasseraufnahme des Papierwerkstoffs erreicht werden. Damit kann sich der Papierwerkstoff auch für einen Mehrfacheinsatz eignen. Veredelung kann beispielsweise eine Lackierung sein, dabei kann beispielsweise UV-Hochglanzlack, Strukturlack, Softtouch-Lack oder Glitterlack verwendet werden. Die Veredelung kann eine Beflockung sein. Die Veredelung kann eine Prägung, beispielsweise eine Silberfolienprägung oder eine Hologramm-Prägung, sein. Die Veredelung kann ein Duftlack sein.

Als eine weitere Möglichkeit der Vorbehandlung ist beispielsweise das Prägen des Papierwerkstoffs vorgesehen. Dabei wird der Papierwerkstoff mittels Druck mindestens teilweise verformt. Damit ist auf der Oberfläche des Papierwerkstoffs in Teilbereichen der Oberfläche eine dreidimensionale Gestaltung und/oder eine Oberflächenstruktur zu erreichen. Es ist ferner möglich, mittels Prägung Hohlräume innerhalb der Schichten von Papierwerkstoffen zu erzielen. Damit wird eine bessere Griffigkeit bez. Rutschfestigkeit des Griffkörpers erreicht und damit die Handhabung des Mundhygieneprodukts erleichtert. Geprägt können grundsätzlich alle Papierwerkstoffe, seien diese in einem u.a. flächigen, gerollten, laminierten, gesiegelten und/oder geschweißten Zustand. Eine Prägung kann mit einem Prägewerkzeug (Negativform) mittels Druck und/oder Wärme und/oder Dampf etc. unterstützt werden. Eine Prägung kann im flächigen Zustand bei Ausstanzen des Papierwerkstoffs gemacht werden. Es ist aber auch möglich, dass eine Prägung an einem gerollten Papierwerkstoff durchgeführt wird.

Vorzugsweise wird der materielle Griffkörper ab Bogen, Rolle oder Nutzen geschichtet. Ferner ist denkbar, dass die zumindest zwei Schichten vor einer Verarbeitung vorbehandelt, bedruckt oder veredelt werden (siehe dazu die zusätzlichen Ausführungen in dieser Schrift). Die Vorbehandlung, Bedruckung, Veredelung kann nur eine Schicht betreffen. Als Vorbehandlung ist beispielsweise ein Prägen denkbar, mit dem Ziel, eine Struktur auf der Oberfläche des Endprodukts zu erzeugen. Alternativ oder zusätzlich ist als Vorbehandlung ein Beschichten denkbar. Beispielsweise könnte eine Innenseite jeder Schicht zu einem Kleben vorgesehen sein, während eine Außenseite einen Schutz gegen Feuchtigkeit aufweist. Jede Schicht kann daher zwei Seiten mit unterschiedlichen Beschichtungen aufweisen. Alternativ oder zusätzlich ist als Vorbehandlung eine Bedruckung denkbar. Des Weiteren ist denkbar, dass die zumindest zwei Schichten nach einer Herstellung nachbehandelt werden. Als Nachbehandlung ist beispielsweise ein Beschichten, ein Bedrucken, ein Beschichten der Anwendungseinheit und/oder ein Laminieren denkbar. Vorzugsweise ist die Anwendungseinheit direkt mit dem materiellen Griffkörper verbunden. Die Anwendungseinheit wird insbesondere zwischen die Schichten des materiellen Grundkörpers eingelegt und zusammen mit den Schichten aus Papierwerkstoff verbunden.

Eine Folienlaminierung, Folienkaschierung und/oder Heißfolienprägung kann genutzt werden. Es können dabei u.a. mehrere Lagen, beispielsweise Karton mit einer Folie, verbunden werden. Dabei können Mattfolien, Glanzfolien oder auch Strukturfolien genutzt werden. Dadurch werden die Oberflächen insbesondere langlebiger. Prägungen/Prägefoliendruck kann verwendet werden, dabei ist insbesondere ein Plan- oder Strukturprägen möglich. Auch eine Laminierung oder eine Einsiegelung kann zur Veredelung genutzt werden.

Ferner wird vorgeschlagen, dass der materielle Griffkörper der Griffeinheit zumindest eine Schicht aus einem Papierwerkstoff aufweist, welche gerollt ausgebildet ist. Dadurch kann insbesondere vorteilhaft ein Griffkörper hergestellt werden. Es kann insbesondere ein vorteilhaft stabiler und leicht herzustellender Griffkörper bereitgestellt werden. Vorzugsweise ist die Schicht aus dem Papierwerkstoff um eine definierte Rollachse aufgerollt. Die Rollachse verläuft vorzugsweise parallel, insbesondere im Wesentlichen koaxial, zu einer Längsachse der Griffeinheit. Insbesondere wird die Schicht bei einer Herstellung des materiellen Griffkörpers aufgerollt. Vorzugsweise bildet die Schicht in einem Endzustand, insbesondere nach einer Herstellung eine Rolle, insbesondere eine zylindrische Rolle, aus. Bevorzugt weist die Schicht in einer Ebene senkrecht zu der Längsachse der Griffeinheit zumindest abschnittsweise, insbesondere vollständig, einen spiralförmigen Querschnitt auf. Eine größte Fläche der Schicht erstreckt sich insbesondere um eine Längsachse der Griffeinheit. Es wäre auch denkbar, dass die Schicht, insbesondere innerhalb der Rolle, einen von einer Spirale abweichenden Querschnitt aufweist. Beispielsweise wäre denkbar, dass die Schicht innerhalb der Rolle insbesondere im Kern zu einer weiteren Versteifung eine dreieckige Querschnittsform aufweist.

Des Weiteren wird vorgeschlagen, dass die zumindest eine Schicht des materiellen Griffkörpers in eine zylindrische Form gerollt ist. Dadurch kann insbesondere vorteilhaft ein Griffkörper hergestellt werden. Es kann insbesondere ein vorteilhaft stabiler und leicht herzustellender Griffkörper bereitgestellt werden. Es kann insbesondere eine vorteilhafte Griffform bereitgestellt werden. Vorzugsweise ist die zumindest eine Schicht des materiellen Griffkörpers in eine hohlzylindrische Form gerollt. Die zylindrische Form kann dabei insbesondere eine von einem Kreis verschiedene Form einer Grundseite aufweisen. Beispielsweise wäre denkbar, dass die zylindrische Form eine ovale, regelmäßige oder unregelmäßige n-eckige z.B. dreieckige, 4-, 5-, 6-, 7-, 8-eckige, quadratische, rechteckige, trapezförmige und/oder rhombusförmige Grundseite aufweist. Die zylindrische Grundform kann auch konkave Stellen / Elemente aufweisen. Es wäre auch denkbar, dass der materielle Grundkörper mehrere Schichten aufweist, welche gemeinsam in eine zylindrische Form gerollt sind. Unter einer "zylindrischen Form" soll in diesem Zusammenhang insbesondere die Form eines Körpers verstanden werden, mit zwei einander gegenüberliegenden im Wesentlichen gleichgeformten Grundseiten, welche über einen Mantel miteinander verbunden sind und einen Körper ausformen. Vorzugsweise weist der Griffkörper zumindest im Wesentlichen die Form eines Kreiszylinders oder eines Hohlzylinders auf. Der Zylinder kann auch schraubenartig verdreht sein. Unter "zumindest im Wesentlichen" soll in diesem Zusammenhang insbesondere verstanden werden, dass eine Abweichung von einem vorgegebenen Wert insbesondere weniger als 25%, vorzugsweise weniger als 10% und besonders bevorzugt weniger als 5% des vorgegebenen Werts beträgt.

Dabei sind insbesondere verschiedene, einem Fachmann als sinnvoll erscheinende Ausgestaltungen des Körperpflegeprodukts denkbar. Der materielle Griffkörper kann insbesondere sowohl aus einem oder mehreren Bögen eines Papierwerkstoffs und/oder von Rollen abgerollt hergestellt werden und/oder als eine Rolle aus Streifen bzw. einem Band gerollt sein. Die Schichten des materiellen Griffkörpers können beispielsweise mittels Kleben, Siegeln, Schweißen und/oder Laminieren miteinander verbunden sein. Ferner ist denkbar, dass die zumindest eine Schicht vor einer Verarbeitung vorbehandelt wird. Als Vorbehandlung ist beispielsweise ein Prägen denkbar, mit dem Ziel, eine Struktur auf der Oberfläche des Endprodukts zu erzeugen. Alternativ oder zusätzlich ist als Vorbehandlung des Papierwerkstoffs ein Beschichten denkbar. Beispielsweise könnte eine Innenseite jeder Schicht des Papierwerkstoffs zu einem Kleben vorgesehen sein, während eine Außenseite einen Schutz gegen Feuchtigkeit aufweist. Jede Schicht kann daher zwei Seiten mit unterschiedlichen Behandlungen bzw. Beschichtungen aufweisen. Alternativ oder zusätzlich ist als Vorbehandlung eine Bedruckung denkbar. Des Weiteren ist denkbar, dass die zumindest eine Schicht nach einer Herstellung nachbehandelt wird. Als Nachbehandlung ist beispielsweise ein Beschichten, ein Bedrucken und/oder ein Laminieren denkbar. Alternativ oder zusätzlich ist als Nachbehandlung ein Verformen denkbar, wie insbesondere mittels Drucks und Wärme, beispielsweise für eine Greifgeometrie.

Vorzugsweise kann der Papierwerkstoff sowohl zu einer massiven Rolle gerollt sein als auch zu einer hohlen Rolle gerollt sein. Bei einer hohlen Rolle wäre insbesondere zudem denkbar, dass die Rolle zusätzlich mit einem Füllmaterial, wie beispielsweise einem Schüttgut und/oder einem Kern aus einem Papierwerkstoff oder einem weiteren Material, mindestens teilweise gefüllt ist. Zu einer Herstellung einer hohlen Rolle, insbesondere eines hohlen materiellen Griffkörpers, werden insbesondere Papierbänder von Rollen abgewickelt und gemeinsam zu einem Rohr bzw. wie oben definiert zu einem Hohlzylinder geformt. Die Papierbänder werden dabei insbesondere auf einen Kern aufgerollt und dann darauf verbunden. Das Verfahren entspricht dabei im Wesentlichen dem Verfahren zur Herstellung von Papier-Trinkhalmen. Die Anwendungseinheit, insbesondere mit einem Kunststoff-Kopf und/oder einer Interdentalbürste, ist insbesondere mit einer Schnittstelle zu dem hohlen Rohr hergestellt und kann direkt auf dem Rohr des materiellen Griffkörpers montiert werden. Dabei wäre insbesondere auch denkbar, dass die Anwendungseinheit als ein Wechselkopf ausgebildet ist, wobei die Schnittstelle in der Anwendungseinheit zwischen einem Verbindungsabschnitt mit dem materiellen Griffkörper und der Reinigungseinheit integriert ist. Eine Schwierigkeit besteht insbesondere in der Erreichung von genügend Stabilität sowie einer stabilen Montage, welche die Nutzungskräfte des Körperpflegeprodukts absorbieren kann. Vorzugsweise kann bei einer Ausbildung der Anwendungseinheit als Wechselkopf jedoch insbesondere eine Griffeinheit mit verschiedenen Anwendungseinheiten bereitgestellt werden. Ein Rohr aus einem Papierwerkstoff stellt ferner insbesondere ein einfaches Standardprodukt dar und kann dadurch vorteilhaft günstig hergestellt werden.

Der materielle Griffkörper kann insbesondere gerade oder spiralförmig gerollt sein. Verschiedene Bänder können dabei insbesondere verschiedene Eigenschaften aufweisen, wie beispielsweise verschiedene Farben zu einem Erreichen einer Spiral-Optik. Der materielle Griffkörper weist insbesondere einen (größten) Durchmesser von 1 mm bis 25 mm, vorzugsweise 1.5 mm bis 20 mm, besonders bevorzugt 2 mm bis 18 mm auf. Optional kann der materielle Griffkörper neben dem Rohr aus Papierwerkstoff zudem mit einem Kunststoff-Kopf versehen sein, welcher beispielsweise ein Eindringen von Wasser in den Papierwerkstoff und/oder in einen Hohlraum innerhalb der Papierwerkstoffschichten verhindert oder zumindest reduziert. Dazu sollte v.a. die Stirnseite des Papierwerkstoffs sowie Hohlräume besonders geschützt sein.

Ferner ist insbesondere eine Ausbildung des Körperpflegeprodukts als Zahnstocher bzw. Interdentalreiniger oder Dentalstick denkbar. Das Körperpflegeprodukt kann dabei insbesondere von einem Rohr oder Stab aus einem Papierwerkstoff gebildet sein, welches/welcher mechanisch bearbeitet bzw. in Form gebracht wird, z.B. schräg abgeschnitten wird und so einen Zahnstocher bildet. Zu einer Herstellung des Körperpflegeprodukts werden dabei z.B. insbesondere Papierbänder von Rollen abgewickelt und gemeinsam zu einem Rohr geformt. Die Papierbänder werden dabei insbesondere auf einen Kern abgerollt und dann darauf verbunden. Darauffolgend kann ein Ende des Rohrs schräg geschnitten und/oder angespitzt werden, sodass an einem Ende die Anwendungseinheit entsteht. Es können auch andere in dieser Schrift beschriebene Verfahren angewendet werden, um eine Rolle oder einen Zylinder zu bilden.

Die Anwendungseinheit ist dabei insbesondere einstückig mit der Griffeinheit ausgebildet. Optional wäre denkbar, dass zumindest ein Teil der Oberfläche des Körperpflegeprodukts nachbehandelt wird. Mögliche Verfahren sind Bedrucken, Tauchen, Sprayen etc. Dabei wäre denkbar, dass das Körperpflegeprodukt für weniger Wasseraufnahme lackiert wird oder das Körperpflegeprodukt bedruckt wird. Eine vorteilhafte Wasserfestigkeit ist dabei insbesondere sinnvoll, da die Anwendungseinheit aus Papier ist. Es können auch Körperpflegeprodukte aufgebracht werden. Selbstverständlich können auch andere in dieser Schrift beschriebene Verfahren zu Veredelungen, zum Einsatz von Füllstoffen oder Behandlungen des Papierwerkstoffs angewendet werden.

Es wird ferner vorgeschlagen, dass der materielle Griffkörper der Griffeinheit zumindest zwei Schichten aus einem Papierwerkstoff aufweist, die geschichtet miteinander verbunden sind. Dadurch kann insbesondere vorteilhaft ein Griffkörper hergestellt werden. Es kann insbesondere ein vorteilhaft stabiler und leicht herzustellender Griffkörper bereitgestellt werden. Es kann insbesondere eine vorteilhafte Griffform bereitgestellt werden. Vorzugsweise besteht der materielle Griffkörper, insbesondere die gesamte Griffeinheit, aus den zumindest zwei Schichten. Vorzugsweise sind die zumindest zwei Schichten mindestens teilweise übereinander angeordnet und miteinander verbunden. Vorzugsweise sind von 2 bis 6, vorzugsweise von 2 bis 4, Schichten vorgesehen. Die Schichten können aus unterschiedlichen Papierwerkstoffen, unterschiedlichen Dichten bzw. Grammaturen von Papierwerkstoffen bestehen. Es können zusätzliche Schichten aus Folien, insbesondere Kunststofffolien vorgesehen sein. Bevorzugt sind die zumindest zwei Schichten senkrecht zu einer Haupterstreckungsebene der Schichten mindestens teilweise übereinander angeordnet und miteinander verbunden. Die zumindest zwei Schichten grenzen insbesondere mit einer größten Fläche aneinander an. Die Schichten können beispielsweise mittels Kleben und/oder Siegeln und/oder Pressen und/oder Schweißen und/oder Laminieren miteinander verbunden werden. Die Schichten werden insbesondere bei einer Herstellung des Körperpflegeprodukts miteinander verbunden. Unter einer "Haupterstreckungsebene" eines Objekts soll insbesondere eine Ebene verstanden werden, welche parallel zu einer größten Seitenfläche eines kleinsten gedachten Quaders ist, welcher das Objekt gerade noch vollständig umschließt, und durch den Mittelpunkt des Quaders verläuft.

Vorzugsweise wird der materielle Griffkörper ab Bogen, Rolle oder Nutzen geschichtet. Ferner ist denkbar, dass die zumindest zwei Schichten vor einer Verarbeitung vorbehandelt, bedruckt oder veredelt werden (siehe dazu die zusätzlichen Ausführungen in dieser Schrift). Die Vorbehandlung, wie z.B. Prägung Bedruckung, Veredelung kann auch nur eine Schicht betreffen. Als Vorbehandlung ist beispielsweise auch ein Prägen denkbar, mit dem Ziel, eine Struktur auf der Oberfläche des Endprodukts zu erzeugen. Ferner können wie beschrieben mittels Prägung des Papierwerkstoffs auch Hohlräume zwischen den Schichten der Papierwerkstoffe gebildet werden. Dabei werden z.B. bei 2 Schichten an gleicher Position des Griffkörpers vor dem Verbinden der Papierwerkstoffschichten 2 entgegengesetzte Prägungen vorgesehen, um einen mindestens in Teilbereichen 3-dimensionalen statt flächigen Griffkörper zu erzeugen.

Alternativ oder zusätzlich ist als Vorbehandlung ein Beschichten denkbar. Beispielsweise könnte eine Innenseite jeder Schicht des flächigen Papierwerkstoffs zu einem Kleben vorgesehen sein, während eine Außenseite einen Schutz gegen Feuchtigkeit aufweist. Jede Schicht kann daher zwei Seiten mit unterschiedlichen Beschichtungen bzw. Eigenschaften aufweisen. Alternativ oder zusätzlich ist als Vorbehandlung eine Bedruckung denkbar. Des Weiteren ist denkbar, dass die zumindest zwei Schichten des flächigen Papierwerkstoffs nach der Verbindung nachbehandelt werden. Als Nachbehandlung ist beispielsweise ein Beschichten, ein Bedrucken, ein Beschichten der Anwendungseinheit und/oder ein Laminieren denkbar. Vorzugsweise ist die Anwendungseinheit direkt mit dem materiellen Griffkörper verbunden. Die Anwendungseinheit wird insbesondere zwischen die Schichten des materiellen Grundkörpers eingelegt und zusammen mit den Schichten aus Papierwerkstoff verbunden. Dabei kommen z.B. Schweißen, Siegeln, Laminieren, Leimen oder andere Verbindungstechniken in Frage. Die Anwendungseinheit wird insbesondere quasi zwischen die zu verbindenden Schichten geklemmt. Die Anwendungseinheit kann dabei beispielsweise von einer Interdentalbürste einer Zahnseide, einem Band, einer Folie, einem Zahnstocher oder einem anderen Körperpflegeprodukt gebildet sein. Beim Verbinden der beiden flächigen Papierwerkstoffschichten verdrängt der überlappende Teil der Anwendungseinheit den Papierwerkstoff mindestens teilweise. Damit wird der Halt verstärkt. Der überlappende Teil der Anwendungseinheit kann deformiert werden, um den Halt im Papierwerkstoff zu erhöhen.

Als Körperpflegeprodukt mit einem entsprechenden materiellen Grundkörper wäre insbesondere ein Flosser denkbar. Eine Zahnseide kann dabei insbesondere in mindestens teilweise vorgestanzte Bögen der Schichten eingelegt werden. Bei einem Herstellungsverfahren werden insbesondere Bögen oder Nutzen aus Papierwerkstoff, welche die Schichten ausbilden, vorgestanzt. Eine Vorstanzung erfolgt insbesondere im Bereich der Anordnung des funktionalen Elements. Beim Vorstanzen können auch Nutzen gebildet werden. Es erfolgt insbesondere eine Vorstanzung der Bereiche des materiellen Griffkörpers, die nach Montage des funktionalen Elements nicht mehr gestanzt werden können. Anschließend wird insbesondere der vorgestanzte Bogen bzw. Nutzen aus Papierwerkstoff bereitgelegt und ein Faden, ein Band oder eine Folie eingelegt. Eine Folie ist dünn und kann aus einem stabilen, reißfesten Papierwerkstoff oder einem Kunststoff insbesondere Hart- oder Weichkomponente oder aus einem nachhaltigen Kunststoffmaterial bestehen. Darauffolgend wird ein weiterer vorgestanzter Bogen bzw. Nutzen aus Papierwerkstoff darübergelegt und die Bögen bzw. Nutzen verbunden, wie beispielsweise durch Laminieren, Siegeln, Schweißen und/oder Kleben. Anschließend kann das Körperpflegeprodukt, insbesondere dessen Außengeometrie, in einzelne Flosser oder Nutzen von Flossern fertig gestanzt werden und überschüssiger Faden, Band oder Folie entfernt werden.

Es wäre auch denkbar, dass auf einen entsprechenden Schritt verzichtet wird und direkt anstatt des Vorstanzens direkt richtig gestanzt wird und der Faden, das Band oder die Folie direkt nicht über die finale Außengeometrie ragt. Der Faden, das Band oder die Folie kann auf verschiedene, einem Fachmann als sinnvoll erscheinende Arten verankert werden. Eine Verankerung kann beispielsweise durch Befestigen des Fadens, des Bands oder der Folie mittels Klebstoffs erfolgen. Alternativ oder zusätzlich kann eine Verankerung durch Schweißen erfolgen. Der Faden, das Band oder die Folie kann mittels Schweißen zwischen den Schichten befestigt werden. Alternativ oder zusätzlich kann eine Verankerung durch geschlitzte Arme an dem materiellen Griffkörper und einem darin eingelegten Faden oder Band erfolgen. Hierzu ist insbesondere ein mehrlagiger Aufbau vorgesehen, wobei eine Schlitzung in der mittleren Lage erfolgt, damit der Faden oder das Band im Verankerungsbereich überall an den Schichten anliegt. Anschließend wird der Faden oder das Band durch die Schlitzung geführt. Zusätzlich können insbesondere mehrere Schlitze vorgesehen sein, wobei der Faden oder das Band bei mehreren Schlitzen wie beim Weben abwechselnd durch die Schlitze geführt wird. Alternativ oder zusätzlich kann eine Verankerung durch umlegbare Ohren an dem materiellen Grundkörper erfolgen. Vorzugsweise weist der materielle Grundkörper dafür Arme mit je einer seitlichen Ausstülpung auf, welche einander entgegengesetzt sind. Der Faden oder das Band kann zu einer Verankerung über die Ausstülpung geführt werden, wobei jedes Fadenende bzw. Bandende auf einer Ausstülpung liegt. Anschließend wird der Faden oder das Band verschweißt und die Ausstülpungen werden gefaltet, insbesondere derart, dass der Faden oder das Band abgeknickt um ein Eck geführt wird. Dies bringt insbesondere eine vorteilhafte Stabilisierung. Darauffolgend wird insbesondere je Seite mindestens eine weitere Schicht angebracht, damit der Faden oder das Band im Verankerungsbereich jeweils an einer weiteren Schicht anliegt.

Alternativ oder zusätzlich kann eine Verankerung des Fadens, des Bandes oder der Folie durch Verformen des Papierwerkstoffs zum Schaffen von stabilisierenden Strukturen erfolgen. Es kann beispielsweise eine rechenartige Struktur in die Schichten eingebracht werden. Das Verformen kann insbesondere vor oder nach dem Verbinden erfolgen. Alternativ oder zusätzlich kann eine Verankerung durch Schaffen einer strukturierten Oberfläche für die Befestigung der Anwendungseinheit erfolgen. Durch Schweißen können beispielsweise geriffelte Flächen und/oder raue Oberflächen erzeugt werden. Alternativ kann mittels Verstemmen oder Verkrallen eine entsprechende Verankerung erreicht werden.

Alternativ oder zusätzlich kann eine Verankerung durch Schaffen von Spannungslaschen erreicht werden. Der Faden oder das Band kann hierzu insbesondere um die Spannungslaschen herumgeführt werden. Hierdurch kann ein späteres Ausreißen verhindert werden, wenn die Spannungslaschen zwischen den Schichten fixiert sind. Die Spannungslaschen haben eine Länge von 5 mm bis 30 mm, vorzugsweise von 10 mm bis 20 mm, bei einer Breite von 2 mm bis 6 mm vorzugsweise von 3 mm bis 4 mm. Die Spannungslaschen sind so angeordnet, dass sie neben dem Freiraum des Fadens oder des Bands liegen. Ferner kann der Faden oder das Band zudem an einer anderen Stelle fixiert werden. Es kann beispielsweise ein Punkt aus Klebstoff zu einer Fixierung vorgesehen sein. Alternativ kann der Faden oder das Band auch als Ring oder Schlinge geformt sein, also insbesondere als geschlossener Ring ohne eigentliches Ende, sodass auf eine weitere Fixierung zu den Spannungslaschen verzichtet werden kann. Der Ring aus dem Faden oder dem Band kann dabei direkt als Ring hergestellt sein oder durch Knüpfen hergestellt sein. Der Papierwerkstoff des materiellen Griffkörpers wird bei einer Herstellung insbesondere gesiegelt, wobei die Spannungslaschen mit eingesiegelt werden. Es ist insbesondere auch denkbar, dass verschiedene Verankerungstechniken kombiniert werden, wie beispielsweise Schweißen und Oberflächenstrukturen, Schlitzen und Schweißen, Spannungslaschen und Schweißen, Spannungslaschen und Kleben oder auch Spannungslaschen, Oberflächenstrukturen und Schweißen. Es sind dabei insbesondere verschiedene, einem Fachmann als sinnvoll erscheinende Verbindungstechniken und/oder Kombinationen von Verbindungstechniken denkbar. Mittels der Verbindungstechnik muss jedoch insbesondere ein Ausreißen des Fadens oder des Bands in der Anwendung verhindert werden. Vorzugsweise kann durch einen entsprechenden Aufbau insbesondere erreicht werden, dass der Flosser direkt mit der Papier- oder Kartonsammlung entsorgt werden kann.

Das Auszugsgewicht des Fadens oder des Bandes beträgt senkrecht zur Längsausdehnung des Fadens / des Bandes gemessen mindestens 10N.

Als Körperpflegeprodukt mit einem entsprechenden materiellen Grundkörper wäre insbesondere eine Interdentalbürste denkbar. Hierzu kann insbesondere zu einer Herstellung in einem ersten Schritt eine Interdentalbürstenanwendungseinheit bereitgestellt werden. Anschließend oder parallel kann ein flächiger Papierwerkstoff in Form eines Papierbogens oder Papiernutzens bereitgestellt werden. Darauffolgend wird die Interdentalbürstenabwendungseinheit auf den Papierbogen gelegt, welcher zumindest eine erste Schicht ausbildet, und anschließend ein weiterer Papierbogen, welcher die zweite Schicht ausbildet, auf den ersten Papierbogen gelegt. Anschließend werden die Schichten verbunden z.B. verschweißt, gesiegelt, laminiert oder verleimt und der materielle Griffkörper abgestanzt und/oder bei Gruppen von Produkten als Nutzen teilweise abgestanzt und teilweise perforiert. Die Produkte können, insbesondere, wenn sie als Interdentalbürste gestaltet sind, auch auf einem längeren Nutzen, bzw. Streifen angeordnet sein und über eine perforierte Stelle verbunden sein. Es werden 2 bis 24, vorzugsweise 4 bis 16, besonders bevorzugt 6 bis 12 Interdentalbürsten pro Nutzen vorgesehen. Der Streifen kann in einer anderen Ausgestaltungsvariante auch länger als Band gerollt oder flächig gefaltet werden, sodass dieser in eine Umverpackung mit Schlitz eingebracht werden kann, welche als Spender dient. Dabei kann jeweils ein Produkt rausgezogen bzw. abgewickelt und abgetrennt werden. Die übrigen Produkte bleiben so geschützt im Spender zurück.

Optional wäre denkbar, dass ein Standfuß durch Auseinanderklappen der Schichten des flächigen Papierwerkstoffs bereitgestellt werden kann. Die Schichten können dazu insbesondere von einem Endkunden mit Perforation getrennt und/oder einer Schlitzung auseinandergebogen werden. Alternativ können die Schichten bereichsweise unverbunden ausgebildet sein, sodass ein Endkunde die Schichten bereichsweise auseinanderziehen kann. Diese Lösungen können zur Ausbildung eines Standfußes genutzt werden und/oder zur wie bereits erwähnten Trennung der Schichten, um die Interdentalbürste vom Papierwerkstoff für die Entsorgung materialseitig zu trennen. Damit kann der Papierwerkstoff in der Papier-Sammlung und die Anwendungseinheit (Interdentalbürste) im Haushaltsmüll getrennt entsorgt werden.

Optional können vorstehende Stellen und oder Verdünnungen der Schichten durch Materialverdrängung des Papierwerkstoffs, welche beim Verbinden der Schichten durch den verdrillten Draht entstehen, durch Einbringen einer Zwischenlage vermieden werden, wobei der verdrillte Draht in der Zwischenlage in einer entsprechend vorgestanzten Ausnehmung liegt. Die Zwischenlage bedeckt die obere und untere Schicht dabei insbesondere nicht komplett.

Optional kann das verdrillte Ende der Bürste verformt werden, um die Auszugskraft des verdrillten Drahtes aus dem Papierwerkstoff zu erhöhen. Insbesondere kann dazu das Ende der eingedrehten Bürste mit einer Verformung versehen sein. Hierdurch kann ein Verbessern des Haltens im Papierwerkstoff sowie ein Verteilen der Kraft in der Anwendung erreicht werden. Die Verformung der Anwendungseinheit erfolgt insbesondere quer zur Längsrichtung, beispielsweise in L-Form oder S-Form, in Wellenform oder kleiderbügelartig, wobei die Verformung auf zwei Seiten der eingedrehten Bürste verläuft. Die Verformung des verdrillten Drahtes erhöht die Auszugskraft aus dem Griffkörper. Die Auszugskraft des verdrillten Drahtes aus dem Griffkörper aus Papierwerkstoff liegt zwischen 1.5 kg und 6 kg vorzugsweise im Bereich 3 kg bis 4.5 kg.

Bei einer Ausbildung des Körperpflegeprodukts als Interdentalreiniger wäre ferner denkbar, dass die Anwendungseinheit in einem Spritzgussprozess oder Extrusionsprozess aus Kunststoff hergestellt wird, wobei ein Aufbau und/oder eine Herstellung ansonsten dem Aufbau und/oder der Herstellung des Körperpflegeprodukts bei einer Ausbildung als Interdentalbürste entspricht. Optional kann die Anwendungseinheit des Interdentalreinigers eine definierte Schnittstellenstruktur sein, welche Mittel zur Verankerung zwischen den flächigen Papierwerkstoffschichten aufweist. Zudem kann die Anwendungseinheit des Interdentalreinigers optional zwischen zwei Schichten aus Papierwerkstoff eingelegt oder auf Schichten aus Papierwerkstoff montiert werden.

Bei einer Ausbildung des Körperpflegeprodukts als Interdentalreiniger wäre ferner denkbar, dass die Anwendungseinheit aus einem Papierwerkstoff, insbesondere aus einem gerollten Papierwerkstoff, aus einem organischen Material oder aus Holz oder Bambus besteht. Ansonsten entspricht der Aufbau und/oder die Herstellung des Körperpflegeprodukts wie oben beschrieben der Ausbildung als Interdentalbürste. Ferner wäre denkbar, dass die Anwendungseinheit des Körperpflegeprodukts an dem materiellen Grundkörper montiert ist. Als Körperpflegeprodukt wäre hierzu beispielsweise ein Flosser mit einem entsprechenden materiellen Grundkörper denkbar, wobei der Faden der Zahnseide mit zumindest einer Schicht des materiellen Grundkörpers vernäht ist. Dabei ist insbesondere denkbar, dass der Zahnseidefaden auf die erste Schicht aufgenäht ist, wobei der Zahnseidefaden als Nähmedium verwendet wird. Alternativ oder zusätzlich kann der Zahnseidefaden angenäht sein, wobei ein weiterer Faden genutzt wird, um den Zahnseidefaden zu befestigen. Vorzugsweise sind bei einer entsprechenden Verbindung insbesondere von 1 bis 6, vorzugsweise von 1 bis 3, Schichten vorgesehen. Der Papierwerkstoff der Schichten ist insbesondere in einer Stärke bereitgestellt, welche sich direkt für die Nutzung eignet, sodass keine weiteren Verbindungsprozesse nötig sind, um den Träger zu formen. Die Schichten weisen insbesondere eine Materialstärke von 0,5 mm bis 2 mm, vorzugsweise von 0,5 mm bis 1,5 mm, auf, bei einer Dichte von 180 g/m² bis 500 g/m², vorzugsweise 240 g/m² bis 400 g/m², insbesondere damit eine Verbindung mittels Nähen realisierbar ist. Bei einer Herstellung eines Körperpflegeprodukts, insbesondere bei einer Ausbildung als Flosser, kann daher insbesondere die Anwendungseinheit auf den materiellen Griffkörper aufgenäht werden. Hierzu wird in einem ersten Verfahrensschritt insbesondere ein Bogen aus Papierwerkstoff in Form der ersten Schicht zumindest teilweise gestanzt. Es werden insbesondere zumindest die Bereiche des materiellen Griffkörpers gestanzt, in welchen die Anwendungseinheit angeordnet wird. Es werden insbesondere die Bereiche gestanzt, die nach Montage des Zahnseidefadens der Anwendungseinheit nicht mehr gestanzt werden können. Anschließend wird der gestanzte Bogen bereitgelegt und der Zahnseidefaden bereitgestellt und angenäht. Das Nähen kann entweder mit der Zahnseide selbst oder mit einem zusätzlichen Faden erfolgen. Bei einem Verwenden des Zahnseidefadens als Nähmedium wird der Zahnseidefaden insbesondere an den beiden Enden, insbesondere an den Armen des materiellen Griffkörpers, vernäht, wobei der zwischen den Enden gespannte Bereich des Zahnseidefadens als Anwendungsbereich dient. Optional können insbesondere weitere Schichten aus einem Papierwerkstoff vorgesehen sein. Beispielsweise kann insbesondere eine weitere Teilschicht auf eine erste Teilschicht verbunden oder gefaltet werden, um mehrere Lagen zu schaffen. Dazu kann der materielle Griffkörper insbesondere ein Scharnier oben auf den Armen aufweisen. Hierdurch können insbesondere Verletzungen vermieden werden. Ferner wäre denkbar, dass der Stich des Nähens als optisches Gestaltungselement genutzt wird. Alternativ oder zusätzlich wäre denkbar, dass das Laminieren der Schichten zusätzlich als Abschluss genutzt wird. Alternativ oder zusätzlich wäre denkbar, dass ein Befestigen der Anwendungseinheit mittels Klebstoffs oder durch Anspritzen in einem Spritzguss erfolgt. Mittels der Verbindungstechnik muss jedoch insbesondere ein Ausreißen des Fadens in der Anwendung verhindert werden. Durch das Annähen ist insbesondere nur ein Stanzschritt nötig, wobei insbesondere ein Verstecken der Fadenenden der Zahnseide schwierig ist.

Es ist insbesondere eine Option denkbar, bei welcher die Anzahl der Schichten variiert. Beispielsweise können im Bereich der Verankerung, wo eine bestimmte Stabilität erreicht werden muss, mehr Schichten vorhanden sein als am restlichen Griff - oder auch umgekehrt. Nicht jede Schicht muss zwingend über den ganzen Bereich der angrenzenden Schicht reichen. Eine reduzierte Schicht kann außen liegen oder zwischen größeren Schichten angeordnet sein. Durch das Anordnen zwischen den Schichten kann eine Beschädigung durch Wegreißen vermieden werden. Es bestehen insbesondere keine außenliegenden störenden Kanten, von welchen eine Verletzungsgefahr ausgehen kann.

Es wird weiter vorgeschlagen, dass der materielle Griffkörper der Griffeinheit zumindest eine Schicht aus einem Papierwerkstoff aufweist, die zumindest teilweise gefaltet ausgebildet ist. Dadurch kann insbesondere vorteilhaft ein Griffkörper hergestellt werden. Es kann insbesondere ein vorteilhaft stabiler und leicht herzustellender Griffkörper bereitgestellt werden. Es kann insbesondere eine vorteilhafte Griffform bereitgestellt werden. Vorzugsweise ist die Schicht aus einem zusammenhängenden, insbesondere zugeschnittenen Papier und/oder Karton hergestellt. Die Schicht umfasst vorteilhaft zumindest eine Faltachse und/oder zumindest eine Faltkante. Die Schicht ist in einem Gebrauchszustand vorzugsweise an der zumindest einen Faltachse und/oder der zumindest eine Faltkante gefaltet. Vorteilhaft wird durch die zumindest eine Faltachse und/oder Faltkante ein Falten der Schicht in einen Gebrauchszustand erleichtert. Es wäre denkbar, dass die Schicht in der Faltkante und/oder Faltachse perforiert und/oder geprägt ausgebildet ist, um ein definiertes Falten zu ermöglichen. Vorzugsweise ist die Schicht durch die zumindest eine Faltachse in zwei Seiten, insbesondere Teilschichten, unterteilt. Vorzugsweise verläuft die zumindest eine Faltachse und/oder Faltkante parallel zur Haupterstreckungsrichtung des Körperpflegeprodukts. Denkbar ist auch, dass zumindest eine Faltachse senkrecht zur Haupterstreckungsrichtung des Körperpflegeprodukts verläuft. Der materielle Griffkörper kann dabei insbesondere aus Papierbögen oder ab Rolle gefaltet werden. Ferner kann eine Verbindung von Faltseiten insbesondere mittels Siegelung oder Klebung erfolgen. Als Vorbehandlung ist beispielsweise ein Prägen denkbar, mit dem Ziel eine Struktur auf der Oberfläche des Endprodukts zu erzeugen. Alternativ oder zusätzlich ist als Vorbehandlung ein Beschichten denkbar. Beispielsweise könnte eine Innenseite einer Schicht zu einem Kleben vorgesehen sein, während eine Außenseite einen Schutz gegen Feuchtigkeit aufweist. Die Schicht kann daher zwei Seiten mit unterschiedlichen Vorbehandlungen, wie beispielsweise Beschichtungen, aufweisen. Alternativ oder zusätzlich ist als Vorbehandlung eine Bedruckung denkbar. Des Weiteren ist denkbar, dass die zumindest eine Schicht nach einer Herstellung nachbehandelt wird. Als Nachbehandlung ist beispielsweise ein Beschichten, ein Bedrucken und/oder ein Laminieren denkbar. Vorzugsweise wird die Schicht des materiellen Griffkörpers geschnitten oder gestanzt.

Als Körperpflegeprodukt mit einem entsprechenden materiellen Grundkörper wäre insbesondere eine Zahnbürste denkbar. Das Körperpflegeprodukt ist insbesondere flach hergestellt, wobei insbesondere Rillen zu einem Falten vor dem ersten Gebrauch vorgesehen sind. Die Rillen sind insbesondere in der Faltachse angeordnet. Zudem wäre eine Kombination aus Falten und einem anschließenden Verbinden, wie beispielsweise mittels Kleben, Siegeln, Schweißen, Leimen, Laminieren denkbar. Bei einem als Zahnbürste ausgebildeten Körperpflegeprodukt könnte das Körperpflegeprodukt mit Origami-Anlehnung ein Fachwerk aus Papier bilden. Alternativ oder zusätzlich könnte das Körperpflegeprodukt bei einer Ausbildung als Zahnbürste Reinigungselemente aus einem Papierwerkstoff umfassen.

Als Körperpflegeprodukt mit einem entsprechenden materiellen Grundkörper wäre insbesondere ein Zahnstocher oder Interdentalreiniger denkbar. Das Körperpflegeprodukt ist insbesondere aus einem flächigen Papierwerkstoff hergestellt, wobei insbesondere Rillen zu einem Falten vor dem ersten Gebrauch vorgesehen sind. Die Rillen sind insbesondere in der Faltachse angeordnet. Das Körperpflegeprodukt kann im Gebrauch insbesondere zu einem Dreieck bzw. pyramidenförmigen oder kegelförmigen Gebilde gefaltet werden. Der Papierwerkstoff, welcher für die Herstellung eines Körperpflegeprodukts in der Form eines Zahnstochers vorzugsweise genutzt wird, ist ein Transparentpapier. Dieses Transparentpapier ist vorzugsweise aus neuem Zellstoff (holzfrei) hergestellt. Für die genannte Anwendung hat das Transparentpapier ein Flächengewicht von 150 g/m2 bis 300 g/m2, vorzugsweise von 200 g/m2 bis 250 g/m2. Optional könnte bei einem gefalteten Körperpflegeprodukt zudem innen ein Fachwerk aus Papierwerkstoff vorgesehen sein, während außen eine Hülle das Fachwerk umgibt.

Erfindungsgemäß wird vorgeschlagen, dass die Anwendungseinheit zumindest einen Reinigungselementträger, insbesondere Draht, und mehrere mit dem Reinigungselementträger verbundene, insbesondere eingedrehte, Reinigungselemente aufweist, wobei der Reinigungselementträger zu einer Verbindung mit der Griffeinheit mit einem den Reinigungselementen abgewandten Verbindungsabschnitt mit dem materiellen Griffkörper verbunden ist. Dadurch kann insbesondere eine vorteilhafte Verbindung des Körperpflegeprodukts bereitgestellt werden. Es kann insbesondere eine Anzahl an Elementen des Körperpflegeprodukts gering gehalten werden. Vorzugsweise sind die Reinigungselemente von Borsten gebildet. Bevorzugt ist der Reinigungselementträger direkt mit den Reinigungselementen gekoppelt und direkt mit dem materiellen Griffkörper gekoppelt. Der Reinigungselementträger ist insbesondere einstückig, vorzugsweise einteilig, ausgebildet. Unter "einteilig" soll insbesondere in einem Stück geformt verstanden werden. Vorzugsweise wird dieses eine Stück aus einem einzelnen Rohling, einer Masse und/oder einem Guss, besonders bevorzugt in einem Spritzgussverfahren, insbesondere einem Ein- und/oder Mehrkomponenten-Spritzgussverfahren, hergestellt. Besonders bevorzugt ist der Reinigungselementträger in einem Verbindungsabschnitt insbesondere direkt mit einem Papierwerkstoff des materiellen Griffkörpers verbunden.

Des Weiteren wird vorgeschlagen, dass der Verbindungsabschnitt des Reinigungselementträgers der Anwendungseinheit in den materiellen Griffkörper ragt und von diesem umschlossen ist. Es wird insbesondere vorgeschlagen, dass der zumindest eine Reinigungselementträger der Anwendungseinheit an einem den Reinigungselementen abgewandten Verbindungsabschnitt in zumindest eine Schicht des materiellen Griffkörpers eingerollt ausgebildet ist. Dadurch kann insbesondere eine vorteilhafte Verbindung zwischen der Anwendungseinheit und der Griffeinheit bereitgestellt werden. Alternativ kann der Reinigungselementträger der Anwendungseinheit an einem den Reinigungselementen abgewandten Verbindungsabschnitt in einen Hohlraum des materiellen Griffkörpers eingeführt werden. Der Hohlraum kann bei der Herstellung des materiellen Griffkörpers mittels Rollen, Prägen etc. vorgesehen sein oder mit mechanischen Mitteln erzeugt werden, d.h. der Hohlraum wird mittels Verdrängen, (Dorn) Bohren oder dergleichen erzeugt. Vorzugsweise ist die Anwendungseinheit über den Verbindungsabschnitt direkt mit dem materiellen Griffkörper verbunden. In einer Variante kann die Anwendungseinheit in einem Verbindungsabschnitt während des Rollens des materiellen Griffkörpers mit dem materiellen Griffkörper verbunden werden. Der Verbindungsabschnitt der Anwendungseinheit wird dabei insbesondere in den materiellen Griffkörper eingerollt. In einer zweiten Variante kann die Anwendungseinheit in dem Verbindungsabschnitt nach dem Rollen des materiellen Griffkörpers mit dem materiellen Griffkörper verbunden werden. Die Anwendungseinheit kann dabei insbesondere durch Einstecken und/oder Aufstecken auf den materiellen Griffkörper mit dem materiellen Griffkörper verbunden werden. Wie erwähnt, ist es möglich den gerollten Griffkörper vorher mechanisch zu bearbeiten, um den nötigen Freiraum für den Verbindungsabschnitt zu schaffen. In einer dritten Variante kann die Anwendungseinheit zumindest teilweise ein Teil des gerollten Bereichs des materiellen Griffkörpers ausbilden. Die Anwendungseinheit kann dabei beispielsweise von einer Interdentalbürste, einem Zahnbürstenkopf, einem Zahnstocher gebildet sein.

Die Fixierung des Verbindungsabschnitts in der Anwendungseinheit wird mit bekannten Verfahren wie Schweißen, Siegeln, Leimen, Pressen, Laminieren etc. vollzogen.

Alternativ oder zusätzlich kann der Verbindungsabschnitt der Anwendungseinheit als eine Schnittstelle für ein Wechselteil, wie beispielsweise einen Wechselkopf für eine manuelle Zahnbürste, ausgebildet sein.

Der materielle Griffkörper kann insbesondere sowohl gerade gerollt sein als auch spiralförmig gerollt sein. Es ist auch denkbar, dass der materielle Grundkörper wie ein Papierwischer, insbesondere eine sogenannte Estompe, gerollt ist. Der als Rolle ausgestaltete materielle Griffkörper weist insbesondere einen (größten) Durchmesser von 1 mm bis 25 mm, vorzugsweise 1.5 mm bis 20 mm, besonders bevorzugt 2 bis 18mm auf.

Als Körperpflegeprodukt mit einem entsprechenden materiellen Grundkörper wäre insbesondere eine Interdentalbürste vorteilhaft. Dabei kann insbesondere das Papier während dem Eindrehen der Bürste ebenfalls gleich gerollt werden. Hierdurch könnte insbesondere erreicht werden, dass direkt fertige Interdentalbürsten aus der Maschine kommen. Ein entsprechender Verfahrensablauf zur Herstellung könnte dabei in einer Variante derart erfolgen, dass in einem ersten Schritt die Anwendungseinheit in Form einer Interdentalbürste durch Eindrehen hergestellt wird. Anschließend wird in einem zweiten Verfahrensschritt die zumindest eine Schicht des materiellen Griffkörpers gerollt und gleichzeitig ein Verbindungsabschnitt der Anwendungseinheit in den materiellen Griffkörper eingerollt. Eine Verankerung der Anwendungseinheit in dem materiellen Griffkörper kann insbesondere durch Klebstoff, Schweißen oder Verpressen erfolgen. Hierfür kann allenfalls ein weiterer Verfahrensschritt nötig sein. In einer zweiten Variante zur Herstellung des Körperpflegeprodukts wird in einem ersten Schritt die Anwendungseinheit in Form einer Interdentalbürste durch Eindrehen hergestellt. Parallel dazu wird der materielle Griffkörper durch Rollen der zumindest einen Schicht hergestellt. Anschließend wird in einem zweiten Schritt der materielle Griffkörper zu einer Verbindung mit der Anwendungseinheit vorbereitet. Hierzu wird beispielsweise ein Loch im materiellen Griffkörper erstellt. Dazu kann der Griffkörper stirnseitig gebohrt werden oder das Material kann mit einem Dorn verdrängt werden. Es wäre jedoch auch denkbar, dass das Loch bereits bei der Herstellung des materiellen Griffkörpers, beispielsweise indem ein Leerraum gelassen wird (Hohlzylinder), geschaffen wird, wobei hier insbesondere auf den zweiten Schritt (mechanische Ausbildung eines Lochs) verzichtet werden könnte. Darauffolgend wird die Anwendungseinheit in einem weiteren Verfahrensschritt in einer Ausnehmung in dem materiellen Griffkörper verankert, wie beispielsweise durch Einkleben, Schweißen, Verpressen, Siegeln oder dergleichen. In einer dritten Variante zur Herstellung des Körperpflegeprodukts wird in einem ersten Schritt die Anwendungseinheit in Form einer Interdentalbürste durch Eindrehen hergestellt. Parallel dazu wird der materielle Griffkörper durch Rollen der zumindest einen Schicht hergestellt, wobei der materielle Griffkörper insbesondere als hohles Rohr hergestellt wird. Anschließend wird die Anwendungseinheit in einem weiteren Verfahrensschritt in der Ausnehmung in dem materiellen Griffkörper verankert, wie beispielsweise durch Einkleben, Schweißen, Verpressen oder dergleichen. Die Anwendungseinheit kann insbesondere direkt in das zentrale Loch des Rohrs gesteckt und verankert werden.

Optional kann der materielle Griffkörper bereits aus einem bedruckten Papierwerkstoff hergestellt sein. Hierdurch könnte ein farbiges Endprodukt ohne Nachbearbeitung geschaffen werden.

Alternativ oder zusätzlich wäre denkbar, dass die Stielenden des materiellen Griffkörpers verschlossen werden. Durch ein Verschließen der offenliegenden Seiten kann insbesondere ein dichter Abschnitt des materiellen Griffkörpers bereitgestellt werden, damit Wasser nicht in die Kanten des Papierwerkstoffs eindringen kann. Hierfür könnte insbesondere auch ein montierter Abdeckteil bzw. Deckel oben und/oder unten bereitgestellt werden. Das Abdeckteil kann ein Bestandteil der Anwendungseinheit bzw. des Verbindungsabschnittes sein. Alternativ oder zusätzlich wäre auch denkbar, dass die Enden des materiellen Griffkörpers verpresst sind oder beispielsweise mit wasserabweisenden Mitteln wie einem Wachs, Lack oder dergleichen behandelt bzw. versiegelt werden. Bei einer Versiegelung wäre beispielsweise denkbar, dass bereits ein Füllstoff, wie beispielsweise Wachs, auf dem Papierwerkstoff vorgesehen ist und zu einer Versiegelung lediglich die Enden des materiellen Griffkörpers erhitzt werden müssen. Als Schwierigkeit könnte insbesondere das Schaffen der stabilen Schnittstelle zwischen Papierwerkstoff und der Anwendungseinheit angesehen werden.

Bei einer Ausbildung des Körperpflegeprodukts als Interdentalreiniger wäre insbesondere ferner denkbar, dass die Anwendungseinheit in einem Spritzgussprozess hergestellt ist, wobei ein Aufbau und/oder eine Herstellung ansonsten dem Aufbau und/oder der Herstellung des Körperpflegeprodukts bei einer Ausbildung als Interdentalbürste entspricht.

Erfindungsgemäß wird vorgeschlagen, dass der zumindest eine Reinigungselementträger der Anwendungseinheit zu einer zumindest teilweise formschlüssigen Verbindung mit der Griffeinheit in dem Verbindungsabschnitt gebogen ausgebildet ist. Vorzugsweise ist der zumindest eine Reinigungselementträger der Anwendungseinheit zu einer zumindest teilweise formschlüssigen Verbindung mit der Griffeinheit in dem Verbindungsabschnitt zwischen zwei Schichten des materiellen Griffkörpers, welche insbesondere geschichtet miteinander verbunden sind, gebogen ausgebildet. Dadurch kann insbesondere ein Ausreißen des Reinigungselementträgers vermieden werden. Es kann insbesondere ein vorteilhaft stabiles Körperpflegeprodukt bereitgestellt werden. Als Körperpflegeprodukt mit einer entsprechenden Verbindung wäre insbesondere eine Interdentalbürste denkbar. Insbesondere kann dazu das Ende der eingedrehten Bürste mit einer Verformung versehen sein. Hierdurch kann ein Verbessern des Haltens im Papierwerkstoff sowie ein Verteilen der Kraft in der Anwendung erreicht werden. Die Verformung der Anwendungseinheit erfolgt insbesondere quer zur Längsrichtung, beispielsweise in L-Form, S-Form in Wellenform oder kleiderbügelartig, wobei die Verformung auf zwei Seiten der eingedrehten Bürste verläuft. Alternativ oder zusätzlich wäre denkbar, dass der Verbindungsabschnitt durch den gesamten materiellen Griffkörper verläuft, wobei der Griffkörper durch den Verbindungsabschnitt, insbesondere bei einer Ausbildung aus Draht, in eine gewünschte Form gebogen werden kann. Dabei ist es möglich, den Verbindungsabschnitt als formgebendes Element bzw. Einstellelement zu nutzen, beispielsweise um der Griffeinheit eine nicht flächige bzw. eine drei-dimensionale Form geben zu können, z.B. eine Wölbung, eine Verdrehung, einen Winkel etc.. Alternativ oder zusätzlich wäre denkbar, dass mittels des Verbindungsabschnitts eine Drahtschlaufe in dem Griffkörper gelegt wird, durch welche eine ringförmige Erhöhung auf dem Griffkörper entsteht, die beispielsweise als Daumengriff verwendet werden kann.

Ferner wird vorgeschlagen, dass der zumindest eine Reinigungselementträger der Anwendungseinheit zumindest teilweise stoffschlüssig, insbesondere mittels Klebung, Sieglung, Laminierung und/oder Schweißung, mit dem materiellen Griffkörper verbunden ist. Dadurch kann insbesondere ein Ausreißen des Reinigungselementträgers vermieden werden. Es kann insbesondere ein vorteilhaft stabiles Körperpflegeprodukt bereitgestellt werden.

Des Weiteren wird vorgeschlagen, dass die Anwendungseinheit zumindest ein einen Papierwerkstoff umfassendes Reinigungselement aufweist. Das Reinigungselement kann dabei insbesondere sowohl von einzelnen Borsten oder einem Borstenbündel gebildet sein, als auch von einem Zahnseidefaden, einem Zahnseideband, insbesondere einem Zahnzwischenraumtape, von einer Folie oder dergleichen gebildet sein. Es sind verschiedene, einem Fachmann als sinnvoll erscheinende Ausbildungen des Reinigungselements denkbar. So könnten insbesondere die Griffeinheit und die Anwendungseinheit aus demselben Papierwerkstoff bzw. Papierbogen hergestellt werden. Durch Falten, Rollen und Fixieren kann eine massige Griffeinheit und ein lockeres Borstenfeld der Anwendungseinheit hergestellt werden. Alternativ kann die Anwendungseinheit geschichtet und geklebt sein, wobei die Reinigungselemente aus einem Bogen ausgestanzt sind. Die Reinigungselemente können insbesondere aus mechanisch bearbeitetem Papierwerkstoff bestehen, z.B. kann der Papierwerkstoff geschlitzt, geschnitten, vereinzelt, gestanzt, gepresst, verdichtet etc. sein. Allfällige Schlitze im Papierwerkstoff können mit oder ohne Abstand ausgebildet sein. Es ist jedoch insbesondere ein seitlicher Abstand für eine seitliche Bewegungsmöglichkeit der Reinigungselemente vorgesehen. Hierfür sind insbesondere verschiedene geschlitzte und ungeschlitzte Schichten bzw. auch reduzierte Schichten übereinander vorgesehen. Ferner wäre auch eine Hybrid-Lösung denkbar, beispielsweise aus Papier mit Kunststoff, wie dies beispielsweise bei Joghurt-Bechern bereits vorgesehen ist. Es wäre insbesondere auch eine Variante mit innen Papier und außen Kunststoff denkbar.

Dadurch kann insbesondere ein vorteilhaft ökologisches Körperpflegeprodukt bereitgestellt werden. Es kann insbesondere eine vorteilhaft einfache Entsorgung des Körperpflegeprodukts erreicht werden.

Zudem wird vorgeschlagen, dass das zumindest eine Reinigungselement zumindest teilweise von einer Folie oder Band gebildet ist, diese kann als Zahnzwischenraumreinigungsreiniger ausgestaltet sein. Das Reinigungselement ist insbesondere von einem Band bzw. Zahnzwischenraumtape gebildet. Vorzugsweise ist die Anwendungseinheit, insbesondere das Reinigungselement, von einem Zahnzwischenraum-Reiniger aus einer folienartigen Struktur, wie beispielsweise auf Basis von Kunststoff und/oder Papier, gebildet. Als Grundwerkstoff kann hierbei insbesondere ein reißfestes Papier, eine Kunststoff-Folie, wie beispielsweise eine Kunststoff-Monofolie oder -Verbundfolie, also insbesondere mehrere verbundene Monofolien, ein nachhaltiges und/oder abbaubares Material, wie beispielsweise PLA, und/oder ein vliesartiger Stoff, wie beispielsweise Tyvek von duPont, eingesetzt werden. Der Zahnzwischenraum-Reiniger wird dabei verarbeitet wie die bereits oben ausgeführte Zahnseide. Der Zahnzwischenraum-Reiniger wird insbesondere zwischen Schichten des materiellen Griffkörpers mit der Griffeinheit verbunden. Ein Produktaufbau kann dabei insbesondere zumindest abschnittsweise zumindest drei Schichten aufweisen, eine erste Schicht aus einem Papierwerkstoff, eine zweite Schicht, welche von dem Zahnzwischenraum-Reiniger gebildet ist, und eine dritte Schicht aus einem Papierwerkstoff. Das Körperpflegeprodukt ist insbesondere nicht überall 3-lagig und/oder hat nicht überall gleich viele Lagen. Der Zahnzwischenraum-Reiniger ist insbesondere nur in einem Teilbereich eingebracht. Der Zahnzwischenraum-Reiniger kann insbesondere zudem mechanisch bearbeitet sein. Der Zahnzwischenraum-Reiniger kann mit einer Prägung versehen sein, insbesondere zu einer Erzeugung von Oberflächenstrukturen sowie einer Topographie. Der Zahnzwischenraum-Reiniger kann nutzungsseitig gefaltet sein. Ferner kann der Zahnzwischenraum-Reiniger mit Additiven versehen sein, wie beispielsweise antibakteriellen Mitteln, Reinigungsmitteln, wie insbesondere Mundhygienereinigungsmitteln, Geschmacksadditiven und/oder Abrasionsmitteln, wie beispielsweise einer Körnung zur Unterstützung der Reinigung. Mittels des Abrasionsmittels kann ferner wiederum eine Oberflächenstruktur erzeugt werden. Des Weiteren ist denkbar, dass der Zahnzwischenraum-Reiniger verschiedene Ränder mit verschiedenen Formen aufweist. Der Zahnzwischenraum-Reiniger weist insbesondere eine Dicke von 0,05 mm bis 0,5 mm, vorzugsweise von 0,15 mm bis 0,35 mm, auf. Ferner weist der Zahnzwischenraum-Reiniger insbesondere eine Breite oder Höhe von 0.5 mm bis 10 mm, vorzugsweise von 1 mm bis 6 mm, auf. Die Länge ist dem Freiraum in der Anwendungseinheit angepasst.

Ferner wird vorgeschlagen, dass die Anwendungseinheit zwischen den zumindest zwei Schichten des materiellen Griffkörpers an der Griffeinheit angeordnet ist. Die Anwendungseinheit ist insbesondere von einer Interdentalbürste, einer Zahnseide und/oder einem Zahnzwischenraum-Reiniger gebildet. Dadurch kann insbesondere ein vorteilhaft kompakter und stabiler Aufbau bereitgestellt werden.

Des Weiteren wird vorgeschlagen, dass das Körperpflegeprodukt eine Steckverbindungseinheit aufweist, welche zu einer lösbaren Steckverbindung der Anwendungseinheit, welche insbesondere als ein Zahnbürstenkopf ausgebildet ist, mit der Griffeinheit vorgesehen ist. Dabei wäre insbesondere auch denkbar, dass die Anwendungseinheit als ein Wechselkopf ausgebildet ist, wobei die Steckverbindungseinheit direkt zwischen der Anwendungseinheit und der Griffeinheit angeordnet ist. Die Steckverbindungseinheit ist insbesondere zumindest teilweise einstückig mit der Anwendungseinheit und/oder zumindest teilweise einstückig mit der Griffeinheit ausgebildet. Vorzugsweise weist die Steckverbindungseinheit zumindest ein erstes Steckverbindungselement auf, welches fest mit der Griffeinheit verbunden ist, und zumindest ein zu dem ersten Steckverbindungselement korrespondierendes zweites Steckverbindungselement auf, welches fest mit der Anwendungseinheit verbunden ist. Über die Steckverbindungseinheit kann insbesondere die Anwendungseinheit von dem materiellen Griffkörper getrennt werden. Die Steckverbindungseinheit ist insbesondere zu einer Bereitstellung einer lösbaren oder unlösbaren Rastverbindung vorgesehen. Das erste Steckverbindungselement bildet insbesondere einen Rastfortsatz aus, während das zweite Steckverbindungselement eine zu dem ersten Steckverbindungselement korrespondierende Rastausnehmung ausbildet. Es wäre jedoch auch eine getauschte und/oder eine andere, einem Fachmann als sinnvoll erscheinende Ausgestaltung der Steckverbindungseinheit denkbar.

Ferner wäre denkbar, dass der materielle Grundkörper der Griffeinheit zumindest teilweise aus mehreren Teilelementen aus einem Papierwerkstoff zusammengesteckt ist. Dazu wird insbesondere ein Papierwerkstoff in die Teilelemente geschnitten und anschließend die Teilelemente zusammengesteckt. In einem Grundverfahren wird ein Papierwerkstoff insbesondere in verschiedene Formen geschnitten, anschließend zusammengesteckt und allenfalls mit einer Sicherung/Verbindung versehen. Die Verbindung kann beispielsweise über Stecken, Kleben und/oder Schweißen erfolgen. Als Vorbehandlung ist beispielsweise ein Prägen denkbar, mit dem Ziel, eine Struktur auf der Oberfläche des Endprodukts zu erzeugen. Alternativ oder zusätzlich ist als Vorbehandlung ein Beschichten denkbar, wobei eine Außenseite einen Schutz gegen Feuchtigkeit aufweist. Alternativ oder zusätzlich ist als Vorbehandlung eine Bedruckung denkbar. Des Weiteren ist denkbar, dass die zumindest eine Schicht nach einer Herstellung nachbehandelt wird. Als Nachbehandlung ist beispielsweise ein Beschichten, ein Bedrucken und/oder ein Laminieren denkbar. Die Nachbehandlung kann nach dem Herstellen der Teilelemente oder nach dem Montieren der Teilelemente passieren. Es können insbesondere bereits beschriebene, insbesondere flache, Griffkörper, wie insbesondere die bereits beschriebenen Flosser, mit zumindest einem zusätzlichen Teilelement versehen werden, welches sich im Wesentlichen in eine weitere, insbesondere senkrecht zu einer Haupterstreckungsebene verlaufende, Richtung ausdreht und insbesondere einen 3D-Effekt erzeugt. Alternativ kann das Körperpflegeprodukt als Zahnbürste ausgebildet sein, wobei beispielsweise ein Kunststoffteil als Grundkörper mit Geometrien versehen ist, die die Kombination mit Papierwerkstoff-Stücken erlauben. Hierdurch kann insbesondere eine vorteilhaft dreidimensionale Griffeinheit bereitgestellt werden, wobei ein Materialaufwand gering gehalten werden kann. Vorzugsweise kann bei einer Zahnbürste die Anwendungseinheit mit einem Bürstenkopf und einer Hals-Verlängerung aus Kunststoff ausgebildet werden, an welche der materielle Griffkörper mit den Teilelementen angesteckt wird. Das Körperpflegeprodukt kann dabei insbesondere aus 2 bis 25, vorzugsweise 4 bis 12, Einzelteilen bestehen.

Bei einer Ausbildung des Körperpflegeprodukts als Flosser ist insbesondere denkbar, dass verschiedene flache Stücke aus Papierwerkstoff, insbesondere für die Griffeinheit, geschnitten werden und anschließend zu einer 3D-Kontur zusammengesteckt werden. Bei einer Herstellung werden insbesondere die Anwendungseinheit und die Teilelemente der Griffeinheit separat hergestellt. Ein Zusammenstecken des Körperpflegeprodukts kann dabei sowohl bereits bei einer Herstellung als auch durch einen Anwender selbst erfolgen. Bei einem Zusammenstecken durch den Anwender kann insbesondere eine Verpackungsgröße des Körperpflegeprodukts vorteilhaft gering gehalten werden. Es sind dabei insbesondere verschiedene Bauvarianten denkbar. Es kann insbesondere ein Netz aus Teilelementen ohne einen Kern gebildet werden, oder aber es ist ein Kern vorgesehen, auf welchen die Teilelemente aufgesteckt werden. Hierbei muss insbesondere eine nötige Stabilität der Verbindungen im Hinblick auf die Anwendung bereitgestellt werden. Es muss insbesondere ein Auseinanderfallen, beispielsweise mit Hilfe von Hinterschnitten, vermieden werden.

Bei einer Ausbildung des Körperpflegeprodukts als Interdentalreiniger wäre insbesondere denkbar, dass die Anwendungseinheit in einem Spritzgussprozess hergestellt ist, wobei die Anwendungseinheit auf einer zusammengesteckten Griffeinheit aus Papierwerkstoff aufgesteckt ist.

Ferner wird vorgeschlagen, dass das Körperpflegeprodukt zumindest teilweise aus einem Papierwerkstoff gefräst ist. Das Körperpflegeprodukt wird dabei insbesondere zumindest teilweise aus Vollpappe hergestellt, welche in eine definierte Form gefräst ist. Dabei ist insbesondere denkbar, dass die zumindest eine Vollpappe nach einer Fräsung nachbehandelt wird. Als Nachbehandlung ist beispielsweise ein Beschichten, ein Bedrucken und/oder ein Laminieren denkbar. Vorzugsweise besteht insbesondere zumindest die Griffeinheit aus Vollpappe. Es sind verschiedene, einem Fachmann als sinnvoll erscheinende Vollpappen denkbar, wie insbesondere Finnpappe. Als Körperpflegeprodukt wäre in diesem Zusammenhang insbesondere eine gestanzte Zahnbürste denkbar. Hierzu wird insbesondere ein Block aus einem Papierwerkstoff in eine Kontur gefräst, wobei im späteren Bürstenkopf Löcher gebohrt werden. Bei einer Herstellung wird insbesondere in einem ersten Schritt die Griffeinheit und Teile der Anwendungseinheit aus einem Block aus einem Papierwerkstoff gefräst und gebohrt. Anschließend kann die Griffeinheit und/oder die Anwendungseinheit optional bedruckt und lackiert werden. Darauffolgend werden Borstenbündel der Anwendungseinheit gestanzt, insbesondere eingesetzt und profiliert. Dabei muss insbesondere ein definiertes Abzugsgewicht der Borstenbündel bereitgestellt werden. Alternativ wäre denkbar, dass die Borstenbündel mittels AFT befestigt werden. Hierzu wird die Griffeinheit und Teile der Anwendungseinheit aus einem Block aus einem Papierwerkstoff gefräst. Im späteren Bürstenkopf der Anwendungseinheit wird eine Ausnehmung geschaffen. Parallel wird ein Borstenplättchen erstellt, welches dann in der Ausnehmung verankert werden kann. Bei einer Herstellung wird dazu insbesondere in einem ersten Schritt die Griffeinheit und Teile der Anwendungseinheit aus einem Block aus einem Papierwerkstoff gefräst, wobei direkt eine Ausnehmung in einem Bürstenkopf der Anwendungseinheit eingebracht wird. Anschließend kann die Griffeinheit und/oder die Anwendungseinheit optional bedruckt und lackiert werden. Darauffolgend wird ein Borstenplättchen spritzgegossen und mit Borsten versehen. Anschließend kann das fertige Borstenplättchen in den Bürstenkopf der Anwendungseinheit einsetzt und durch Verstemmen und/oder Kleben fixiert werden. Dabei muss insbesondere das Borstenplättchen zuverlässig mit dem Papierwerkstoff verbunden werden.

Des Weiteren wird vorgeschlagen, dass das Körperpflegeprodukt zumindest teilweise aus einem Papierwerkstoff gegossen ist. Der Papierwerkstoff wird dabei insbesondere in einem spitzgussähnlichen Prozess verarbeitet. Die Verarbeitung des Papierwerkstoffs wird jedoch bei deutlich tieferen Temperaturen und Spritzdrucken umgesetzt. Entsprechende Verfahren sind beispielsweise bei der PurePaperPak, einer konstruktiven Papierverpackung, bekannt. Dabei ist insbesondere ein ökologischer Rohstoffeinsatz möglich, wobei die Inhaltsstoffe ihren Ursprung in der Natur finden und insbesondere zu 100% aus natürlichen Ressourcen bestehen. Die Rohprodukte setzen sich insbesondere aus Industriestärke, vorzugsweise aus Kartoffeln, Papierfasern, Wasser, inklusive Zumischung zusammen. Die dadurch entstehende Papiermischung wird anschließend für die Weiterverarbeitung mittels Spritzgusstechnik verwendet. Der Papierspritzguss erfolgt insbesondere in drei Schritten. In einem ersten Schritt erfolgt eine Einspritzung, bei der die Papiermixtur in ein Aluminium-Werkzeug gespritzt wird. In einem zweiten Schritt erfolgt ein Backprozess, bei dem die Papiermixtur im Werkzeug gebacken, insbesondere erhitzt, wird. In einem dritten Schritt erfolgt eine Entformung, bei der die fertige Verpackung entnommen werden kann. Ferner sind bei Verpackungen sogenannte PAPACKS^{®}-Verpackungen bekannt. Die Verpackungen bestehen insbesondere aus lebensmittelzertifiziertem PAPACKS^{®}-Faserguss. Entsprechende Produkte sind insbesondere kompostierbar und auch in den Altpapier- oder Biomüll-Kreislauf bringbar. Dabei ist insbesondere denkbar, dass das gegossene Körperpflegeprodukt nach einem Guss nachbehandelt wird. Als Nachbehandlung ist beispielsweise ein Beschichten, ein Bedrucken und/oder ein Laminieren denkbar. Als Körperpflegeprodukt wäre in diesem Zusammenhang insbesondere eine gestanzte Zahnbürste denkbar. Ein Zahnbürstenkörper, insbesondere die Griffeinheit und Teile der Anwendungseinheit, werden dafür insbesondere aus einem Papierwerkstoff gegossen und, vorzugsweise analog zum heutigen Spritzguss, Borstenlöcher geformt. Bei einer Herstellung wird insbesondere in einem ersten Schritt die Griffeinheit und Teile der Anwendungseinheit aus einem Papierwerkstoff gegossen. Anschließend kann die Griffeinheit und/oder die Anwendungseinheit optional bedruckt und lackiert werden. Darauffolgend werden Borsten der Anwendungseinheit gestanzt, insbesondere eingesetzt und profiliert.

Es wird ferner vorgeschlagen, dass der Papierwerkstoff des Körperpflegeprodukts, insbesondere der Griffeinheit, zumindest abschnittsweise umspritzt ausgebildet ist. Der Papierwerkstoff kann dabei insbesondere gemäß einer der oben genannten Ausgestaltungen vorgesehen sein. Der Papierwerkstoff des Körperpflegeprodukts, insbesondere der Griffeinheit, ist insbesondere mit einem Kunststoff umspritzt. Eine Verbindung der Umspritzung zu dem Papierwerkstoff kann dabei insbesondere durch Hinterschnitte, Durchbrüche, also insbesondere einen Formschluss, oder chemisch, wie insbesondere durch einen Materialschluss, erreicht werden. Der Papierwerkstoff kann dazu insbesondere vorbehandelt ausgebildet sein, insbesondere vor dem Umspritzen, damit die Verbindung erstellt werden kann, beispielsweise mittels spezieller Lacke und/oder Beschichtungen. Eine Reinigungseinheit der Anwendungseinheit kann beispielsweise zumindest teilweise durch die Umspritzung geschaffen werden. Es kann insbesondere ein Lochfeld oder eine Ausnehmung für ein Borstenplättchen in einem Bürstenkopf der Anwendungseinheit mittels der Umspritzung geschaffen werden. Alternativ oder zusätzlich können gespritzte Borsten vorgesehen sein, welche direkt mittels der Umspritzung hergestellt sind. Als Körperpflegeprodukt wäre in diesem Zusammenhang insbesondere eine Zahnbürste denkbar. Hierzu wird insbesondere ein Kartonröhrchen umspritzt, wobei insbesondere direkt ein Bürstenkopf auf das Kartonröhrchen gespritzt wird. Bei einer Herstellung wird insbesondere in einem ersten Schritt ein Kartonröhrchen hergestellt, welches die Griffeinheit ausbildet. Anschließend wird das Kartonröhrchen umspritzt, wobei dabei insbesondere die Anwendungseinheit mit einem Lochfeld ausgebildet wird. Darauffolgend werden Borsten in das Lochfeld gestanzt, insbesondere eingesetzt und profiliert. Alternativ wäre als Körperpflegeprodukt in diesem Zusammenhang insbesondere ein Flosser denkbar. Hierzu wird insbesondere ein Kartonröhrchen umspritzt, wobei insbesondere direkt ein Flosserkopf auf das Kartonröhrchen gespritzt wird. Bei einer Herstellung wird insbesondere in einem ersten Schritt ein Kartonröhrchen hergestellt, welches die Griffeinheit ausbildet. Anschließend wird das Kartonröhrchen umspritzt, wobei dabei insbesondere die Anwendungseinheit in Form eines Flosserkopfs ausgebildet wird. Alternativ wäre als Körperpflegeprodukt in diesem Zusammenhang insbesondere ein Interdentalreiniger denkbar. Hierzu wird insbesondere ein Kartonröhrchen umspritzt, wobei insbesondere direkt ein Interdentalreiniger auf das Kartonröhrchen gespritzt wird. Bei einer Herstellung wird insbesondere in einem ersten Schritt ein Kartonröhrchen hergestellt, welches die Griffeinheit ausbildet. Anschließend wird das Kartonröhrchen umspritzt, wobei dabei insbesondere die Anwendungseinheit in Form eines Interdentalreinigers ausgebildet wird. Weiter kann eine Griffeinheit mit einer Schnittstelle für den Wechsel des Körperpflegeprodukts vorgesehen sein. Bei einer Herstellung wird insbesondere in einem ersten Schritt ein Kartonröhrchen hergestellt, welches die Griffeinheit ausbildet. Anschließend wird das Kartonröhrchen umspritzt, wobei dabei insbesondere eine Schnittstelle zu einer Anwendungseinheit ausgebildet wird. Die Anwendungseinheit wird mit dem Gegenstück der Schnittstelle ausgestattet und kann somit die verschiedensten Anwendungen umfassen, beispielsweise Flosser, Zahnbürsten, Interdentalreiniger.

Es wird ferner vorgeschlagen, dass das Körperpflegeprodukt zumindest einen funktionalen Teil aufweist, welcher mit einem Papierwerkstoff umspritzt ist. Der funktionale Teil kann dabei insbesondere von einem Kern der Griffeinheit und/oder der Anwendungseinheit gebildet sein, welcher in einem bekannten Verfahren, wie beispielsweise einem Kunststoff-Spritzgussverfahren, hergestellt ist. Eine Verbindung der Umspritzung zu dem funktionalen Teil kann dabei insbesondere durch Hinterschnitte, Durchbrüche, also insbesondere einen Formschluss, oder chemisch, wie insbesondere durch einen Materialschluss, erreicht werden. Der funktionale Teil kann dazu insbesondere vorbehandelt ausgebildet sein, insbesondere vor dem Umspritzen, damit die Verbindung erstellt werden kann, beispielsweise mittels spezieller Lacke und/oder Beschichtungen. Als Körperpflegeprodukt wäre in diesem Zusammenhang insbesondere ein Interdentalreiniger oder eine Zahnbürste denkbar. Hierzu wird insbesondere die Anwendungseinheit und/oder Teile der Griffeinheit in einem Kunststoffspritzguss hergestellt, wobei die Anwendungseinheit und/oder Teile der Griffeinheit teilweise mit einem Papierwerkstoff umspritzt sind.

Ferner wird vorgeschlagen, dass das Körperpflegeprodukt, insbesondere der materielle Griffkörper, zumindest eine Schicht aufweist, die zumindest teilweise verformt und/oder verpresst ist. Die zumindest eine Schicht kann insbesondere sowohl aus einem oder mehreren Bögen eines Papierwerkstoffs und/oder von Rollen abgerollt hergestellt werden. Das Körperpflegeprodukt weist insbesondere mehrere Schichten auf. Die Schichten, insbesondere des materiellen Griffkörpers, können beispielsweise mittels Kleben, Schweißen und/oder Laminieren miteinander verbunden sein. Ferner ist denkbar, dass die zumindest eine Schicht vor einer Verarbeitung vorbehandelt wird. Als Vorbehandlung ist beispielsweise ein Prägen denkbar, mit dem Ziel, eine Struktur auf der Oberfläche des Endprodukts zu erzeugen. Alternativ oder zusätzlich ist als Vorbehandlung ein Beschichten denkbar. Beispielsweise könnte eine Innenseite jeder Schicht zu einem Kleben vorgesehen sein, während eine Außenseite einen Schutz gegen Feuchtigkeit aufweist. Jede Schicht kann daher zwei Seiten mit unterschiedlichen Beschichtungen aufweisen. Alternativ oder zusätzlich ist als Vorbehandlung eine Bedruckung denkbar. Des Weiteren ist denkbar, dass die zumindest eine Schicht nach einer Herstellung nachbehandelt wird. Als Nachbehandlung ist beispielsweise ein Beschichten, ein Bedrucken und/oder ein Laminieren denkbar. Als Körperpflegeprodukt wäre in diesem Zusammenhang insbesondere ein Zahnstocher oder ein Flosser denkbar. Es ist jedoch auch eine alternative generelle Grundform für eine Griffeinheit entsprechend denkbar. Die zumindest eine Schicht des Körperpflegeprodukts wird insbesondere verformt, bevorzugt tiefgezogen, insbesondere mittels Drucks und Wärme. Hierdurch wird insbesondere beispielsweise eine Greifgeometrie für eine bessere Ergonomie erzeugt. Es kann insbesondere ein Körperpflegeprodukt mit einer voluminösen Griffeinheit bereitgestellt werden. Es wäre auch denkbar, dass die Griffeinheit aus zwei zusammengesetzten, tiefgezogenen Halbschalen besteht, die in einem miteinander verbundenen Zustand einen Hohlraum einschließen. Die Verbindung der Halbschalen kann beispielsweise mittels Kleben, Schweißen und/oder Stecken erfolgen. So kann insbesondere beispielsweise die Griffeinheit einer Zahnbürste hergestellt werden.

Optional kann insbesondere bereits bei einer Herstellung der Körperpflegeprodukte eine Gruppierung der Produkte erfolgen. Beispielsweise kann eine Gruppe von Flossern ähnlich wie heute bei Interdentalreinigern bereitgestellt werden, die bei einer Verwendung getrennt werden. Die Körperpflegeprodukte können insbesondere bereits über den Papierwerkstoff verbunden sein, wobei insbesondere eine Perforation existiert, an welcher die Körperpflegeprodukte getrennt werden können. Hierdurch kann insbesondere eine einfachere Verarbeitung erreicht werden, wobei eine Herstellung in Gruppen erfolgt und erst bei Gebrauch eine Separierung erfolgt.

Alternativ zu einem Rollen von Papierwerkstoff wäre insbesondere auch ein Extrudieren von Papierwerkstoff zum Schaffen einer zylindrischen Struktur denkbar. Alternativ zu einem Fräsen von Papierwerkstoff wäre insbesondere auch das Drehen von Papierwerkstoff zu einem Schaffen von rotationssymmetrischen Geometrien denkbar.

Ferner wäre denkbar, dass an dem Körperpflegeprodukt funktionale Schnittkanten und/oder Ränder an dem Papierwerkstoff vorgesehen sind. Es kann insbesondere Papier mit verschiedenen Rändern vorgesehen sein, wobei die Ränder funktional andere Eigenschaften aufweisen können. Eine Funktionsseite kann beispielsweise gewellt ausgebildet sein, während eine Seite ohne Funktion glatt ausgebildet ist. Es wäre jedoch auch eine umgekehrte Ausgestaltung denkbar. Es kann insbesondere eine nachträgliche Behandlung der Schnittkanten erfolgen, um beispielsweise eine Verletzungsgefahr oder eine Wasseraufnahme zu vermeiden. Es kann insbesondere eine mechanische Nachbearbeitung gegen die Scharfkantigkeit vorgesehen sein, wie insbesondere ein Brechen der Kanten. Ferner können auch Beschichtungen aus Wachs oder Lack vorgesehen sein.

Des Weiteren kann eine spezielle Oberfläche des Papierwerkstoffs geschaffen werden. Es können insbesondere auf verschiedenen Seiten unterschiedliche Oberflächen geschaffen werden, beispielsweise in Bezug auf Farbe oder Oberflächenbeschaffenheit.

Ferner wäre denkbar, dass Falten für Füße vorgesehen sind. Insbesondere kann der Papierwerkstoff am Produktende gefaltet werden, damit das Körperpflegeprodukt stehend gelagert werden kann. Es kann insbesondere ein Standfuß geschaffen werden.

Des Weiteren kann durch abschnittsweise Reduktion der Materialstärke eine Flexibilität geschaffen werden. Es können insbesondere Biegekanten geschaffen werden. Eine entsprechende Reduktion der Materialstärke kann beispielsweise durch Rillung bei flachen Varianten oder durch Perforation geschaffen werden.

Eine Verbindung mittels Schweißung muss gemäß der Offenbarung der Erfindung nicht über die ganze berührende Fläche erfolgen. So können beispielsweise Aufreißlaschen und/oder gewisse Randpositionen nicht geschweißt werden. Ferner kann eine Schweißung beispielsweise nur am Rand erfolgen, während Innenflächen ausgelassen werden. Des Weiteren wäre auch denkbar, dass Schweißungen ausgelassen werden, beispielsweise für das Schaffen von Beweglichkeit. Alternativ wäre auch denkbar, dass bewusst zur Schaffung von Beweglichkeit geschweißt wird, insbesondere im Bereich einer Faltkante. Alternativ oder zusätzlich kann auch nur im Bereich der Schnittstelle zur Anwendungseinheit geschweißt werden, beispielsweise nur bei dem Verbindungsabschnitt des Reinigungselementträgers.

Bei einer Ausbildung des Körperpflegeprodukts als Interdentalbürste ist die Griffeinheit insbesondere leichter als ein bekannter Kunststoffgriff. Eine Interdentalbürste mit einem gerollten oder geschichteten Griffkörper hat ein Gesamtgewicht von unter 2 g, vorzugsweise von unter 1 g, am meisten bevorzugt von unter 0,6g. Ein Flosser mit einem geschichteten Griffkörper hat ein Gesamtgewicht von unter 2 g, vorzugsweise von unter 1,2 g, am meisten bevorzugt von unter 0,8g. Der Schwerpunkt der Interdentalbürste mit gerolltem oder geschichtetem Griffkörper liegt vom unteren Ende her gemessen nach 40 % bis 70%, vorzugsweise nach 45 % bis 60 %, der Gesamtlänge.

Es wird ferner vorgeschlagen, dass der Papierwerkstoff eine wasserabweisende Beschichtung und/oder Imprägnierung aufweist. Durch die entsprechende Beschichtung und/oder Imprägnierung kann auch eine Verminderung der Wasseraufnahme des Papierwerkstoffs erreicht werden. Damit kann sich der Papierwerkstoff auch für einen Mehrfacheinsatz eignen. Eine Imprägnierung kann dabei beispielsweise mittels eines Kunststoffs und/oder eines Wachses oder dergleichen erfolgen.

Es wird weiter vorgeschlagen, dass der Papierwerkstoff lackiert und/oder bedruckt ausgebildet ist. Dadurch kann insbesondere eine vorteilhafte Individualisierung des Papierwerkstoffs erreicht werden. Auf einem Papierwerkstoff kann insbesondere eine vorteilhaft einfache Bedruckung ermöglicht werden. Es kann insbesondere eine Veredelung erreicht werden. Eine Veredelung kann beispielsweise eine Lackierung sein, dabei kann beispielsweise UV-Hochglanzlack, Strukturlack, Softtouch-Lack oder Glitterlack verwendet werden. Alternativ oder zusätzlich kann der Papierwerkstoff eine Bedruckung aufweisen. Der Papierwerkstoff kann beispielsweise eine Bedruckung auf wasserbasierten Lacken aufweisen, welche gegenüber konventionellen Lacken ökologisch sind.

Ferner geht die Erfindung aus von einem Verfahren zur Herstellung eines Körperpflegeprodukts. Es wird vorgeschlagen, dass der materielle Griffkörper der Griffeinheit auf zumindest eine Schicht aus einem Papierwerkstoff gerollt wird. Dadurch kann insbesondere ein vorteilhaft stabiler Griffkörper hergestellt werden. Vorzugsweise wird die Schicht aus dem Papierwerkstoff um eine definierte Rollachse aufgerollt. Die Rollachse verläuft vorzugsweise parallel, insbesondere koaxial, zu einer Längsachse der Griffeinheit. Bevorzugt wird die Schicht in einer Ebene senkrecht zu der Längsachse der Griffeinheit zumindest abschnittsweise, insbesondere vollständig, in einen spiralförmigen Querschnitt gerollt. Eine größte Fläche der Schicht erstreckt sich insbesondere um eine Längsachse der Griffeinheit. Vorzugsweise wird die zumindest eine Schicht des materiellen Griffkörpers in eine zylindrische Form gerollt.

Des Weiteren wird vorgeschlagen, dass die Anwendungseinheit in zumindest einem Verbindungsabschnitt in den materiellen Griffkörper eingerollt wird. Dadurch kann insbesondere eine vorteilhafte Verbindung zwischen der Anwendungseinheit und der Griffeinheit bereitgestellt werden.

Es wird ferner vorgeschlagen, dass der materielle Griffkörper der Griffeinheit aus zumindest zwei miteinander verbundenen Schichten aus einem Papierwerkstoff geschichtet wird. Dadurch kann insbesondere vorteilhaft ein Griffkörper hergestellt werden. Es kann insbesondere ein vorteilhaft stabiler und leicht herzustellender Griffkörper bereitgestellt werden. Bei einem Herstellungsverfahren werden insbesondere Bögen aus Papierwerkstoff, welche die Schichten ausbilden, vorgestanzt. Eine Vorstanzung erfolgt insbesondere im Bereich der Anordnung des funktionalen Elements, insbesondere der Anwendungseinheit. Es erfolgt insbesondere eine Vorstanzung der Bereiche des materiellen Griffkörpers, die nach Montage des funktionalen Elements nicht mehr gestanzt werden können. Anschließend wird insbesondere der vorgestanzte Bogen bereitgelegt und ein Faden eingelegt. Darauffolgend wird ein weiterer vorgestanzter Bogen darübergelegt und die Bögen verbunden, wie beispielsweise durch Laminieren, Schweißen und/oder Kleben. Anschließend kann das Körperpflegeprodukt, insbesondere dessen Außengeometrie, fertig gestanzt werden und überschüssiger Faden entfernt werden. Es wäre auch denkbar, dass auf einen entsprechenden Schritt verzichtet wird und anstatt des Vorstanzens direkt richtig gestanzt wird und der Faden direkt nicht über die finale Außengeometrie ragt.

Es wird weiter vorgeschlagen, dass die Anwendungseinheit in zumindest einem Verbindungsabschnitt zwischen den zumindest zwei Schichten in dem materiellen Griffkörper eingeklebt und/oder einlaminiert wird. Der Anwendungseinheit kann auf verschiedene, einem Fachmann als sinnvoll erscheinende Arten verankert werden. Eine Verankerung kann beispielsweise durch Befestigen der Anwendungseinheit mittels eines Klebstoffs erfolgen. Alternativ oder zusätzlich kann eine Verankerung durch Schweißen erfolgen. Die Anwendungseinheit kann mittels Schweißen zwischen den Schichten befestigt werden.

Das erfindungsgemäße Körperpflegeprodukt soll hierbei nicht auf die oben beschriebenen Anwendungen und Ausführungsformen beschränkt sein. Insbesondere kann das erfindungsgemäße Körperpflegeprodukt zu einer Erfüllung einer hierin beschriebenen Funktionsweise eine von einer hierin genannten Anzahl von einzelnen Elementen, Bauteilen und Einheiten abweichende Anzahl und/oder eine beliebig sinnvolle Kombination derselben aufweisen. Zudem sollen bei den in dieser Offenbarung angegebenen Wertebereichen auch innerhalb der genannten Grenzen liegende Werte als offenbart und als beliebig einsetzbar gelten.

Selbstverständlich sind die in dieser Schrift gezeigten Ausgestaltungsvarianten beispielhaft. Im Rahmen der Erfindung können die einzelnen Ausprägungen und Elemente dieser Ausgestaltungsvarianten mit anderen Ausgestaltungsvarianten kombiniert werden, ohne den Rahmen dieser Erfindung zu verlassen.

### Zeichnungen

Weitere Vorteile ergeben sich aus der folgenden Zeichnungsbeschreibung. In den Zeichnungen sind drei Ausführungsbeispiele der Erfindung sowie weitere allgemeine, nicht erfindungsgemäße Ausführungsbeispiele dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Ausführungsformen der beanspruchten Erfindung sind in den Figuren 1, 5, 7, 9, 11, 12, 14, 15 und 17 dargestellt. Die anderen Ausführungsformen fallen nicht in den Schutzbereich der Ansprüche.
- Fig. 1A: eine Anwendungseinheit eines als Interdentalbürste ausgebildeten Körperpflegeprodukts in einer schematischen Darstellung,
- Fig. 1B: das Körperpflegeprodukt mit der Anwendungseinheit und mit einer Griffeinheit bei einer Herstellung in einer schematischen Darstellung,
- Fig. 1C: das fertige Körperpflegeprodukt mit der Anwendungseinheit und mit einer Griffeinheit in einer schematischen Darstellung,
- Fig. 1D: einen Teilausschnitt einer Schicht eines materiellen Griffkörpers der Griffeinheit in einer schematischen Schnittdarstellung,
- Fig. 2A: eine erste Schicht mehrerer Griffeinheiten jeweils eines alternativen, als Flosser ausgebildeten Körperpflegeprodukts bei einer Herstellung in einer schematischen Darstellung,
- Fig. 2B: die erste Schicht der mehreren Griffeinheiten und mehrere Anwendungseinheiten mit gezeigtem Reinigungselement der alternativen Körperpflegeprodukte bei einer Herstellung in einer schematischen Darstellung,
- Fig. 2C: die Griffeinheiten und die Anwendungseinheiten der alternativen Körperpflegeprodukte bei einer Herstellung in einer schematischen Darstellung,
- Fig. 2D: das fertige alternative Körperpflegeprodukt mit der Anwendungseinheit und mit einer Griffeinheit in einer schematischen Darstellung,
- Fig. 3A: ein weiteres alternatives, als Flosser ausgebildetes Körperpflegeprodukt mit einer Anwendungseinheit und mit einer Griffeinheit in einem ungefalteten Zustand bei einer Herstellung in einer schematischen Darstellung,
- Fig. 3B: das weitere alternative Körperpflegeprodukt mit der Anwendungseinheit und mit der Griffeinheit in einem teilweise gefalteten Zustand bei einer Herstellung in einer schematischen Darstellung,
- Fig. 3C: das fertige weitere alternative Körperpflegeprodukt mit der Anwendungseinheit und mit einer Griffeinheit in einer schematischen Darstellung,
- Fig. 4A: eine erste Schicht mehrerer Griffeinheiten jeweils eines weiteren alternativen, als Flosser ausgebildeten Körperpflegeprodukts bei einer Herstellung in einer schematischen Darstellung,
- Fig. 4B: die erste Schicht der mehreren Griffeinheiten und mehrere Anwendungseinheiten mit gezeigtem Reinigungselement der weiteren alternativen Körperpflegeprodukte bei einer Herstellung in einer schematischen Darstellung,
- Fig. 4C: die Griffeinheiten und die Anwendungseinheiten der weiteren alternativen Körperpflegeprodukte bei einer Herstellung in einer schematischen Darstellung,
- Fig. 4D: das fertige weitere alternative Körperpflegeprodukt mit der Anwendungseinheit und mit einer Griffeinheit in einer schematischen Darstellung,
- Fig. 5A: eine Anwendungseinheit eines weiteren alternativen erfindungsgemäßen, als Interdentalbürste ausgebildeten Körperpflegeprodukts in einer schematischen Darstellung,
- Fig. 5B: eine erste Schicht der mehreren Griffeinheiten und mehrere der Anwendungseinheiten der weiteren alternativen erfindungsgemäßen Körperpflegeprodukte bei einer Herstellung in einer schematischen Darstellung,
- Fig. 5C: die Griffeinheiten und die Anwendungseinheiten der weiteren alternativen erfindungsgemäßen Körperpflegeprodukte bei einer Herstellung in einer schematischen Darstellung,
- Fig. 5D: das fertige weitere alternative erfindungsgemäße Körperpflegeprodukt mit der Anwendungseinheit und mit einer Griffeinheit in einer schematischen Darstellung,
- Fig. 5E: mehrere fertige weitere alternative erfindungsgemäße Körperpflegeprodukte, welche miteinander verbunden sind, mit der Anwendungseinheit und mit einer Griffeinheit in einer schematischen Darstellung,
- Fig. 6A: eine Griffeinheit eines weiteren alternativen, als Flosser ausgebildeten Körperpflegeprodukts in einer schematischen Darstellung,
- Fig. 6B: das fertige weitere alternative Körperpflegeprodukt mit der Anwendungseinheit und mit einer Griffeinheit in einer schematischen Darstellung,
- Fig. 7A: ein fertiges weiteres alternatives, als Zahnbürste ausgebildetes Körperpflegeprodukt mit einer Anwendungseinheit und mit einer gesteckten Griffeinheit in einer schematischen Darstellung,
- Fig. 7B: das weitere alternative Körperpflegeprodukt mit der Anwendungseinheit und mit der gesteckten Griffeinheit in einer schematischen Explosionsdarstellung,
- Fig. 8A: ein weiteres alternatives, als Zahnstocher ausgebildetes Körperpflegeprodukt bei einer Herstellung in einer schematischen Darstellung,
- Fig. 8B: das fertige weitere alternative Körperpflegeprodukt in einer schematischen 3D-Ansicht,
- Fig. 8C: das fertige weitere alternative Körperpflegeprodukt in einer schematischen Seitenansicht,
- Fig. 9A: eine Anwendungseinheit des weiteren alternativen Körperpflegeprodukts bei einer Herstellung in einer schematischen Darstellung,
- Fig. 9B: das fertige weitere alternative Körperpflegeprodukt mit der Anwendungseinheit und mit der Griffeinheit in einer schematischen Darstellung,
- Fig. 10A: ein weiteres alternatives, als Zahnstocher ausgebildetes Körperpflegeprodukt in einem ungefalteten Zustand in einer schematischen Darstellung,
- Fig. 10B: das weitere alternative Körperpflegeprodukt in einem gefalteten Anwendungszustand in einer schematischen Darstellung,
- Fig. 11A: eine Griffeinheit eines weiteren alternativen erfindungsgemäßen, als Zahnbürste ausgebildeten Körperpflegeprodukts bei einer Herstellung in einer schematischen Darstellung,
- Fig. 11B: das fertige weitere alternative Körperpflegeprodukt mit einer Anwendungseinheit und mit der Griffeinheit in einer schematischen Darstellung,
- Fig. 12A: eine Anwendungseinheit eines weiteren alternativen, als Zahnbürste ausgebildeten Körperpflegeprodukts bei einer Herstellung in einer schematischen Darstellung,
- Fig. 12B: das fertige weitere alternative Körperpflegeprodukt mit der Anwendungseinheit und mit einer Griffeinheit in einer schematischen Darstellung,
- Fig. 13A: ein fertiges weiteres alternatives, als Flosser ausgebildetes Körperpflegeprodukt mit einer Anwendungseinheit und mit einer Griffeinheit, welche eine Mulde aufweist, in einer schematischen Darstellung,
- Fig. 13B: eine schematische Schnittdarstellung durch die Griffeinheit des weiteren alternativen Körperpflegeprodukts entlang der Schnittlinie A-A,
- Fig. 14A: ein fertiges weiteres alternatives erfindungsgemäßes Körperpflegeprodukt mit einer Anwendungseinheit und mit einer Griffeinheit in einer schematischen Darstellung,
- Fig. 14B: das fertige weitere alternative erfindungsgemäße Körperpflegeprodukt mit der Anwendungseinheit und mit der Griffeinheit in einer schematischen Seitenansicht in einem aufgestellten Zustand,
- Fig. 15A: ein fertiges weiteres alternatives erfindungsgemäßes Körperpflegeprodukt mit einer Anwendungseinheit und mit einer Griffeinheit in einer schematischen Darstellung,
- Fig. 15B: das fertige weitere alternative erfindungsgemäße Körperpflegeprodukt mit der Anwendungseinheit und mit der Griffeinheit in einer schematischen Seitenansicht in einem aufgestellten Zustand,
- Fig. 16A: eine erste Schicht einer Griffeinheit eines weiteren alternativen, als Flosser ausgebildeten Körperpflegeprodukts bei einer Herstellung in einer schematischen Darstellung,
- Fig. 16B: eine zweite, eine Anwendungseinheit und teilweise die Griffeinheit ausbildende Schicht der weiteren alternativen Körperpflegeprodukte bei einer Herstellung in einer schematischen Darstellung,
- Fig. 16C: das fertige weitere alternative Körperpflegeprodukt mit der Anwendungseinheit und mit einer Griffeinheit in einer schematischen Darstellung,
- Fig. 17A: eine Anwendungseinheit eines weiteren alternativen, als Interdentalbürste ausgebildeten Körperpflegeprodukts bei einer Herstellung in einer schematischen Darstellung,
- Fig. 17B: eine Griffeinheit des weiteren alternativen Körperpflegeprodukts bei einer Herstellung in einer schematischen Darstellung,
- Fig. 17C: das fertige weitere alternative Körperpflegeprodukt mit einer Anwendungseinheit und mit der Griffeinheit in einer schematischen Darstellung,
- Fig. 18: ein weiteres alternatives, als Zahnstocher ausgebildetes Körperpflegeprodukt mit einer ersten möglichen Ausbildung einer Anwendungseinheit in einer schematischen Darstellung,
- Fig. 19: ein weiteres alternatives, als Zahnstocher ausgebildetes Körperpflegeprodukt mit einer zweiten möglichen Ausbildung einer Anwendungseinheit in einer schematischen Darstellung und
- Fig. 20: ein weiteres alternatives, als Zahnstocher ausgebildetes Körperpflegeprodukt mit einer dritten möglichen Ausbildung einer Anwendungseinheit in einer schematischen Darstellung,

### Beschreibung der Ausführungsbeispiele

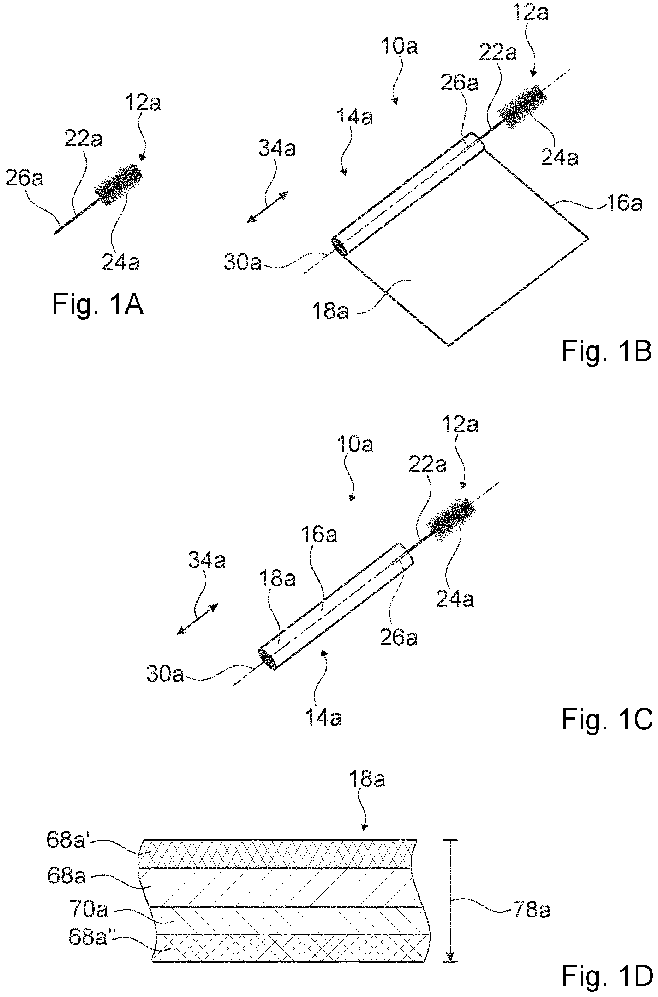

Die Figur 1A zeigt eine Anwendungseinheit 12a eines Körperpflegeprodukts 10a in einer schematischen perspektivischen Darstellung. Das Körperpflegeprodukt 10a ist von einem Mundhygienemittel gebildet. Das Körperpflegeprodukt 10a ist im vorliegenden Fall als eine Interdentalbürste ausgebildet. Das Körperpflegeprodukt 10a kann ebenso als eine Einmalzahnbürste oder auch als eine Wechselkopfzahnbürste ausgebildet sein. Zudem könnte das Körperpflegeprodukt 10a als ein Flosser, eine Single-Tuft-Bürste, ein Zwischenzahnreiniger, ein Zungenreiniger oder dergleichen ausgebildet sein. Ferner sind kombinierte Körperpflegeprodukte 10a denkbar, die zumindest zwei unterschiedliche Funktionen kombinieren, beispielsweise eine Zahnbürste mit Zungenreiniger, eine Zahnbürste mit Massageelementen, einen Interdentalreiniger mit Flosser oder dergleichen mehr.

Das Körperpflegeprodukt 10a weist die Anwendungseinheit 12a auf. Ferner weist das Körperpflegeprodukt 10a zumindest eine mit der Anwendungseinheit 12a verbundene Griffeinheit 14a auf.

Im Folgenden wird auf die Figuren 1A bis 1C Bezug genommen, welche unterschiedliche Ansichten und Fertigungsstufen des Körperpflegeprodukts 10a zeigen. Aufgrund der unterschiedlichen Ansichten sind einige Elemente nicht in sämtlichen Figuren dargestellt und entsprechend nicht in sämtlichen Figuren mit Bezugszeichen versehen.

Das Körperpflegeprodukt 10a weist eine Längsachse 30a und eine Höhenachse auf. Die Längsachse 30a ist parallel zu einer Haupterstreckungsrichtung 34a des Körperpflegeprodukts 10a angeordnet.

Die Anwendungseinheit 12a ist an der Oberseite des Körperpflegeprodukts 10a angeordnet. Die Anwendungseinheit 12a bildet einen obersten Punkt des Körperpflegeprodukts 10a.

Die Griffeinheit 14a ist zu einem Greifen durch den Bediener vorgesehen. Die Griffeinheit 14a weist eine zylindrische Grundform auf. Die Griffeinheit 14a weist ferner einen materiellen Griffkörper 16a auf. Der materielle Griffkörper 16a bildet ein materielles Volumen der Griffeinheit 14a. Der materielle Griffkörper 16a weist eine zylindrische Form auf. Der materielle Griffkörper 16a der Griffeinheit 14a besteht zumindest teilweise, insbesondere zumindest zu einem Großteil, aus einem Papierwerkstoff. Der materielle Griffkörper 16a besteht vollständig aus einem Papierwerkstoff. Der materielle Griffkörper 16a der Griffeinheit 14a weist zumindest eine Schicht 18a aus einem Papierwerkstoff auf, welche gerollt ausgebildet ist. Der materielle Griffkörper 16a weist beispielhaft genau eine Schicht 18a auf, es wäre jedoch auch ein mehrschichtiger Aufbau denkbar. Die Schicht 18a aus dem Papierwerkstoff ist um eine definierte Rollachse aufgerollt. Die Rollachse verläuft parallel, insbesondere koaxial, zu der Längsachse 30a des Körperpflegeprodukts 10a. Die Schicht 18a wird bei einer Herstellung des materiellen Griffkörpers 16a aufgerollt. Die Schicht 18a bildet in einem Endzustand, insbesondere nach einer Herstellung, eine Rolle, insbesondere eine zylindrische Rolle, aus. Die Schicht 18a des materiellen Griffkörpers 16a ist in eine zylindrische Form gerollt. Die Schicht 18a des materiellen Griffkörpers 16a ist mittels Kleben, Schweißen und/oder Laminieren in der Rolle mit sich selbst verbunden. Der Papierwerkstoff ist zu einer massiven Rolle gerollt.

Der materielle Griffkörper 16a kann insbesondere gerade oder spiralförmig gerollt sein. Der materielle Griffkörper 16a weist einen Durchmesser von 2 mm bis 10 mm, vorzugsweise 3 mm bis 7 mm, auf. Optional kann der materielle Griffkörper 16a neben dem Rohr aus Papierwerkstoff zudem mit einem Kunststoff-Kopf versehen sein, welcher beispielsweise ein Eindringen von Wasser verhindert.

Ferner weist die Anwendungseinheit 12a einen Reinigungselementträger 22a und mehrere mit dem Reinigungselementträger 22a verbundene, insbesondere eingedrehte, Reinigungselemente 24a auf. Der Reinigungselementträger 22a ist beispielhaft von einem Draht gebildet, wobei die Reinigungselemente 24a von in den Draht eingedrehten Borsten gebildet sind. Es wäre jedoch auch eine andere, einem Fachmann als sinnvoll erscheinende Ausgestaltung des Reinigungselementträgers 22a und/oder der Reinigungselemente 24a denkbar. Der Reinigungselementträger 22a ist zu einer Verbindung mit der Griffeinheit 14a mit einem den Reinigungselementen 24a abgewandten Verbindungsabschnitt 26a mit dem materiellen Griffkörper 16a verbunden. Der Verbindungsabschnitt 26a ist insbesondere von einem den Reinigungselementen 24a abgewandten Drahtende gebildet. Der Verbindungsabschnitt 26a des Reinigungselementträgers 22a der Anwendungseinheit 12a ragt, insbesondere in einem hergestellten Zustand des Körperpflegeprodukts 10a, in den materiellen Griffkörper 16a und ist von diesem umschlossen. Die Anwendungseinheit 12a ist über den Verbindungsabschnitt 26a direkt mit dem materiellen Griffkörper 16a verbunden. Der Reinigungselementträger 22a der Anwendungseinheit 12a ist an dem den Reinigungselementen 24a abgewandten Verbindungsabschnitt 26a in die Schicht 18a des materiellen Griffkörpers 16a eingerollt ausgebildet. Der Verbindungsabschnitt 26a der Anwendungseinheit 12a wird dabei bei einer Herstellung des materiellen Griffkörpers 16a in den materiellen Griffkörper 16a eingerollt. Der Verbindungsabschnitt 26a der Anwendungseinheit 12a wird dabei direkt bei einem Rollen der Schicht 18a mit dem materiellen Griffkörper 16a verbunden, wie insbesondere verklebt. Der Reinigungselementträger 22a der Anwendungseinheit 12a ist stoffschlüssig, insbesondere mittels Klebung und/oder Verschweißung, mit dem materiellen Griffkörper 16a verbunden.

Es wird insbesondere ein Verfahren zur Herstellung des Körperpflegeprodukts 10a offenbart. Bei dem Verfahren wird der materielle Griffkörper 16a der Griffeinheit 14a aus der Schicht 18a aus einem Papierwerkstoff gerollt. Ferner wird die Anwendungseinheit 12a in dem zumindest einen Verbindungsabschnitt 26a in den materiellen Griffkörper 16a eingerollt. Ein entsprechender Verfahrensablauf zur Herstellung könnte dabei in einer Variante derart erfolgen, dass in einem ersten Schritt die Anwendungseinheit 12a in Form einer Interdentalbürste durch Eindrehen hergestellt wird (siehe Fig. 1A). Anschließend wird in einem zweiten Verfahrensschritt die zumindest eine Schicht 18a des materiellen Griffkörpers 16a gerollt und gleichzeitig der Verbindungsabschnitt 26a der Anwendungseinheit 12a in den materiellen Griffkörper 16a eingerollt (siehe Fig. 1B). Eine Verankerung der Anwendungseinheit 12a in dem materiellen Griffkörper 16a kann insbesondere durch Klebstoff, Schweißen oder Verpressen erfolgen. Hierfür kann allenfalls ein weiterer Verfahrensschritt nötig sein. Ein fertiges Körperpflegeprodukt 10a ist in der Figur 1C gezeigt.

Ferner ist vorgesehen, dass der Papierwerkstoff des materiellen Griffkörpers 16a eine wasserabweisende Beschichtung und/oder Imprägnierung aufweist. Durch die entsprechende Beschichtung und/oder Imprägnierung kann auch eine Verminderung der Wasseraufnahme des Papierwerkstoffs erreicht werden. Damit kann sich der Papierwerkstoff auch für einen Mehrfacheinsatz eignen. Eine Imprägnierung kann dabei beispielsweise mittels eines Kunststoffs und/oder eines Wachses oder dergleichen erfolgen. Des Weiteren ist vorgesehen, dass der Papierwerkstoff des materiellen Griffkörpers 16a lackiert und/oder bedruckt ausgebildet ist. Es kann insbesondere eine Veredelung erreicht werden. Eine Veredelung kann beispielsweise eine Lackierung sein, dabei kann beispielsweise UV-Hochglanzlack, Strukturlack, Softtouch-Lack oder Glitterlack verwendet werden. Alternativ oder zusätzlich kann der Papierwerkstoff eine Bedruckung aufweisen. Der Papierwerkstoff kann beispielsweise eine Bedruckung auf wasserbasierten Lacken aufweisen, welche gegenüber konventionellen Lacken ökologisch sind. Alternativ oder zusätzlich wäre auch denkbar, dass die Enden des materiellen Griffkörpers 16a verpresst sind oder beispielsweise mit einem Wachs versiegelt sind. Bei einer Versiegelung wäre beispielsweise denkbar, dass bereits ein Füllstoff, wie beispielsweise Wachs, auf dem Papierwerkstoff vorgesehen ist und zu einer Versiegelung lediglich die Enden des materiellen Griffkörpers 16a erhitzt werden müssen.

Figur 1D zeigt einen beispielhaften Aufbau der Schicht 18a des materiellen Griffkörpers 16a. Die Schicht 18a weist mehrere Teilschichten 68a, 68a', 68a" auf, welche die Schicht 18a ausbilden. Die Schicht 18a weist einen erste Teilschicht 68a auf, welche eine Grundschicht, insbesondere eine Trägerschicht, ausbildet. Die erste Teilschicht 68a besteht aus einem Papierwerkstoff, insbesondere aus einem Papier. Die erste Teilschicht 68a weist insbesondere eine Bedruckung 70a auf einer Außenseite auf. Die Bedruckung 70a ist insbesondere zu einer Gestaltung des materiellen Griffkörpers 16a vorgesehen. Die Bedruckung 70a ist insbesondere in einer Vorbehandlung auf die erste Teilschicht 68a aufgebracht. Ferner weist die Schicht 18a eine zweite Teilschicht 68a' auf. Die zweite Teilschicht 68a' ist auf einer Innenseite der ersten Teilschicht 68a angeordnet. Die zweite Teilschicht 68a' ist von einem Siegellack gebildet. Der zweite Teilschicht 68a' ist in einer Vorbehandlung auf die erste Teilschicht 68a aufgebracht. Der Siegellack dient zu einer Verbindung der Schicht 18a bei einem Rollen. Des Weiteren weist die Schicht 18a eine dritte Teilschicht 68a" auf. Die dritte Teilschicht 68a" ist auf einer Außenseite der ersten Teilschicht 68a angeordnet. Die dritte Teilschicht 68a" ist von einem Schutzlack gebildet. Der dritte Teilschicht 68a" ist in einer Vorbehandlung auf die erste Teilschicht 68a aufgebracht. Der Schutzlack dient zu einem Schutz der Schicht 18a vor einem Eindringen von Feuchtigkeit.

Ein Pfeil 78a in der Figur 1D definiert beispielhaft eine Richtung von einer Innenseite der Schicht 18a hin zu einer Außenseite der Schicht 18a.

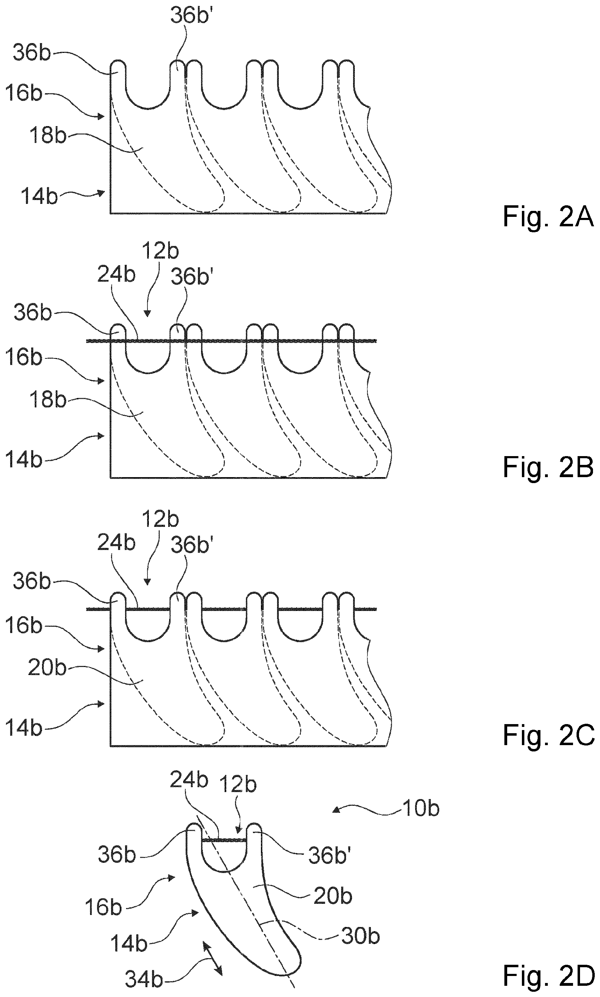

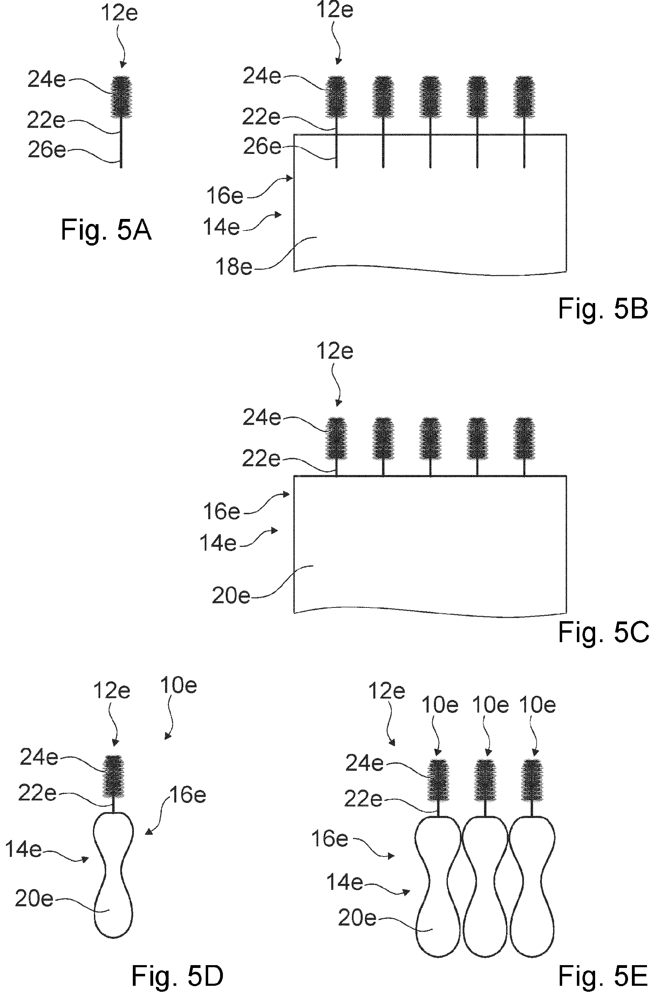

In den Figuren 5, 7, 9, 11, 12, 14, 15 und 17 sind weitere Ausführungsbeispiele der Erfindung gezeigt. Die nachfolgenden Beschreibungen beschränken sich im Wesentlichen auf die Unterschiede zwischen den Ausführungsbeispielen, wobei bezüglich gleichbleibender Bauteile, Merkmale und Funktionen auf die Beschreibung der anderen Ausführungsbeispiele, insbesondere der Figuren 1A bis 1C, verwiesen werden kann. Zur Unterscheidung der Ausführungsbeispiele ist der Buchstabe a in den Bezugszeichen des Erfindungsgemäß Ausführungsbeispiels der Figuren 1A bis 1C durch die Buchstaben b bis t in der Bezugszeichen der Ausführungsbeispiele der Figuren 2A bis 20 ersetzt. Bezüglich gleich bezeichneter Bauteile, insbesondere in Bezug auf Bauteile mit gleichen Bezugszeichen, kann grundsätzlich auch auf die Zeichnungen und/oder die Beschreibung der anderen Ausführungsbeispiele, insbesondere der Figuren 1A bis 1C, verwiesen werden.

Die Figur 2A zeigt eine erste Schicht 18b mehrerer Griffeinheiten 14b jeweils eines als Flosser ausgebildeten Körperpflegeprodukts 10b bei einer Herstellung. Diese Ausführungsform ist nicht Teil der beanspruchten Erfindung. In den Figuren 2A bis 2D sind insbesondere teilweise mehrere identische Körperpflegeprodukte 10b dargestellt, welche gleichzeitig hergestellt werden. Im Folgenden wird jedoch im Wesentlichen lediglich auf eines der Körperpflegeprodukte 10b eingegangen, wobei eine Beschreibung grundsätzlich auch auf die weiteren Körperpflegeprodukte 10b anwendbar ist.

Das Körperpflegeprodukt 10b weist eine Anwendungseinheit 12b auf. Ferner weist das Körperpflegeprodukt 10b zumindest eine mit der Anwendungseinheit 12b verbundene Griffeinheit 14b auf.

Die Anwendungseinheit 12b ist an der Oberseite des Körperpflegeprodukts 10b angeordnet. Die Anwendungseinheit 12b bildet einen obersten Punkt des Körperpflegeprodukts 10b. Die Anwendungseinheit 12b weist ein Reinigungselement 24b auf, welches von einer Zahnseide gebildet ist. Die Anwendungseinheit 12b besteht insbesondere aus dem Reinigungselement 24b.

Die Griffeinheit 14b ist zu einem Greifen durch den Bediener vorgesehen. Die Griffeinheit 14b weist ferner einen materiellen Griffkörper 16b auf. Der materielle Griffkörper 16b bildet ein materielles Volumen der Griffeinheit 14b. Der materielle Griffkörper 16b weist eine gebogene, flache Grundform auf, welche sich zu der Anwendungseinheit 12b hin in zwei Arme 36b, 36b' aufteilt. Die Arme 36b, 36b' sind dazu vorgesehen, die Zahnseide beidseitig zu halten. Der materielle Griffkörper 16b der Griffeinheit 14b besteht zumindest teilweise, insbesondere zumindest zu einem Großteil, aus einem Papierwerkstoff. Der materielle Griffkörper 16b besteht vollständig aus einem Papierwerkstoff. Der materielle Griffkörper 16b der Griffeinheit 14b weist zumindest zwei Schichten 18b, 20b aus einem Papierwerkstoff auf, die geschichtet miteinander verbunden sind. Der materielle Griffkörper 16b der Griffeinheit 14b weist beispielhaft genau zwei Schichten 18b, 20b aus einem Papierwerkstoff auf. Vorzugsweise sind von 2 bis 6, vorzugsweise von 2 bis 4, Schichten 18b, 20b vorgesehen. Bevorzugt sind die zumindest zwei Schichten 18b, 20b senkrecht zu einer Haupterstreckungsebene der Schichten 18b, 20b übereinander angeordnet und miteinander verbunden. Die Schichten 18b, 20b sind im Wesentlichen identisch ausgebildet und bilden insbesondere gegenüberliegende Außenseiten des Körperpflegeprodukts 10b. Die Schichten 18b, 20b sind beispielsweise mittels Kleben und/oder Pressen und/oder Schweißen und/oder Laminieren miteinander verbunden. Die Schichten 18b, 20b werden insbesondere bei einer Herstellung des Körperpflegeprodukts 10b miteinander verbunden.

Die Anwendungseinheit 12b ist zwischen den zumindest zwei Schichten 18b, 20b des materiellen Griffkörpers 16b an der Griffeinheit 14b angeordnet. Die Anwendungseinheit 12b ist im Bereich der beiden Arme 36b, 36b' zwischen den zwei Schichten 18b, 20b des materiellen Griffkörpers 16b angeordnet und fixiert. Das Reinigungselement 24b der Anwendungseinheit 12b spannt sich zwischen den Armen 36b, 36b'.

Es wird insbesondere ein Verfahren zur Herstellung des Körperpflegeprodukts 10b offenbart. Bei dem Verfahren wird der materielle Griffkörper 16b der Griffeinheit 14b aus den zumindest zwei miteinander verbundenen Schichten 18b, 20b aus einem Papierwerkstoff geschichtet. Bei einem Herstellungsverfahren werden insbesondere Bögen aus Papierwerkstoff, welche die Schichten 18b, 20b ausbilden, vorgestanzt. Eine Vorstanzung erfolgt mindestens im Bereich der Anordnung der Anwendungseinheit 12b. Es erfolgt insbesondere eine Vorstanzung der Bereiche des materiellen Griffkörpers 16b, die nach Montage der Anwendungseinheit 12b nicht mehr gestanzt werden können. Anschließend wird insbesondere der vorgestanzte Bogen, welcher die erste Schicht 18b bildet, bereitgelegt und das Reinigungselement 24b eingelegt (siehe Figuren 2A und 2B). Der Bogen ist dabei beispielhaft für die Herstellung mehrerer Körperpflegeprodukte 10b gleichzeitig vorgesehen. Darauffolgend wird ein weiterer vorgestanzter Bogen, welcher die zweite Schicht 20b ausbildet, darübergelegt und die Schichten 18b, 20b verbunden, wie beispielsweise durch Laminieren, Schweißen und/oder Kleben (siehe Figur 2C). Anschließend kann das Körperpflegeprodukt 10b, insbesondere dessen Außengeometrie, fertig gestanzt werden und überschüssiger Zahnseidefaden der Anwendungseinheit 12b entfernt werden (siehe Figur 2D). Es wäre auch denkbar, dass auf einen entsprechenden Schritt verzichtet wird und direkt anstatt des Vorstanzens direkt richtig gestanzt wird und der Zahnseidefaden der Anwendungseinheit 12b direkt nicht über die finale Außengeometrie ragt. Die Anwendungseinheit 12b wird in zumindest einem Verbindungsabschnitt 26b zwischen den zumindest zwei Schichten 18b, 20b in dem materiellen Griffkörper 16b eingeklebt und/oder einlaminiert. Das Reinigungselement 24b der Anwendungseinheit 12b kann auf verschiedene, einem Fachmann als sinnvoll erscheinende Arten verankert werden. Eine Verankerung kann beispielsweise durch Befestigen des Reinigungselements 24b der Anwendungseinheit 12b mittels Klebstoffs erfolgen. Alternativ oder zusätzlich kann eine Verankerung durch Schweißen erfolgen. Das Reinigungselement 24b der Anwendungseinheit 12b kann mittels Schweißen zwischen den Schichten 18b, 20b befestigt werden. Ein einzelnes fertiges Körperpflegeprodukt 10b ist in der Figur 2D gezeigt.

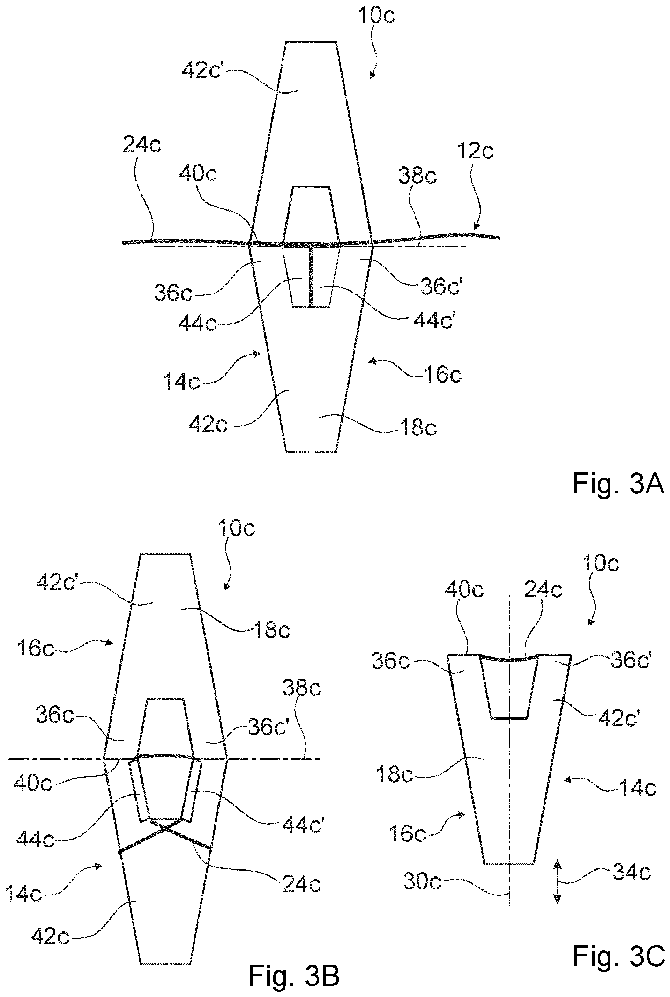

Die Figur 3A zeigt ein als Flosser ausgebildetes Körperpflegeprodukt 10c in einem ungefalteten Zustand bei einer Herstellung. Diese Ausführungsform ist nicht Teil der beanspruchten Erfindung. Das Körperpflegeprodukt 10c weist die Anwendungseinheit 12c auf. Ferner weist das Körperpflegeprodukt 10c zumindest eine mit der Anwendungseinheit 12c verbundene Griffeinheit 14c auf. Die Anwendungseinheit 12c ist an der Oberseite des Körperpflegeprodukts 10c angeordnet. Die Anwendungseinheit 12c bildet einen obersten Punkt des Körperpflegeprodukts 10c. Die Anwendungseinheit 12c weist ein Reinigungselement 24c auf, welches von einer Zahnseide gebildet ist. Die Anwendungseinheit 12c besteht insbesondere aus dem Reinigungselement 24c.

Die Griffeinheit 14c ist zu einem Greifen durch den Bediener vorgesehen. Die Griffeinheit 14c weist ferner einen materiellen Griffkörper 16c auf. Der materielle Griffkörper 16c bildet ein materielles Volumen der Griffeinheit 14c. Der materielle Griffkörper 16c weist in einem Endzustand eine flache Grundform auf, welche sich zu der Anwendungseinheit 12c hin in zwei Arme 36c, 36c' aufteilt. Die Arme 36c, 36c' sind dazu vorgesehen, die Zahnseide beidseitig zu halten. Der materielle Griffkörper 16c der Griffeinheit 14c besteht zumindest teilweise, insbesondere zumindest zu einem Großteil, aus einem Papierwerkstoff. Der materielle Griffkörper 16c besteht vollständig aus einem Papierwerkstoff. Der materielle Griffkörper 16c der Griffeinheit 14c weist zumindest eine Schicht 18c aus einem Papierwerkstoff auf, die zumindest teilweise gefaltet ausgebildet ist. Der materielle Griffkörper 16c der Griffeinheit 14c weist beispielhaft genau eine Schicht 18c aus einem Papierwerkstoff auf. Die Schicht 18c umfasst eine Faltachse 38c. In der Faltachse 38c weist die Schicht 18c eine Faltkante 40c auf. Die Schicht 18c ist in einem Gebrauchszustand an der Faltkante 40c um die Faltachse 38c gefaltet. Die Schicht 18c ist in der Faltkante 40c vorzugsweise perforiert und/oder geprägt ausgebildet, um ein definiertes Falten zu ermöglichen. Die Schicht 18c ist durch die Faltkante 40c in zwei Seiten, insbesondere Teilschichten 42c, 42c', unterteilt. Eine zweite Teilschicht 42c' der Teilschichten 42c, 42c' ist auf eine erste Teilschicht 42c der Teilschichten 42c, 42c' gefaltet, um mehrere Lagen zu schaffen. Dazu weist der materielle Griffkörper 16c oben auf den Armen 36c, 36c' die als Scharnier ausgebildete Faltkante 40c auf. In der Faltkante 40c ist die Schicht 18c um 180° faltbar. Die Faltachse 38c der Schicht 18c verläuft senkrecht zur Haupterstreckungsrichtung 34c des Körperpflegeprodukts 10c. Der materielle Griffkörper 16c kann dabei insbesondere aus Papierbögen oder ab Rolle gestanzt werden. Ferner sind die Teilschichten 42c, 42c' des materiellen Griffkörpers 16c mittels Siegelung oder Klebung verbunden. Die Schicht 18c des materiellen Griffkörpers 16c wird geschnitten oder gestanzt.

Die Anwendungseinheit 12c ist an Spannungslaschen 44c, 44c' des materiellen Griffkörpers 16c verankert. Die Spannungslaschen 44c, 44c' sind einstückig mit der ersten Teilschicht 42c der Schicht 18c verbunden. Die Spannungslaschen 44c, 44c' ragen von den Armen 36c, 36c' nach innen und sind dazu vorgesehen, umgebogen zu werden. Das Reinigungselement 24c der Anwendungseinheit 12c ist um die Spannungslaschen 44c, 44c' herumgeführt. Hierdurch kann ein Ausreißen verhindert werden. Ferner kann das Reinigungselement 24c der Anwendungseinheit 12c zudem an anderer Stelle fixiert werden, wie beispielsweise mittels eines Punkts aus Klebstoff. Alternativ kann das Reinigungselement 24c der Anwendungseinheit 12c auch als Ring oder Schlinge geformt sein, also insbesondere als geschlossener Ring ohne Ende, sodass auf eine weitere Fixierung zu den Spannungslaschen 44c, 44c' verzichtet werden kann. Der Ring aus dem Faden kann dabei direkt als Ring hergestellt sein oder durch Knüpfen hergestellt sein. Der Papierwerkstoff des materiellen Griffkörpers 16c wird bei einer Herstellung insbesondere gesiegelt, wobei die Spannungslaschen 44c, 44c' mit eingesiegelt werden, womit das Reinigungselement 24c fixiert wird.

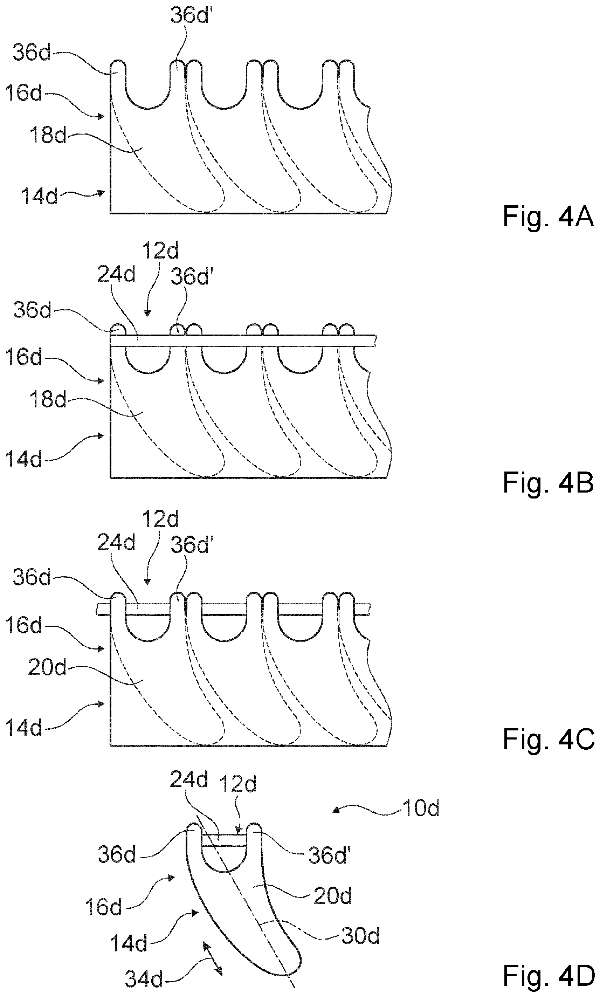

Es wird insbesondere ein Verfahren zur Herstellung des Körperpflegeprodukts 10c offenbart. Bei dem Verfahren wird der materielle Griffkörper 16c der Griffeinheit 14c aus den zumindest zwei miteinander verbundenen Schichten 18c, 20c aus einem Papierwerkstoff geschichtet. Bei einem Herstellungsverfahren wird insbesondere die Schicht 18c gestanzt und die Anwendungseinheit 12c bereitgestellt (siehe Figur 3A). Anschließend werden die Spannungslaschen 44c, 44c' umgebogen und das Reinigungselement 24c der Anwendungseinheit 12c um die Spannungslaschen 44c, 44c' herumgeführt (siehe Figur 3B). Ferner kann das Reinigungselement 24c der Anwendungseinheit 12c anschließend zudem an anderer Stelle fixiert werden, wie beispielsweise mittels eines Punkts aus Klebstoff. Anschließend kann überschüssiger Zahnseidefaden der Anwendungseinheit 12c entfernt werden. Alternativ kann das Reinigungselement 24c der Anwendungseinheit 12c auch als Ring oder Schlinge geformt sein, also insbesondere als geschlossener Ring ohne Ende, sodass auf eine weitere Fixierung zu den Spannungslaschen 44c, 44c' verzichtet werden kann. Darauffolgend wird die zweite Teilschicht 42c' der Teilschichten 42c, 42c' auf die erste Teilschicht 42c der Teilschichten 42c, 42c' gefaltet und gesiegelt, wobei die Spannungslaschen 44c, 44c' mit eingesiegelt werden. Ein fertiges Körperpflegeprodukt 10c ist in der Figur 3C gezeigt. Die Figur 4A zeigt eine erste Schicht 18d mehrerer Griffeinheiten 14d jeweils eines als

Flosser ausgebildeten Körperpflegeprodukts 10d bei einer Herstellung. Diese Ausführungsform ist nicht Teil der beanspruchten Erfindung. In den Figuren 4A bis 4D sind insbesondere teilweise mehrere identische Körperpflegeprodukte 10d dargestellt, welche gleichzeitig hergestellt werden. Im Folgenden wird jedoch im Wesentlichen lediglich auf eines der Körperpflegeprodukte 10d eingegangen, wobei eine Beschreibung grundsätzlich auch auf die weiteren Körperpflegeprodukte 10d anwendbar ist.

Das Körperpflegeprodukt 10d weist eine Anwendungseinheit 12d auf. Ferner weist das Körperpflegeprodukt 10d zumindest eine mit der Anwendungseinheit 12d verbundene Griffeinheit 14d auf.

Die Anwendungseinheit 12d ist an der Oberseite des Körperpflegeprodukts 10d angeordnet. Die Anwendungseinheit 12d bildet einen obersten Punkt des Körperpflegeprodukts 10d. Die Anwendungseinheit 12d besteht zumindest teilweise, insbesondere zumindest zu einem Großteil, aus einem Papierwerkstoff. Die Anwendungseinheit 12d weist ein einen Papierwerkstoff umfassendes Reinigungselement 24d auf. Das Reinigungselement 24d ist von einem Zahnzwischenraumreinigungspapier gebildet. Die Anwendungseinheit 12d besteht aus dem Reinigungselement 24d.

Die Griffeinheit 14d ist zu einem Greifen durch den Bediener vorgesehen. Die Griffeinheit 14d weist ferner einen materiellen Griffkörper 16d auf. Der materielle Griffkörper 16d bildet ein materielles Volumen der Griffeinheit 14d. Der materielle Griffkörper 16d weist eine gebogene, flache Grundform auf, welche sich zu der Anwendungseinheit 12d hin in zwei Arme 36d, 36d' aufteilt. Die Arme 36d, 36d' sind dazu vorgesehen, das Reinigungselement 24d beidseitig zu halten. Der materielle Griffkörper 16d der Griffeinheit 14d besteht zumindest teilweise, insbesondere zumindest zu einem Großteil, aus einem Papierwerkstoff. Der materielle Griffkörper 16d besteht vollständig aus einem Papierwerkstoff. Der materielle Griffkörper 16d der Griffeinheit 14d weist zumindest zwei Schichten 18d, 20d aus einem Papierwerkstoff auf, die geschichtet miteinander verbunden sind. Der materielle Griffkörper 16d der Griffeinheit 14d weist beispielhaft genau zwei Schichten 18d, 20d aus einem Papierwerkstoff auf. Vorzugsweise sind von 2 bis 6, vorzugsweise von 2 bis 4, Schichten 18d, 20d vorgesehen. Bevorzugt sind die zumindest zwei Schichten 18d, 20d senkrecht zu einer Haupterstreckungsebene der Schichten 18d, 20d übereinander angeordnet und miteinander verbunden. Die Schichten 18d, 20d sind im Wesentlichen identisch ausgebildet und bilden insbesondere gegenüberliegende Außenseiten des Körperpflegeprodukts 10d. Die Schichten 18d, 20d sind beispielsweise mittels Kleben und/oder Pressen und/oder Schweißen und/oder Laminieren miteinander verbunden. Die Schichten 18d, 20d werden insbesondere bei einer Herstellung des Körperpflegeprodukts 10d miteinander verbunden.

Die Anwendungseinheit 12d ist zwischen den zumindest zwei Schichten 18d, 20d des materiellen Griffkörpers 16d an der Griffeinheit 14d angeordnet ist. Die Anwendungseinheit 12d ist im Bereich der beiden Arme 36d, 36d' zwischen den zwei Schichten 18d, 20d des materiellen Griffkörpers 16d angeordnet und fixiert. Die Anwendungseinheit 12d spannt sich zwischen den Armen 36d, 36d'.

Es wird insbesondere ein Verfahren zur Herstellung des Körperpflegeprodukts 10d offenbart. Bei dem Verfahren wird der materielle Griffkörper 16d der Griffeinheit 14d aus den zumindest zwei miteinander verbundenen Schichten 18d, 20d aus einem Papierwerkstoff geschichtet. Bei einem Herstellungsverfahren werden insbesondere Bögen aus Papierwerkstoff, welche die Schichten 18d, 20d ausbilden, vorgestanzt. Eine Vorstanzung erfolgt mindestens im Bereich der Anordnung der Anwendungseinheit 12d. Es erfolgt insbesondere eine Vorstanzung der Bereiche des materiellen Griffkörpers 16d, die nach Montage der Anwendungseinheit 12d nicht mehr gestanzt werden können. Anschließend wird insbesondere der vorgestanzte Bogen, welcher die erste Schicht 18d bildet, bereitgelegt und das Reinigungselement 24d eingelegt (siehe Figuren 4A und 4B). Der Bogen ist dabei beispielhaft für die Herstellung mehrerer Körperpflegeprodukte 10d gleichzeitig vorgesehen. Darauffolgend wird ein weiterer vorgestanzter Bogen, welcher die zweite Schicht 20d ausbildet, darübergelegt und die Schichten 18d, 20d verbunden, wie beispielsweise durch Laminieren, Schweißen und/oder Kleben (siehe Figur 4C). Anschließend kann das Körperpflegeprodukt 10d, insbesondere dessen Außengeometrie, fertig gestanzt werden und überschüssiges Reinigungselement 24d der Anwendungseinheit 12d entfernt werden (siehe Figur 4D). Es wäre auch denkbar, dass auf einen entsprechenden Schritt verzichtet wird und anstatt des Vorstanzens direkt richtig gestanzt wird und das Reinigungselement 24d der Anwendungseinheit 12d direkt nicht über die finale Außengeometrie ragt. Die Anwendungseinheit 12d wird in zumindest einem Verbindungsabschnitt 26d zwischen den zumindest zwei Schichten 18d, 20d in dem materiellen Griffkörper 16d eingeklebt und/oder einlaminiert. Das Reinigungselement 24d der Anwendungseinheit 12d kann auf verschiedene, einem Fachmann als sinnvoll erscheinende Arten verankert werden. Eine Verankerung kann beispielsweise durch Befestigen des Reinigungselements 24d der Anwendungseinheit 12d mittels Klebstoffs erfolgen. Alternativ oder zusätzlich kann eine Verankerung durch Schweißen erfolgen. Der Zahnseidefaden der Anwendungseinheit 12d kann mittels Schweißen zwischen den Schichten 18d, 20d befestigt werden. Ein einzelnes fertiges Körperpflegeprodukt 10d ist in der Figur 4D gezeigt.

Die Figur 5A zeigt eine Anwendungseinheit 12e eines Körperpflegeprodukts 10e in einer schematischen Darstellung. Das Körperpflegeprodukt 10e ist im vorliegenden Fall als eine Interdentalbürste ausgebildet. In den Figuren 5A bis 5C und 5E sind insbesondere teilweise mehrere identische Körperpflegeprodukte 10e dargestellt, welche gleichzeitig hergestellt werden. Im Folgenden wird jedoch im Wesentlichen lediglich auf eines der Körperpflegeprodukte 10e eingegangen, wobei eine Beschreibung grundsätzlich auch auf die weiteren Körperpflegeprodukte 10e anwendbar ist.

Das Körperpflegeprodukt 10e weist die Anwendungseinheit 12e auf. Ferner weist das Körperpflegeprodukt 10e zumindest eine mit der Anwendungseinheit 12e verbundene Griffeinheit 14e auf.

Die Anwendungseinheit 12e ist an der Oberseite des Körperpflegeprodukts 10e angeordnet. Die Anwendungseinheit 12e bildet einen obersten Punkt des Körperpflegeprodukts 10e.

Die Griffeinheit 14e ist zu einem Greifen durch den Bediener vorgesehen. Die Griffeinheit 14e weist ferner einen materiellen Griffkörper 16e auf. Der materielle Griffkörper 16e bildet ein materielles Volumen der Griffeinheit 14e. Der materielle Griffkörper 16e weist eine flache, längliche Grundform auf. Der materielle Griffkörper 16e der Griffeinheit 14e besteht zumindest teilweise, insbesondere zumindest zu einem Großteil, aus einem Papierwerkstoff. Der materielle Griffkörper 16e besteht vollständig aus einem Papierwerkstoff. Der materielle Griffkörper 16e der Griffeinheit 14e weist zumindest zwei Schichten 18e, 20e aus einem Papierwerkstoff auf, die geschichtet miteinander verbunden sind. Der materielle Griffkörper 16e der Griffeinheit 14e weist beispielhaft genau zwei Schichten 18e, 20e aus einem Papierwerkstoff auf. Vorzugsweise sind von 2 bis 6, vorzugsweise von 2 bis 4, Schichten 18e, 20e vorgesehen. Bevorzugt sind die zumindest zwei Schichten 18e, 20e senkrecht zu einer Haupterstreckungsebene der Schichten 18e, 20e übereinander angeordnet und miteinander verbunden. Die Schichten 18e, 20e sind im Wesentlichen identisch ausgebildet und bilden insbesondere gegenüberliegenden Außenseiten des Körperpflegeprodukts 10e. Die Schichten 18e, 20e sind beispielsweise mittels Kleben und/oder Pressen und/oder Schweißen und/oder Laminieren miteinander verbunden. Die Schichten 18e, 20e werden insbesondere bei einer Herstellung des Körperpflegeprodukts 10e miteinander verbunden.

Ferner weist die Anwendungseinheit 12e einen Reinigungselementträger 22e und mehrere mit dem Reinigungselementträger 22e verbundene, insbesondere eingedrehte, Reinigungselemente 24e auf. Der Reinigungselementträger 22e ist beispielhaft von einem Draht gebildet, wobei die Reinigungselemente 24 von in den Draht eingedrehten Borsten gebildet sind.

Der Reinigungselementträger 22e ist zu einer Verbindung mit der Griffeinheit 14e mit einem den Reinigungselementen 24e abgewandten Verbindungsabschnitt 26e mit dem materiellen Griffkörper 16e verbunden. Der Verbindungsabschnitt 26e ist insbesondere von einem den Reinigungselementen 24 abgewandten Drahtende gebildet. Der Verbindungsabschnitt 26e des Reinigungselementträgers 22e der Anwendungseinheit 12e ragt, insbesondere in einem hergestellten Zustand des Körperpflegeprodukts 10e, in den materiellen Griffkörper 16e und ist von diesem umschlossen. Die Anwendungseinheit 12e ist über den Verbindungsabschnitt 26e direkt mit dem materiellen Griffkörper 16e verbunden. Der Verbindungsabschnitt 26e ist zwischen den zumindest zwei Schichten 18e, 20e des materiellen Griffkörpers 16e an der Griffeinheit 14e angeordnet. Ferner ist der Reinigungselementträger 22e der Anwendungseinheit 12e zu einer teilweise formschlüssigen Verbindung mit der Griffeinheit 14e in dem Verbindungsabschnitt 26e nicht weiter sichtbar gebogen ausgebildet.

Es wird insbesondere ein Verfahren zur Herstellung des Körperpflegeprodukts 10e offenbart. Bei dem Verfahren wird der materielle Griffkörper 16e der Griffeinheit 14e aus den zumindest zwei miteinander verbundenen Schichten 18e, 20e aus einem Papierwerkstoff geschichtet. Ein entsprechender Verfahrensablauf zur Herstellung könnte dabei in einer Variante derart erfolgen, dass in einem ersten Schritt die Anwendungseinheit 12e in Form einer Interdentalbürste durch Eindrehen hergestellt wird (siehe Fig. 5A). Anschließend wird insbesondere ein Bogen, welcher die erste Schicht 18e bildet, bereitgelegt und die Anwendungseinheit 12e mit dem Verbindungsabschnitt 26e eingelegt (siehe Figur 5B). Der Bogen ist dabei beispielhaft für die Herstellung mehrerer Körperpflegeprodukte 10e gleichzeitig vorgesehen. Darauffolgend wird ein weiterer Bogen, welcher die zweite Schicht 20e ausbildet, darübergelegt und die Schichten 18e, 20e verbunden, wie beispielsweise durch Laminieren, Schweißen und/oder Kleben (siehe Figur 5C). Anschließend kann das Körperpflegeprodukt 10e, insbesondere dessen Außengeometrie, gestanzt werden (siehe Figur 5D). Ein einzelnes fertiges Körperpflegeprodukt 10e ist in der Figur 5D gezeigt.

Optional kann bei einer Herstellung der Körperpflegeprodukte 10e eine Gruppierung der Körperpflegeprodukte 10e erfolgen, die bei einer Verwendung getrennt werden. Die Körperpflegeprodukte 10e können über den Papierwerkstoff teilweise verbunden sein, wobei insbesondere eine nicht weiter sichtbare Perforation existiert, an welcher die Körperpflegeprodukte 10e getrennt werden können. Hierdurch kann insbesondere eine einfachere Verarbeitung erreicht werden, wobei eine Herstellung in Gruppen erfolgt und erst bei Gebrauch eine Separierung erfolgt (siehe Figur 5e).

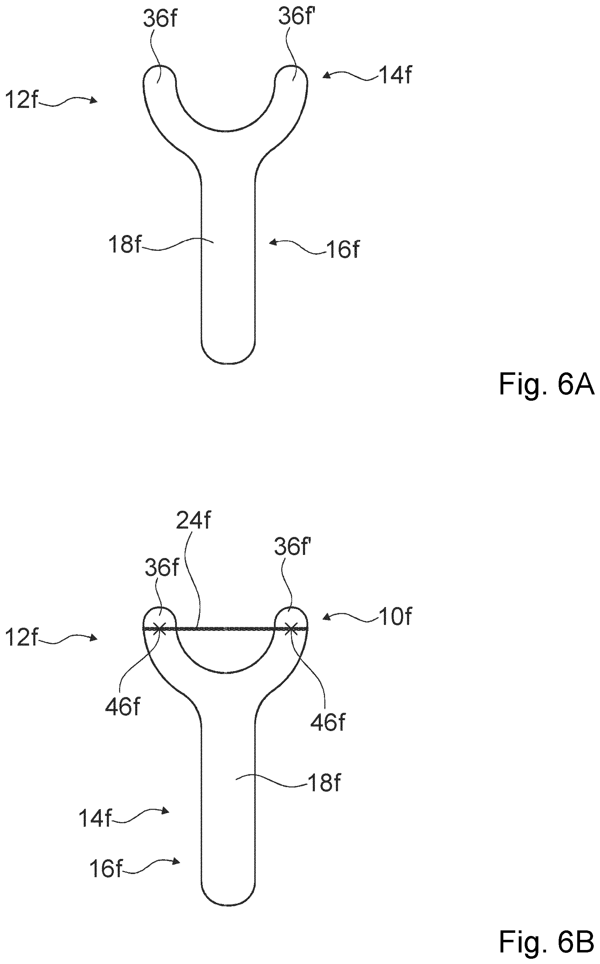

Die Figur 6A zeigt eine Schicht 18f einer Griffeinheit 14f eines als Flosser ausgebildeten Körperpflegeprodukts 10f bei einer Herstellung. Diese Ausführungsform ist nicht Teil der beanspruchten Erfindung. Das Körperpflegeprodukt 10f weist eine Anwendungseinheit 12f auf. Ferner weist das Körperpflegeprodukt 10f zumindest eine mit der Anwendungseinheit 12f verbundene Griffeinheit 14f auf. Die Anwendungseinheit 12f ist an der Oberseite des Körperpflegeprodukts 10f angeordnet. Die Anwendungseinheit 12f bildet einen obersten Punkt des Körperpflegeprodukts 10f. Die Anwendungseinheit 12f weist ein Reinigungselement 24f auf, welches von einer Zahnseide gebildet ist. Die Anwendungseinheit 12f besteht insbesondere aus dem Reinigungselement 24f.

Die Griffeinheit 14f ist zu einem Greifen durch den Bediener vorgesehen. Die Griffeinheit 14f weist ferner einen materiellen Griffkörper 16f auf. Der materielle Griffkörper 16f bildet ein materielles Volumen der Griffeinheit 14f. Der materielle Griffkörper 16f weist in einem Endzustand eine flache Grundform auf, welche sich zu der Anwendungseinheit 12f hin in zwei Arme 36f, 36f' aufteilt. Die Arme 36f, 36f' sind dazu vorgesehen, das Reinigungselement 24f beidseitig zu halten. Der materielle Griffkörper 16f der Griffeinheit 14f besteht zumindest teilweise, insbesondere zumindest zu einem Großteil, aus einem Papierwerkstoff. Der materielle Griffkörper 16f besteht vollständig aus einem Papierwerkstoff. Der materielle Griffkörper 16f der Griffeinheit 14f weist zumindest eine Schicht 18f aus einem Papierwerkstoff auf. Die Schicht 18f des materiellen Griffkörpers 16f wird geschnitten oder gestanzt.

Die Anwendungseinheit 12f ist mit dem materiellen Griffkörper 16f vernäht. Das Reinigungselement 24f ist an beiden Enden an den Enden der Arme 36f, 36f' mit dem materiellen Griffkörper 16f vernäht. Das Körperpflegeprodukt 10f weist weitere Fäden 46f auf, mittels welchen das Reinigungselement 24f an den materiellen Griffkörper 16f angenäht ist. Es wäre auch denkbar, dass der Zahnseidefaden auf die erste Schicht 18f aufgenäht ist, wobei der Zahnseidefaden als Nähmedium verwendet wird. Die Schicht 18f weist eine Materialstärke von 0,5 mm bis 2 mm, vorzugsweise von 0,5 mm bis 1,5 mm, auf, bei einer Dichte von 180 g/m² bis 500 g/m², vorzugsweise 240 g/m² bis 400 g/m², insbesondere damit eine Verbindung mittels Nähen realisierbar ist.

Es wird insbesondere ein Verfahren zur Herstellung des Körperpflegeprodukts 10f offenbart. Bei dem Verfahren wird die Anwendungseinheit 12f auf den materiellen Griffkörper 16f aufgenäht. Bei einem Herstellungsverfahren wird insbesondere die Schicht 18f gestanzt (siehe Figur 6A). Es werden insbesondere zumindest die Bereiche des materiellen Griffkörpers 16f gestanzt, in welchen die Anwendungseinheit 12f angeordnet wird. Anschließend wird die Schicht 18f bereitgelegt und das Reinigungselement 24f bereitgestellt und mittels der Fäden 46f, 46f' angenäht (siehe Figur 6B). Ein fertiges Körperpflegeprodukt 10f ist in der Figur 6B gezeigt.

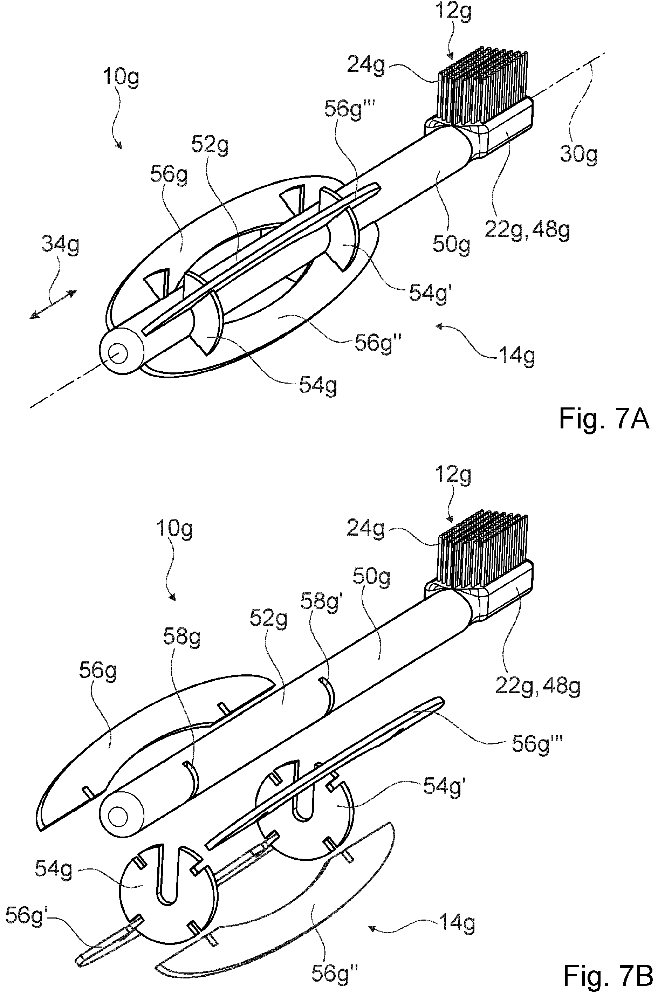

Die Figur 7A zeigt eine Vorderseite eines Körperpflegeprodukts 10g in einer schematischen perspektivischen Darstellung. Das Körperpflegeprodukt 10g ist im vorliegenden Fall als eine Zahnbürste ausgebildet.

Das Körperpflegeprodukt 10g weist zumindest eine Anwendungseinheit 12g auf. Ferner weist das Körperpflegeprodukt 10g zumindest eine Griffeinheit 14g auf.

Die zumindest eine Anwendungseinheit 12g weist einen Kopfbereich 48g auf. Der Kopfbereich 48g bildet einen Bürstenkopf des Körperpflegeprodukts 10g. Der Kopfbereich 48g bildet einen Reinigungselementträger 22g des Körperpflegeprodukts 10g. Im vorliegenden Fall ist der Kopfbereich 48g als ein Zahnbürstenkopf ausgebildet. Der Kopfbereich 48g weist insbesondere einen als Borstenträger ausgebildeten Grundkörper auf. Der Grundkörper des Kopfbereichs 48g ist vollständig aus einer Hartkomponente ausgebildet. Es wäre jedoch auch denkbar, dass der Grundkörper des Kopfbereichs 48g aus einer Hartkomponente und einer Weichkomponente gebildet ist. Der Grundkörper des Kopfbereichs 48g bildet einen Borstenträger aus. Das Körperpflegeprodukt 10g weist ferner im Kopfbereich 48g eine Mehrzahl von Borstenlöchern in Form von Ausnehmungen auf, welche jeweils ein Borstenbündel aufnehmen. Die Borstenbündel bilden Reinigungselemente 24g des Körperpflegeprodukts 10g.

Alternativ könnte das Körperpflegeprodukt 10g ein von dem Kopfbereich 48g aufgenommenes Borstenplättchen aufweisen, welches eine Mehrzahl von Borstenbündeln umfasst. Das Borstenplättchen ist beispielhaft von einem AFT-Plättchen gebildet.

Als Borsten kommen beliebige geeignete Borsten infrage. Ferner umfasst die Anwendungseinheit 12g im vorliegenden Fall einen Halsbereich 50g, welcher insbesondere den Kopfbereich 48g mit der Griffeinheit 14g verbindet. Der Halsbereich 50g bildet einen Hals aus.

Ferner weist das Körperpflegeprodukt 10g einen Grundkörper 52g auf. Der Grundkörper 52g besteht zumindest teilweise aus einer Hartkomponente. Der Grundkörper 52g besteht vollständig aus einer Hartkomponente. Der Grundkörper 52g ist einteilig ausgebildet. Der Grundkörper 52g bildet die Anwendungseinheit 12g aus. Der Grundkörper 52g bildet die Anwendungseinheit 12g vollständig aus. Der Grundkörper 52g bildet ferner teilweise die Griffeinheit 14g aus. Der Grundkörper 52g bildet einen Kern der Griffeinheit 14g aus. Der Grundkörper 52g weist in einem Bereich der Griffeinheit 14g eine zumindest annähernd zylindrische Grundform auf. Der Grundkörper 52g erstreckt sich entlang einer Längsachse 30g entlang des gesamten Körperpflegeprodukts 10g. Der Grundkörper 52g weist an einem hinteren Ende des Körperpflegeprodukts 10g ein abgerundetes Ende auf. Der Grundkörper 52g kann aus Papierwerkstoff bestehen.

Die Griffeinheit 14g ist zu einem Greifen durch den Bediener vorgesehen. Die Griffeinheit 14g weist eine zylindrische Grundform auf. Die Griffeinheit 14g weist ferner einen materiellen Griffkörper 16g auf. Der materielle Griffkörper 16g bildet ein materielles Volumen der Griffeinheit 14g. Der materielle Griffkörper 16g weist eine ellipsoide Form auf. Der materielle Griffkörper 16g der Griffeinheit 14g besteht zumindest teilweise, insbesondere zumindest zu einem Großteil, aus einem Papierwerkstoff. Der materielle Griffkörper 16g besteht vollständig aus einem Papierwerkstoff. Der materielle Griffkörper 16g der Griffeinheit 14g weist mehrere Teilelemente 54g, 54g', 56g, 56g', 56g", 56g‴ auf, welche zu einem Zusammenstecken vorgesehen sind. Dazu wird insbesondere ein Papierwerkstoff in die Teilelemente 54g, 54g', 56g, 56g', 56g", 56g‴ geschnitten und anschließend die Teilelemente 54g, 54g', 56g, 56g', 56g", 56g‴ zusammengesteckt.

Der Grundkörper 52g und der Griffkörper 16g bilden einen Formschluss aus. Die Teilelemente 54g, 54g', 56g, 56g', 56g", 56g‴ des materiellen Griffkörpers 16g sind an den Grundkörper 52g angesteckt. Der Grundkörper 52g weist dazu Formschlussausnehmungen 58g, 58g' auf, welche zu einer formschlüssigen Verbindung mit zumindest zwei Teilelementen 54g, 54g' des Griffkörpers 16g vorgesehen sind. Die ersten zwei Teilelemente 54g, 54g' sind von Scheiben aus einem Papierwerkstoff gebildet, welche jeweils eine Aufnahmenut zu einem Aufschieben auf die Formschlussausnehmungen 58g, 58g' aufweisen. Ferner weisen die ersten zwei Teilelemente 54g, 54g' jeweils an einer Außenseite umlaufend Formschlussausnehmungen zu einer formschlüssigen Verbindung mit den weiteren Teilelementen 56g, 56g', 56g", 56g‴ des Griffkörpers 16g auf. Die weiteren Teilelemente 56g, 56g', 56g", 56g‴ sind jeweils von bogenförmigen Elementen gebildet, welche sich entlang der Längsachse 30g entlang des Grundkörpers 52g erstrecken. Hierdurch kann insbesondere eine vorteilhaft dreidimensionale Griffeinheit 14g bereitgestellt werden, wobei ein Materialaufwand geringgehalten werden kann. Das Körperpflegeprodukt 10g beziehungsweise dessen Körper kann dabei insbesondere aus 2 bis 25, vorzugsweise 4 bis 12, Einzelteilen bestehen.

Ein Zusammenstecken des Körperpflegeprodukts 10g kann dabei sowohl bereits bei einer Herstellung als auch durch einen Anwender selbst erfolgen. Bei einem Zusammenstecken durch den Anwender kann insbesondere eine Verpackungsgröße des Körperpflegeprodukts 10g vorteilhaft geringgehalten werden (siehe Figur 7B).

Das Körperpflegeprodukt 10g ist in einem Verfahren hergestellt. In dem Verfahren wird in einem ersten Verfahrensschritt der Grundkörper 52g mit der Anwendungseinheit 12g in einem Spritzgussverfahren hergestellt und bereitgestellt. Anschließend werden die Teilelemente 54g, 54g', 56g, 56g', 56g", 56g‴ geschnitten und optional die Teilelemente 54g, 54g', 56g, 56g', 56g", 56g‴ an den Grundkörper 52g angesteckt und zusammengesteckt. Es können allenfalls Sicherungen für die Teilelemente 54g, 54g', 56g, 56g', 56g", 56g‴ vorgesehen sein.

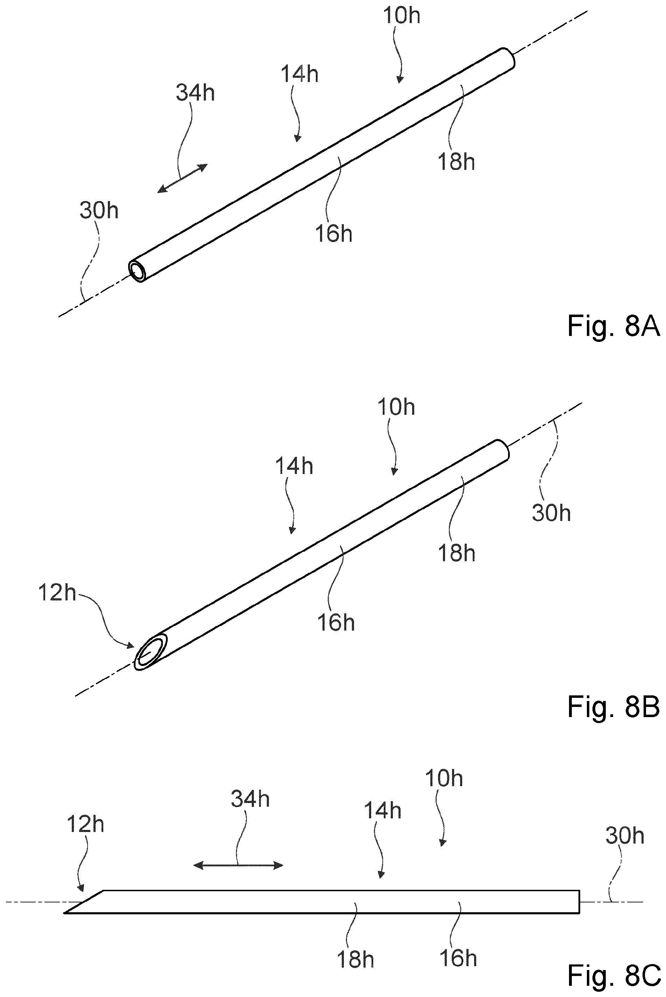

Die Figur 8A zeigt ein Körperpflegeprodukt 10h in einer schematischen perspektivischen

Darstellung. Diese Ausführungsform ist nicht Teil der beanspruchten Erfindung. Das Körperpflegeprodukt 10h ist im vorliegenden Fall als ein Zahnstocher ausgebildet.

Das Körperpflegeprodukt 10h weist eine Anwendungseinheit 12h auf. Ferner weist das Körperpflegeprodukt 10h zumindest eine mit der Anwendungseinheit 12h verbundene Griffeinheit 14h auf.

Die Griffeinheit 14h ist zu einem Greifen durch den Bediener vorgesehen. Die Griffeinheit 14h weist eine zylindrische Grundform auf. Die Griffeinheit 14h weist ferner einen materiellen Griffkörper 16h auf. Der materielle Griffkörper 16h bildet ein materielles Volumen der Griffeinheit 14h. Der materielle Griffkörper 16h weist eine zylindrische Form auf. Der materielle Griffkörper 16h der Griffeinheit 14h besteht zumindest teilweise, insbesondere zumindest zu einem Großteil, aus einem Papierwerkstoff. Der materielle Griffkörper 16h besteht vollständig aus einem Papierwerkstoff. Der materielle Griffkörper 16h der Griffeinheit 14h weist zumindest eine Schicht 18h aus einem Papierwerkstoff auf, welche gerollt ausgebildet ist. Der materielle Griffkörper 16h weist beispielhaft genau eine Schicht 18h auf, es wäre jedoch auch ein mehrschichtiger Aufbau denkbar. Die Schicht 18h aus dem Papierwerkstoff ist um eine definierte Rollachse aufgerollt. Die Rollachse verläuft parallel, insbesondere koaxial, zu der Längsachse 30h des Körperpflegeprodukts 10h. Die Schicht 18h wird bei einer Herstellung des materiellen Griffkörpers 16h aufgerollt. Die Schicht 18h bildet in einem Endzustand, insbesondere nach einer Herstellung eine Rolle, insbesondere eine zylindrische Rolle, aus. Die Schicht 18h des materiellen Griffkörpers 16h ist in eine zylindrische Form gerollt. Die Schicht 18h des materiellen Griffkörpers 16h ist mittels Kleben, Schweißen und/oder Laminieren in der Rolle mit sich selbst verbunden. Der Papierwerkstoff ist zu einer hohlzylindrischen Rolle gerollt. Der materielle Griffkörper 16h kann insbesondere gerade oder spiralförmig gerollt sein.

Die Anwendungseinheit 12h ist einstückig mit der Griffeinheit 14h ausgebildet. Die Anwendungseinheit 12h ist durch ein schräges Abschneiden eines Endes des materiellen Griffkörpers 16h realisiert.

Zu einer Herstellung des Körperpflegeprodukts 10h werden dabei insbesondere Papierbänder von Rollen abgewickelt und gemeinsam zu einem Rohr geformt. Die Papierbänder werden dabei insbesondere auf einen Kern abgerollt und dann darauf verbunden. Darauffolgend kann ein Ende des Rohrs schräg geschnitten und/oder angespitzt werden, sodass an einem Ende die Anwendungseinheit 12h entsteht. Die Anwendungseinheit 12h ist dabei insbesondere einstückig mit der Griffeinheit 14h ausgebildet. Optional wäre denkbar, dass eine Oberfläche des Körperpflegeprodukts 10h nachbehandelt wird. Dabei wäre denkbar, dass das Körperpflegeprodukt 10h für weniger Wasseraufnahme lackiert wird oder das Körperpflegeprodukt 10h bedruckt wird.

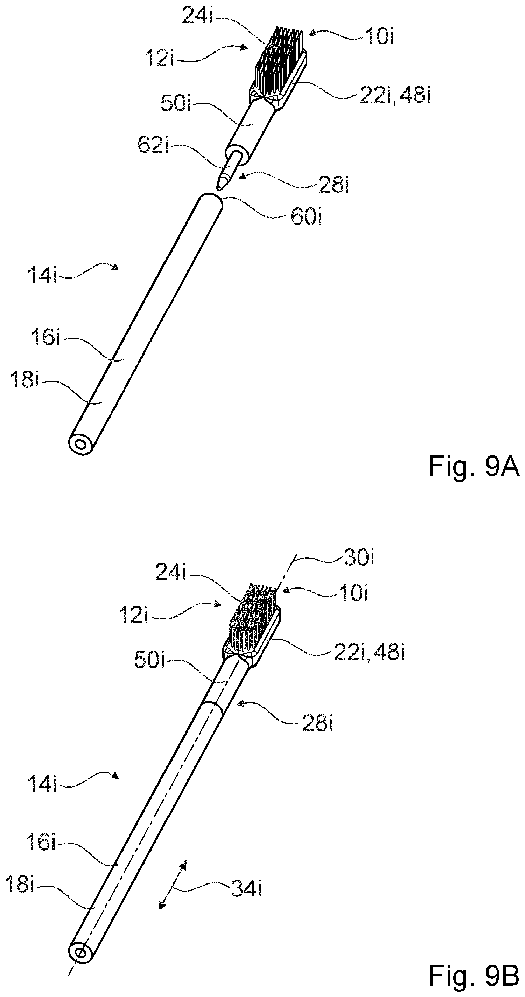

Die Figur 9A zeigt ein Körperpflegeprodukt 10i in einer schematischen perspektivischen Darstellung. Das Körperpflegeprodukt 10i ist im vorliegenden Fall als eine Zahnbürste ausgebildet.

Das Körperpflegeprodukt 10i weist eine Anwendungseinheit 12i auf. Ferner weist das Körperpflegeprodukt 10i zumindest eine mit der Anwendungseinheit 12i verbundene Griffeinheit 14i auf.

Die Anwendungseinheit 12i ist beispielhaft von einem Wechselkopf für eine Zahnbürste gebildet. Die zumindest eine Anwendungseinheit 12i weist einen Kopfbereich 48i auf. Der Kopfbereich 48i bildet einen Bürstenkopf des Körperpflegeprodukts 10i. Im vorliegenden Fall ist der Kopfbereich 48i als ein Zahnbürstenkopf ausgebildet. Der Kopfbereich 48i weist insbesondere einen als Borstenträger ausgebildeten Grundkörper auf. Der Grundkörper des Kopfbereichs 48i ist vollständig aus einer Hartkomponente ausgebildet. Es wäre jedoch auch denkbar, dass der Grundkörper des Kopfbereichs 48i aus einer Hartkomponente und einer Weichkomponente gebildet ist. Der Grundkörper des Kopfbereichs 48i bildet einen Borstenträger aus. Das Körperpflegeprodukt 10i weist ferner im Kopfbereich 48i eine Mehrzahl von Borstenlöchern in Form von Ausnehmungen auf, welche jeweils ein Borstenbündel aufnehmen.

Alternativ könnte das Körperpflegeprodukt 10i ein von dem Kopfbereich 48i aufgenommenes Borstenplättchen aufweisen, welches eine Mehrzahl von Borstenbündeln umfasst. Das Borstenplättchen ist beispielhaft von einem AFT-Plättchen gebildet.

Als Borsten kommen beliebige geeignete Borsten infrage. Ferner umfasst die Anwendungseinheit 12i im vorliegenden Fall einen Halsbereich 50i, welcher insbesondere den Kopfbereich 48i mit der Griffeinheit 14i verbindet. Der Halsbereich 50i bildet einen Hals aus.

Die Griffeinheit 14i ist zu einem Greifen durch den Bediener vorgesehen. Die Griffeinheit 14i weist eine zylindrische Grundform auf. Die Griffeinheit 14i weist ferner einen materiellen Griffkörper 16i auf. Der materielle Griffkörper 16i bildet ein materielles Volumen der Griffeinheit 14i. Der materielle Griffkörper 16i weist eine zylindrische Form auf. Der materielle Griffkörper 16i der Griffeinheit 14i besteht zumindest teilweise, insbesondere zumindest zu einem Großteil, aus einem Papierwerkstoff. Der materielle Griffkörper 16i besteht vollständig aus einem Papierwerkstoff. Der materielle Griffkörper 16i der Griffeinheit 14i weist zumindest eine Schicht 18i aus einem Papierwerkstoff auf, welche gerollt ausgebildet ist. Der materielle Griffkörper 16i weist beispielhaft genau eine Schicht 18i auf, es wäre jedoch auch ein mehrschichtiger Aufbau denkbar. Die Schicht 18i aus dem Papierwerkstoff ist um eine definierte Rollachse aufgerollt. Die Rollachse verläuft parallel, insbesondere koaxial, zu einer Längsachse 30i des Körperpflegeprodukts 10i. Die Schicht 18i wird bei einer Herstellung des materiellen Griffkörpers 16i aufgerollt. Die Schicht 18i bildet in einem Endzustand, insbesondere nach einer Herstellung eine Rolle, insbesondere eine zylindrische Rolle, aus. Die Schicht 18i des materiellen Griffkörpers 16i ist in eine zylindrische Form gerollt. Die Schicht 18i des materiellen Griffkörpers 16i ist mittels Kleben, Schweißen und/oder Laminieren in der Rolle mit sich selbst verbunden. Der Papierwerkstoff ist zu einer hohlzylindrischen Rolle gerollt. Der materielle Griffkörper 16i kann insbesondere gerade oder spiralförmig gerollt sein.

Ferner weist das Körperpflegeprodukt 10i eine Steckverbindungseinheit 28i auf, welche zu einer lösbaren Steckverbindung der Anwendungseinheit 12i, welche insbesondere als ein Zahnbürstenkopf ausgebildet ist, mit der Griffeinheit 14i vorgesehen ist. Die Steckverbindungseinheit 28i ist zumindest teilweise einstückig mit der Anwendungseinheit 12i und zumindest teilweise einstückig mit der Griffeinheit 14i ausgebildet. Die Steckverbindungseinheit 28i weist ein erstes Steckverbindungselement 60i auf, welches einstückig mit der Griffeinheit 14i verbunden ist, und ein zu dem ersten Steckverbindungselement 60i korrespondierendes zweites Steckverbindungselement 62i auf, welches fest mit der Anwendungseinheit 12i verbunden ist. Das erste Steckverbindungselement 60i ist von einer zylindrischen Ausnehmung in dem materiellen Griffkörper 16i gebildet. Die Ausnehmung kann dabei bereits durch eine hohlzylindrische Herstellung des Griffkörpers 16i oder durch eine nachträgliche Bohrung hergestellt werden. Das zweite Steckverbindungselement 62i ist von einem stiftförmigen Fortsatz gebildet, welcher dazu vorgesehen ist, in das erste Steckverbindungselement 60i einzugreifen. Über die Steckverbindungseinheit 28i kann insbesondere die Anwendungseinheit 12i von dem materiellen Griffkörper 16i getrennt werden. Die Steckverbindungseinheit 28i ist insbesondere zu einer Bereitstellung einer lösbaren oder unlösbaren Rastverbindung vorgesehen.

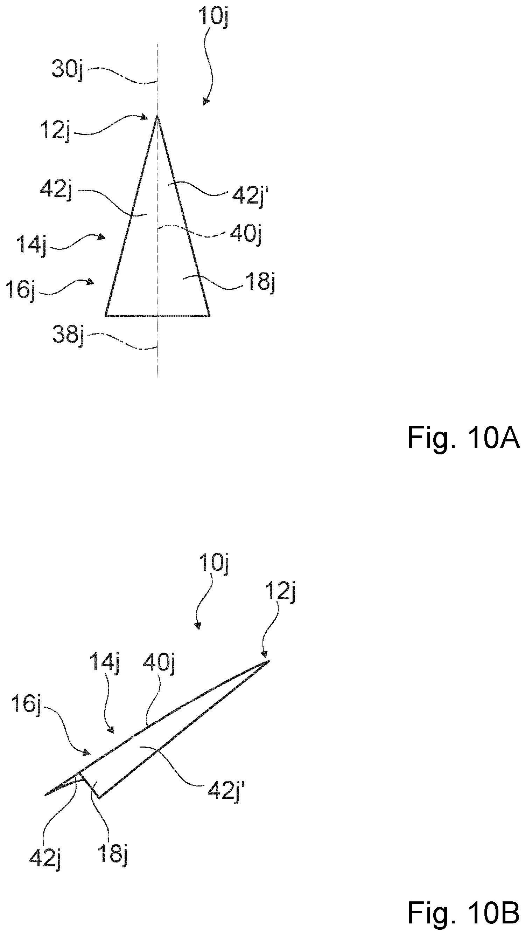

Die Figur 10A zeigt ein als Zahnstocher ausgebildetes Körperpflegeprodukt 10j in einem ungefalteten Zustand bei einer Herstellung. Das Körperpflegeprodukt 10j weist eine Anwendungseinheit 12j auf. Ferner weist das Körperpflegeprodukt 10j zumindest eine mit der Anwendungseinheit 12j verbundene Griffeinheit 14j auf. Diese Ausführungsform ist nicht Teil der beanspruchten Erfindung.

Die Griffeinheit 14j ist zu einem Greifen durch den Bediener vorgesehen. Die Griffeinheit 14j weist ferner einen materiellen Griffkörper 16j auf. Der materielle Griffkörper 16j bildet ein materielles Volumen der Griffeinheit 14j. Der materielle Griffkörper 16j weist in einem Endzustand eine flache, dreieckige Grundform auf. Der materielle Griffkörper 16j der Griffeinheit 14j besteht zumindest teilweise, insbesondere zumindest zu einem Großteil, aus einem Papierwerkstoff. Der materielle Griffkörper 16j besteht vollständig aus einem Papierwerkstoff. Der materielle Griffkörper 16j der Griffeinheit 14j weist zumindest eine Schicht 18j aus einem Papierwerkstoff auf, die in einem Gebrauchszustand zumindest teilweise gefaltet ausgebildet ist. Der materielle Griffkörper 16j der Griffeinheit 14j weist beispielhaft genau eine Schicht 18j aus einem Papierwerkstoff auf. Die Schicht 18j umfasst eine Faltachse 38j. In der Faltachse 38j weist die Schicht 18j eine Faltkante 40j auf. Die Schicht 18j ist in einem Gebrauchszustand an der Faltkante 40j um die Faltachse 38j gefaltet. Die Schicht 18j ist in der Faltkante 40j perforiert, gerillt und/oder geprägt ausgebildet, um ein definiertes Falten zu ermöglichen. Die Faltachse 38j der Schicht 18j ist parallel senkrecht zur Haupterstreckungsrichtung 34j des Körperpflegeprodukts 10j.

Der materielle Griffkörper 16j kann dabei insbesondere aus Papierbögen oder ab Rolle gefaltet werden.

Die Anwendungseinheit 12j ist einstückig mit der Griffeinheit 14j ausgebildet. Die Anwendungseinheit 12j ist durch Falten eines spitzen Endes des materiellen Griffkörpers 16j realisiert. Das Körperpflegeprodukt 10j kann im Gebrauch insbesondere zu einem Dreieck gefaltet werden (siehe Figur 10B).

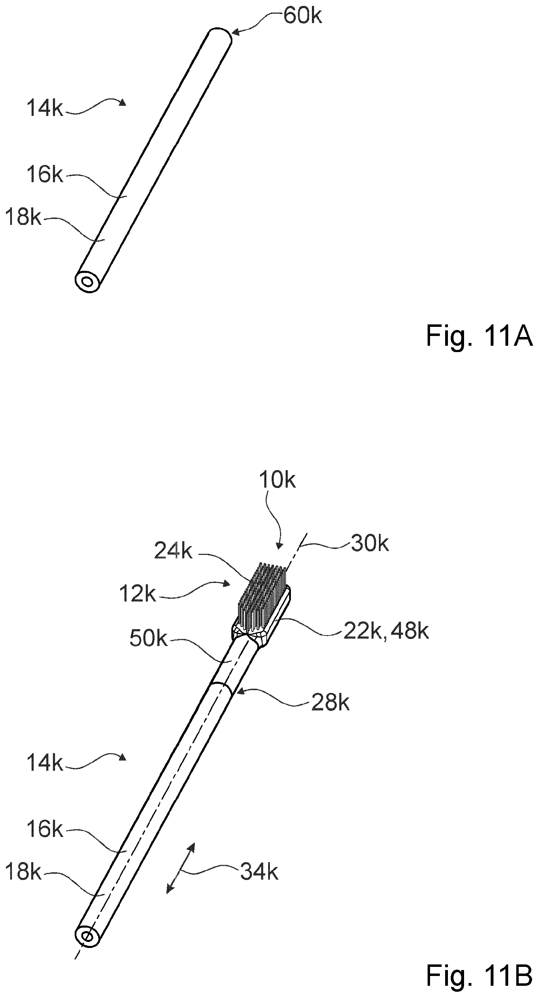

Die Figur 11A zeigt eine Griffeinheit 14k des Körperpflegeprodukts 10k in einer schematischen perspektivischen Darstellung. Das Körperpflegeprodukt 10k ist im vorliegenden Fall als eine Zahnbürste ausgebildet.

Das Körperpflegeprodukt 10k weist eine Anwendungseinheit 12k auf. Ferner weist das Körperpflegeprodukt 10k die mit der Anwendungseinheit 12k verbundene Griffeinheit 14k auf.

Die Anwendungseinheit 12k ist in einem Spitzgussverfahren hergestellt. Die zumindest eine Anwendungseinheit 12k weist einen Kopfbereich 48k auf. Der Kopfbereich 48k bildet einen Bürstenkopf des Körperpflegeprodukts 10k. Im vorliegenden Fall ist der Kopfbereich 48k als ein Zahnbürstenkopf ausgebildet. Der Kopfbereich 48k weist insbesondere einen als Borstenträger ausgebildeten Grundkörper auf. Der Grundkörper des Kopfbereichs 48k ist vollständig aus einer Hartkomponente ausgebildet. Es wäre jedoch auch denkbar, dass der Grundkörper des Kopfbereichs 48k aus einer Hartkomponente und einer Weichkomponente gebildet ist. Der Grundkörper des Kopfbereichs 48k bildet einen Borstenträger aus. Das Körperpflegeprodukt 10k weist ferner im Kopfbereich 48k eine Mehrzahl von Borstenlöchern in Form von Ausnehmungen auf, welche jeweils ein Borstenbündel aufnehmen.

Alternativ könnte das Körperpflegeprodukt 10k ein von dem Kopfbereich 48k aufgenommenes Borstenplättchen aufweisen, welches eine Mehrzahl von Borstenbündeln umfasst. Das Borstenplättchen ist beispielhaft von einem AFT-Plättchen gebildet.

Als Borsten kommen beliebige geeignete Borsten infrage. Ferner umfasst die Anwendungseinheit 12k im vorliegenden Fall einen Halsbereich 50k, welcher insbesondere den Kopfbereich 48k mit der Griffeinheit 14k verbindet. Der Halsbereich 50k bildet einen Hals aus.

Die Griffeinheit 14k ist zu einem Greifen durch den Bediener vorgesehen. Die Griffeinheit 14k weist eine zylindrische Grundform auf. Die Griffeinheit 14k weist ferner einen materiellen Griffkörper 16k auf. Der materielle Griffkörper 16k bildet ein materielles Volumen der Griffeinheit 14k. Der materielle Griffkörper 16k weist eine zylindrische Form auf. Der materielle Griffkörper 16k der Griffeinheit 14k besteht zumindest teilweise, insbesondere zumindest zu einem Großteil, aus einem Papierwerkstoff. Der materielle Griffkörper 16k besteht vollständig aus einem Papierwerkstoff. Der materielle Griffkörper 16k der Griffeinheit 14k weist zumindest eine Schicht 18k aus einem Papierwerkstoff auf, welche gerollt ausgebildet ist. Der materielle Griffkörper 16k weist beispielhaft genau eine Schicht 18k auf, es wäre jedoch auch ein mehrschichtiger Aufbau denkbar. Die Schicht 18k aus dem Papierwerkstoff ist um eine definierte Rollachse aufgerollt. Die Rollachse verläuft parallel, insbesondere koaxial, zu der Längsachse 30k des Körperpflegeprodukts 10k. Die Schicht 18k wird bei einer Herstellung des materiellen Griffkörpers 16k aufgerollt. Die Schicht 18k bildet in einem Endzustand, insbesondere nach einer Herstellung eine Rolle, insbesondere eine zylindrische Rolle, aus. Die Schicht 18k des materiellen Griffkörpers 16k ist in eine zylindrische Form gerollt. Die Schicht 18k des materiellen Griffkörpers 16k ist mittels Kleben, Schweißen und/oder Laminieren in der Rolle mit sich selbst verbunden. Der Papierwerkstoff ist zu einer hohlzylindrischen Rolle gerollt. Der materielle Griffkörper 16k kann insbesondere gerade oder spiralförmig gerollt sein.

Die Anwendungseinheit 12k ist an die Griffeinheit 14k mittels eines Spitzgussverfahrens angespritzt. Die Griffeinheit 14k wird dazu insbesondere in ein Spritzgusswerkzeug für die Anwendungseinheit 12k eingelegt, wobei die Anwendungseinheit 12k direkt an der Griffeinheit 14k hergestellt wird. Eine Verbindung der Umspritzung zu dem Papierwerkstoff kann dabei insbesondere durch Hinterschnitte, Durchbrüche, also insbesondere einen Formschluss, oder chemisch, wie insbesondere durch einen Materialschluss, erreicht werden. Der Papierwerkstoff kann dazu insbesondere vorbehandelt ausgebildet sein, insbesondere vor dem Umspritzen, damit die Verbindung erstellt werden kann, beispielsweise mittels spezieller Lacke und/oder Beschichtungen. Die Figur 11B zeigt das fertige Körperpflegeprodukt 10k.

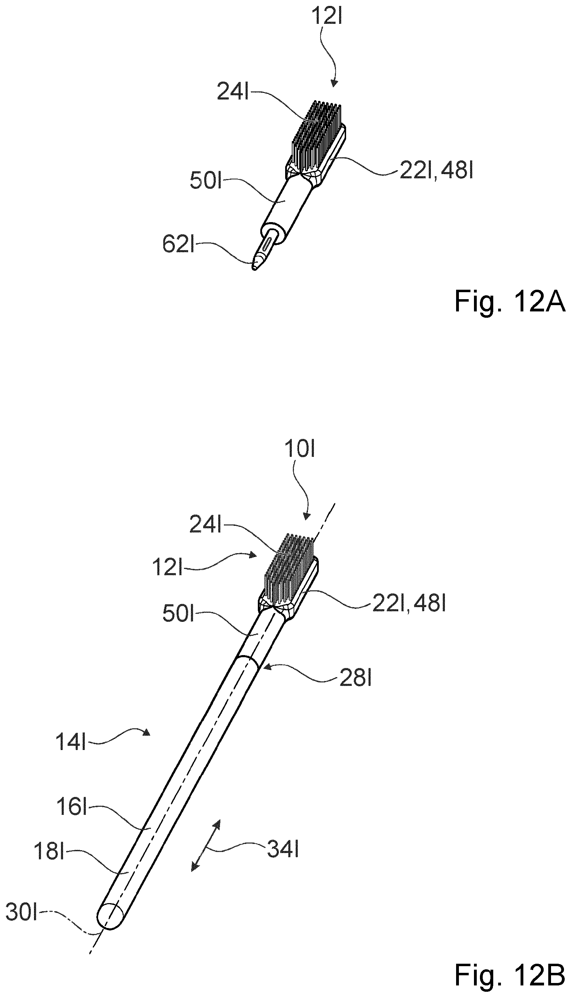

Die Figur 12A zeigt eine Anwendungseinheit 12l eines Körperpflegeprodukts 10l in einer schematischen perspektivischen Darstellung. Das Körperpflegeprodukt 10l ist im vorliegenden Fall als eine Zahnbürste ausgebildet.

Das Körperpflegeprodukt 10l weist die Anwendungseinheit 121 auf. Ferner weist das Körperpflegeprodukt 10l zumindest eine mit der Anwendungseinheit 12l verbundene Griffeinheit 14l auf.

Die Anwendungseinheit 12l ist in einem Kunststoff-Spitzgussverfahren hergestellt. Die zumindest eine Anwendungseinheit 12l weist einen Kopfbereich 48l auf. Der Kopfbereich 48l bildet einen Bürstenkopf des Körperpflegeprodukts 10l. Im vorliegenden Fall ist der Kopfbereich 48l als ein Zahnbürstenkopf ausgebildet. Der Kopfbereich 48l weist insbesondere einen als Borstenträger ausgebildeten Grundkörper auf. Der Grundkörper des Kopfbereichs 48l ist vollständig aus einer Hartkomponente ausgebildet. Es wäre jedoch auch denkbar, dass der Grundkörper des Kopfbereichs 48l aus einer Hartkomponente und einer Weichkomponente gebildet ist. Der Grundkörper des Kopfbereichs 48l bildet einen Borstenträger aus. Das Körperpflegeprodukt 10l weist ferner im Kopfbereich 48l eine Mehrzahl von Borstenlöchern in Form von Ausnehmungen auf, welche jeweils ein Borstenbündel aufnehmen.

Alternativ könnte das Körperpflegeprodukt 10l ein von dem Kopfbereich 48l aufgenommenes Borstenplättchen aufweisen, welches eine Mehrzahl von Borstenbündeln umfasst. Das Borstenplättchen ist beispielhaft von einem AFT-Plättchen gebildet.

Als Borsten kommen beliebige geeignete Borsten infrage. Ferner umfasst die Anwendungseinheit 12l im vorliegenden Fall einen Halsbereich 50l, welcher insbesondere den Kopfbereich 48l mit der Griffeinheit 14l verbindet. Der Halsbereich 50l bildet einen Hals aus.

Die Griffeinheit 14l ist zu einem Greifen durch den Bediener vorgesehen. Die Griffeinheit 14l weist eine zylindrische Grundform auf. Die Griffeinheit 14l weist ferner einen materiellen Griffkörper 16l auf. Der materielle Griffkörper 16l bildet ein materielles Volumen der Griffeinheit 14l. Der materielle Griffkörper 16l weist eine zylindrische Form auf. Der materielle Griffkörper 16l der Griffeinheit 14l besteht zumindest teilweise, insbesondere zumindest zu einem Großteil, aus einem Papierwerkstoff. Der materielle Griffkörper 16l besteht vollständig aus einem Papierwerkstoff. Der Griffkörper 16l ist aus einem Papierwerkstoff gegossen. Der Papierwerkstoff wird dabei insbesondere spitzgussähnlich verarbeitet. Die Rohprodukte setzen sich insbesondere aus Industriestärke, vorzugsweise aus Kartoffeln, Papierfasern, Wasser, inklusive Zumischung zusammen. Die dadurch entstehende Papiermischung wird anschließend für die Weiterverarbeitung mittels Spritzgusstechnik verwendet. Der Papierspritzguss erfolgt insbesondere in drei Schritten. In einem ersten Schritt erfolgt eine Einspritzung, bei der die Papiermixtur in ein Aluminium-Werkzeug gespritzt wird. In einem zweiten Schritt erfolgt ein Backprozess, bei dem die Papiermixtur im Werkzeug gebacken, insbesondere erhitzt, wird. In einem dritten Schritt erfolgt eine Entformung, bei der die fertige Verpackung entnommen werden kann.

Die Anwendungseinheit 12l ist mit dem Papierwerkstoff des Griffkörpers 16l umspritzt. Die Anwendungseinheit 12l ist gemäß eines bekannten Verfahrens, wie insbesondere eines Kunststoff-Spritzgussverfahrens, hergestellt (siehe Figur 12A). Eine Verbindung der Umspritzung zu dem funktionalen Teil kann dabei insbesondere durch Hinterschnitte, Durchbrüche, also insbesondere einen Formschluss, oder chemisch, wie insbesondere durch einen Materialschluss, erreicht werden. Die Anwendungseinheit 12l weist einen Kernfortsatz 64l auf, welcher in den Griffkörper 16l ragt. Die Anwendungseinheit 12l kann insbesondere vorbehandelt ausgebildet sein, insbesondere vor dem Umspritzen, damit die Verbindung erstellt werden kann, beispielsweise mittels spezieller Lacke und/oder Beschichtungen. Die Griffeinheit 14l wird nach einer Herstellung der Anwendungseinheit 12l in einem Papierspritzguss an die Anwendungseinheit 12l angeformt. Die Figur 12B zeigt das fertige Körperpflegeprodukt 10l.

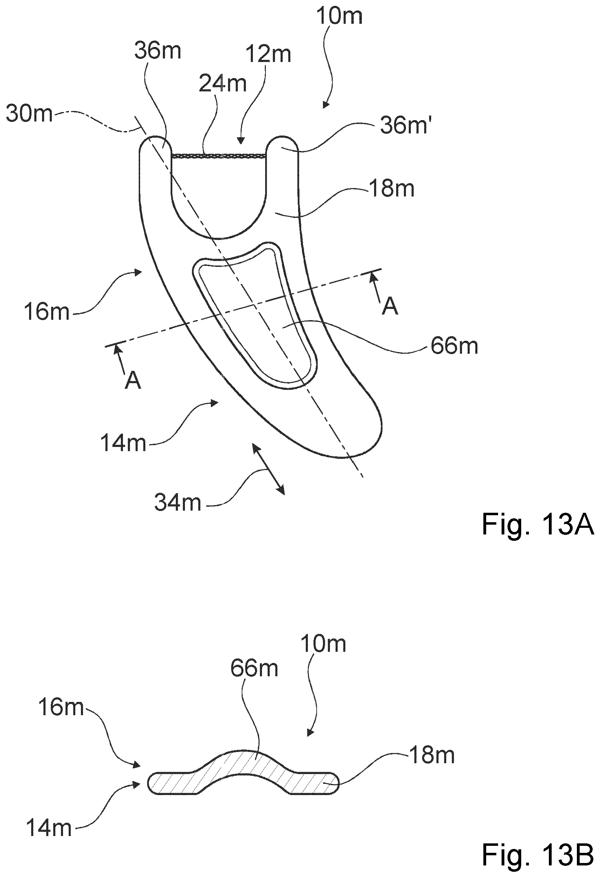

Die Figur 13A zeigt ein als Flosser ausgebildetes Körperpflegeprodukt 10m. Diese Ausführungsform ist nicht Teil der beanspruchten Erfindung. Das Körperpflegeprodukt 10m weist eine Anwendungseinheit 12m auf. Ferner weist das Körperpflegeprodukt 10m zumindest eine mit der Anwendungseinheit 12m verbundene Griffeinheit 14m auf. Die Anwendungseinheit 12m ist an der Oberseite des Körperpflegeprodukts 10m angeordnet. Die Anwendungseinheit 12m bildet einen obersten Punkt des Körperpflegeprodukts 10m. Die Anwendungseinheit 12m weist ein Reinigungselement 24m auf, welches von einer Zahnseide gebildet ist. Die Anwendungseinheit 12m besteht insbesondere aus dem Reinigungselement 24m.

Die Griffeinheit 14m ist zu einem Greifen durch den Bediener vorgesehen. Die Griffeinheit 14m weist ferner einen materiellen Griffkörper 16m auf. Der materielle Griffkörper 16m bildet ein materielles Volumen der Griffeinheit 14m. Der materielle Griffkörper 16m weist in einem Endzustand eine flache Grundform auf, welche sich zu der Anwendungseinheit 12m hin in zwei Arme 36m, 36m' aufteilt. Die Arme 36m, 36m' sind dazu vorgesehen, das Reinigungselement 24m beidseitig zu halten. Der materielle Griffkörper 16m der Griffeinheit 14m besteht zumindest teilweise, insbesondere zumindest zu einem Großteil, aus einem Papierwerkstoff. Der materielle Griffkörper 16m besteht vollständig aus einem Papierwerkstoff. Der materielle Griffkörper 16m der Griffeinheit 14m weist zumindest eine Schicht 18m aus einem Papierwerkstoff auf. Die Schicht 18m des materiellen Griffkörpers 16m wird geschnitten oder gestanzt.

Ferner ist die Schicht 18m des Griffkörpers 16m verformt, bevorzugt tiefgezogen, insbesondere mittels Drucks und Wärme. Der materielle Griffkörper 16m weist eine ovale Verformung 66m auf. Hierdurch wird insbesondere beispielsweise eine Greifgeometrie erzeugt. Es kann insbesondere ein Körperpflegeprodukt 10m mit einer voluminösen Griffeinheit 14m bereitgestellt werden.

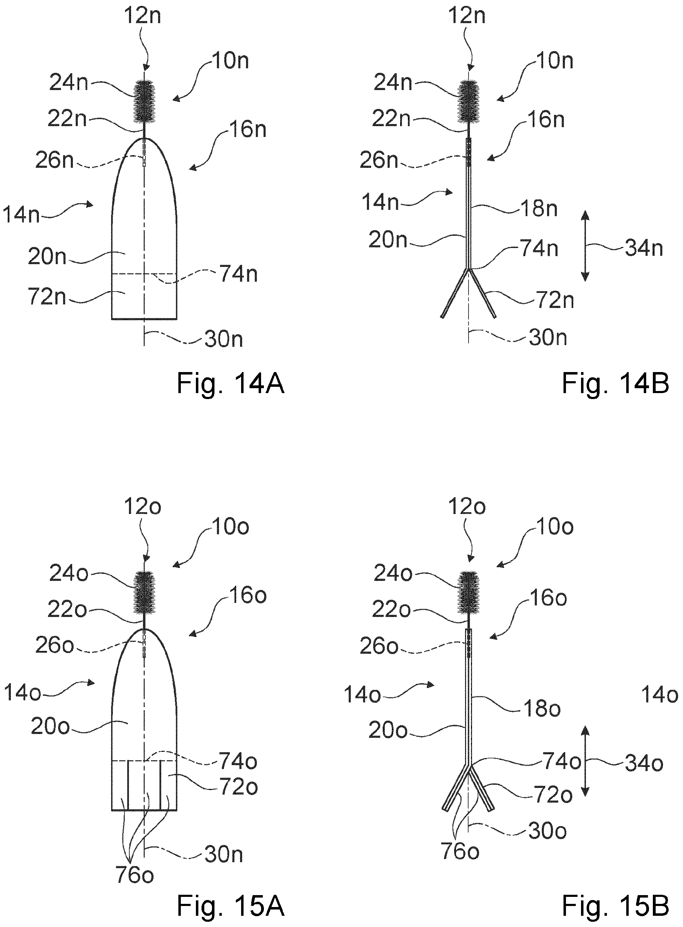

Die Figur 14A zeigt eine Anwendungseinheit 12n eines Körperpflegeprodukts 10n in einer schematischen Darstellung. Das Körperpflegeprodukt 10n ist im vorliegenden Fall als eine Interdentalbürste ausgebildet.

Das Körperpflegeprodukt 10n weist die Anwendungseinheit 12n auf. Ferner weist das Körperpflegeprodukt 10n zumindest eine mit der Anwendungseinheit 12n verbundene Griffeinheit 14n auf.

Die Anwendungseinheit 12n ist an der Oberseite des Körperpflegeprodukts 10n angeordnet. Die Anwendungseinheit 12n bildet einen obersten Punkt des Körperpflegeprodukts 10n.

Die Griffeinheit 14n ist zu einem Greifen durch den Bediener vorgesehen. Die Griffeinheit 14n weist ferner einen materiellen Griffkörper 16n auf. Der materielle Griffkörper 16n bildet ein materielles Volumen der Griffeinheit 14n. Der materielle Griffkörper 16n weist eine flache, längliche Grundform auf. Der materielle Griffkörper 16n der Griffeinheit 14n besteht zumindest teilweise, insbesondere zumindest zu einem Großteil, aus einem Papierwerkstoff. Der materielle Griffkörper 16n besteht vollständig aus einem Papierwerkstoff. Der materielle Griffkörper 16n der Griffeinheit 14n weist zumindest zwei Schichten 18n, 20n aus einem Papierwerkstoff auf, die geschichtet miteinander verbunden sind. Der materielle Griffkörper 16n der Griffeinheit 14n weist beispielhaft genau zwei Schichten 18n, 20n aus einem Papierwerkstoff auf. Vorzugsweise sind von 2 bis 6, vorzugsweise von 2 bis 4, Schichten 18n, 20n vorgesehen. Bevorzugt sind die zumindest zwei Schichten 18n, 20n senkrecht zu einer Haupterstreckungsebene der Schichten 18n, 20n übereinander angeordnet und miteinander verbunden. Die Schichten 18n, 20n sind im Wesentlichen identisch ausgebildet und bilden insbesondere gegenüberliegende Außenseiten des Körperpflegeprodukts 10n. Die Schichten 18n, 20n sind beispielsweise mittels Kleben und/oder Pressen und/oder Schweißen und/oder Laminieren miteinander verbunden. Die Schichten 18n, 20n werden insbesondere bei einer Herstellung des Körperpflegeprodukts 10n miteinander verbunden.

Ferner weist die Anwendungseinheit 12n einen Reinigungselementträger 22n und mehrere mit dem Reinigungselementträger 22n verbundene, insbesondere eingedrehte, Reinigungselemente 24n auf. Der Reinigungselementträger 22n ist beispielhaft von einem Draht gebildet, wobei die Reinigungselemente 24 von in den Draht eingedrehten Borsten gebildet sind.

Der Reinigungselementträger 22n ist zu einer Verbindung mit der Griffeinheit 14n mit einem den Reinigungselementen 24n abgewandten Verbindungsabschnitt 26n mit dem materiellen Griffkörper 16n verbunden. Der Verbindungsabschnitt 26n ist insbesondere von einem den Reinigungselementen 24 abgewandten Drahtende gebildet. Der Verbindungsabschnitt 26n des Reinigungselementträgers 22n der Anwendungseinheit 12n ragt, insbesondere in einem hergestellten Zustand des Körperpflegeprodukts 10n, in den materiellen Griffkörper 16n und ist von diesem umschlossen. Die Anwendungseinheit 12n ist über den Verbindungsabschnitt 26n direkt mit dem materiellen Griffkörper 16n verbunden. Der Verbindungsabschnitt 26n ist zwischen den zumindest zwei Schichten 18n, 20n des materiellen Griffkörpers 16n an der Griffeinheit 14n angeordnet. Ferner ist der Reinigungselementträger 22n der Anwendungseinheit 12n zu einer teilweise formschlüssigen Verbindung mit der Griffeinheit 14n in dem Verbindungsabschnitt 26n nicht weiter sichtbar gebogen ausgebildet.

Ferner bildet die Griffeinheit 14n einen Standfuß 72n aus. Die zwei Schichten 18n, 20n sind dazu in einem unteren, der Anwendungseinheit 12n angewandten Abschnitt unverbunden ausgebildet. Der Standfuß 72n wird durch Auseinanderklappen der zwei Schichten 18n, 20n in dem unteren Abschnitt bereitgestellt. Es sind insbesondere Faltkanten 74n vorgesehen, an welchen die Schichten 18n, 20n in dem unteren Abschnitt voneinander weggeklappt werden können (siehe Figur 14B). Dies kann zusätzlich zur vollständigen Trennung der Schichten 18n, 20n genutzt werden, um das Körperpflegeprodukt 10n vom Papierwerkstoff für die Entsorgung materialseitig zu trennen. Damit kann der Papierwerkstoff in der Papier-Sammlung und die Anwendungseinheit 12n im Haushaltsmüll getrennt entsorgt werden.

Die Figur 15A zeigt eine Anwendungseinheit 12o eines Körperpflegeprodukts 10o in einer schematischen Darstellung. Das Körperpflegeprodukt 10o ist im vorliegenden Fall als eine Interdentalbürste ausgebildet.

Das Körperpflegeprodukt 10o weist die Anwendungseinheit 12o auf. Ferner weist das Körperpflegeprodukt 10o zumindest eine mit der Anwendungseinheit 12o verbundene Griffeinheit 14o auf.

Die Anwendungseinheit 12o ist an der Oberseite des Körperpflegeprodukts 10o angeordnet. Die Anwendungseinheit 12o bildet einen obersten Punkt des Körperpflegeprodukts 10o.

Die Griffeinheit 14o ist zu einem Greifen durch den Bediener vorgesehen. Die Griffeinheit 14o weist ferner einen materiellen Griffkörper 16o auf. Der materielle Griffkörper 16o bildet ein materielles Volumen der Griffeinheit 14o. Der materielle Griffkörper 16o weist eine flache, längliche Grundform auf. Der materielle Griffkörper 16o der Griffeinheit 14o besteht zumindest teilweise, insbesondere zumindest zu einem Großteil, aus einem Papierwerkstoff. Der materielle Griffkörper 16o besteht vollständig aus einem Papierwerkstoff. Der materielle Griffkörper 16o der Griffeinheit 14o weist zumindest zwei Schichten 18o, 20o aus einem Papierwerkstoff auf, die geschichtet miteinander verbunden sind. Der materielle Griffkörper 16o der Griffeinheit 14o weist beispielhaft genau zwei Schichten 18o, 20o aus einem Papierwerkstoff auf. Vorzugsweise sind von 2 bis 6, vorzugsweise von 2 bis 4, Schichten 18o, 20o vorgesehen. Bevorzugt sind die zumindest zwei Schichten 18o, 20o senkrecht zu einer Haupterstreckungsebene der Schichten 18o, 20o übereinander angeordnet und miteinander verbunden. Die Schichten 18o, 20o sind im Wesentlichen identisch ausgebildet und bilden insbesondere gegenüberliegenden Außenseite des Körperpflegeprodukts 10o. Die Schichten 18o, 20o sind beispielsweise mittels Kleben und/oder Pressen und/oder Schweißen und/oder Laminieren miteinander verbunden. Die Schichten 18o, 20o werden insbesondere bei einer Herstellung des Körperpflegeprodukts 10o miteinander verbunden.

Ferner weist die Anwendungseinheit 12o einen Reinigungselementträger 22o und mehrere mit dem Reinigungselementträger 22o verbundene, insbesondere eingedrehte, Reinigungselemente 24o auf. Der Reinigungselementträger 22o ist beispielhaft von einem Draht gebildet, wobei die Reinigungselemente 24 von in den Draht eingedrehten Borsten gebildet sind.

Der Reinigungselementträger 22o ist zu einer Verbindung mit der Griffeinheit 14o mit einem den Reinigungselementen 24o abgewandten Verbindungsabschnitt 26o mit dem materiellen Griffkörper 16o verbunden. Der Verbindungsabschnitt 26o ist insbesondere von einem den Reinigungselementen 24 abgewandten Drahtende gebildet. Der Verbindungsabschnitt 26o des Reinigungselementträgers 22o der Anwendungseinheit 12o ragt, insbesondere in einem hergestellten Zustand des Körperpflegeprodukts 10o, in den materiellen Griffkörper 16o und ist von diesem umschlossen. Die Anwendungseinheit 12o ist über den Verbindungsabschnitt 26o direkt mit dem materiellen Griffkörper 16o verbunden. Der Verbindungsabschnitt 26o ist zwischen den zumindest zwei Schichten 18o, 20o des materiellen Griffkörpers 16o an der Griffeinheit 14o angeordnet. Ferner ist der Reinigungselementträger 22o der Anwendungseinheit 12o zu einer teilweise formschlüssigen Verbindung mit der Griffeinheit 14o in dem Verbindungsabschnitt 26o nicht weiter sichtbar gebogen ausgebildet.

Ferner bildet die Griffeinheit 14o einen Standfuß 72o aus. Die zwei Schichten 18o, 20o sind dazu in einem unteren, der Anwendungseinheit 12o angewandten Abschnitt parallel zu einer Haupterstreckungsrichtung 34o geschlitzt ausgebildet. Die Griffeinheit 14o weist in dem unteren Abschnitt beispielhaft zwei Schlitze auf, welche sich durch beide Schichten 18o, 20o erstrecken. Anstelle der Schlitze wäre auch denkbar, dass eine Perforation vorgesehen ist, welche vom Endkunden zu einem Schlitz getrennt wird. Die Griffeinheit 14o bildet in dem unteren Abschnitt dadurch drei Fortsätze 76o auf. Der Standfuß 72o wird durch entgegengesetztes Abklappen der Fortsätze 76o erzeugt. Es ist insbesondere eine Faltkante 74o vorgesehen, an welcher die Fortsätze 76o in dem unteren Abschnitt umgeklappt bzw. auseinandergebogen werden können.

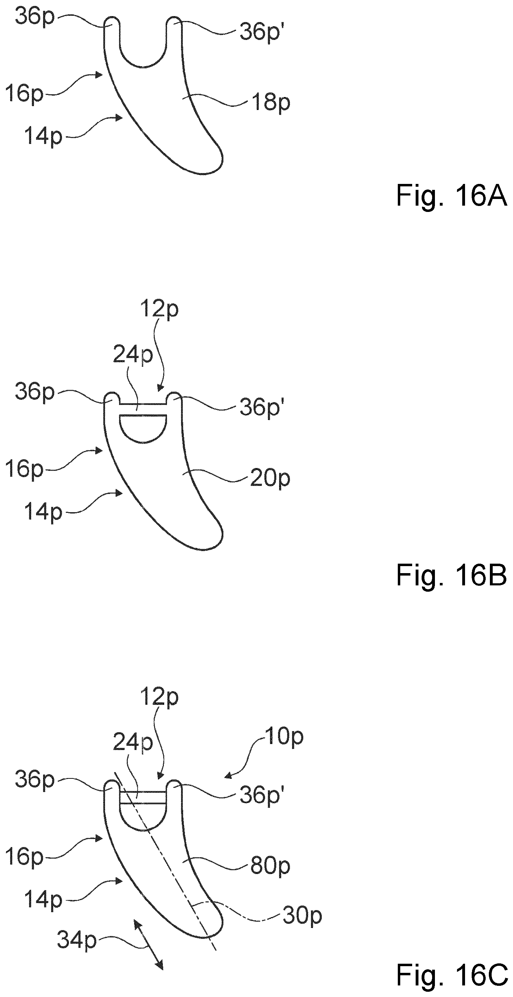

Die Figur 16A zeigt eine erste Schicht 18p einer Griffeinheit 14p eines als Flosser ausgebildeten Körperpflegeprodukts 10p bei einer Herstellung. Diese Ausführungsform ist nicht Teil der beanspruchten Erfindung. Das Körperpflegeprodukt 10p weist eine Anwendungseinheit 12p auf. Ferner weist das Körperpflegeprodukt 10p zumindest eine mit der Anwendungseinheit 12p verbundene Griffeinheit 14p auf.

Die Anwendungseinheit 12p ist an der Oberseite des Körperpflegeprodukts 10p angeordnet. Die Anwendungseinheit 12p bildet einen obersten Punkt des Körperpflegeprodukts 10p. Die Anwendungseinheit 12a besteht zumindest teilweise, insbesondere zumindest zu einem Großteil, aus einem Papierwerkstoff. Die Anwendungseinheit 12p weist ein einen Papierwerkstoff umfassendes Reinigungselement 24p auf. Das Reinigungselement 24p ist von einem Zahnzwischenraumreinigungspapier gebildet. Die Anwendungseinheit 12p besteht aus dem Reinigungselement 24p.

Die Griffeinheit 14p ist zu einem Greifen durch den Bediener vorgesehen. Die Griffeinheit 14p weist ferner einen materiellen Griffkörper 16p auf. Der materielle Griffkörper 16p bildet ein materielles Volumen der Griffeinheit 14p. Der materielle Griffkörper 16p weist eine gebogene, flache Grundform auf, welche sich zu der Anwendungseinheit 12p hin in zwei Arme 36p, 36p' aufteilt. Die Arme 36p, 36p' sind dazu vorgesehen, das Reinigungselement 24p beidseitig zu halten. Der materielle Griffkörper 16p der Griffeinheit 14p besteht zumindest teilweise, insbesondere zumindest zu einem Großteil, aus einem Papierwerkstoff. Der materielle Griffkörper 16p besteht vollständig aus einem Papierwerkstoff. Der materielle Griffkörper 16p der Griffeinheit 14p weist zumindest drei Schichten 18p, 20p, 80p aus einem Papierwerkstoff auf, die geschichtet miteinander verbunden sind. Der materielle Griffkörper 16p der Griffeinheit 14p weist beispielhaft genau drei Schichten 18p, 20p, 80p auf, welche zumindest teilweise aus einem Papierwerkstoff bestehen, wobei eine zweite Schicht 20p zusätzlich die Anwendungseinheit 12p ausbildet. Bevorzugt sind die zumindest drei Schichten 18p, 20p, 80p senkrecht zu einer Haupterstreckungsebene der Schichten 18p, 20p, 80p übereinander angeordnet und miteinander verbunden. Die erste und dritte Schicht 18p, 80p sind im Wesentlichen identisch ausgebildet und bilden insbesondere gegenüberliegende Außenseiten des Körperpflegeprodukts 10p. Die zweite Schicht 20p ist zwischen der ersten Schicht 18p und der dritten Schicht 80p angeordnet. Die Schichten 18p, 20p, 80p sind beispielsweise mittels Kleben und/oder Pressen und/oder Schweißen und/oder Laminieren miteinander verbunden. Die Schichten 18p, 20p, 80p werden insbesondere bei einer Herstellung des Körperpflegeprodukts 10p miteinander verbunden.

Die zweite Schicht 20p bildet zusätzlich die Anwendungseinheit 12p aus. Die zweite Schicht 20p besteht zumindest in einem Bereich der Anwendungseinheit 12p aus einer Folie. Vorzugsweise besteht die zweite Schicht 20p vollständig aus einer Folie.

Es wird insbesondere ein Verfahren zur Herstellung des Körperpflegeprodukts 10p offenbart. Bei dem Verfahren wird das Körperpflegeprodukt 10p aus den drei miteinander verbundenen Schichten 18p, 20p, 80p, welche zumindest teilweise aus einem Papierwerkstoff bestehen, geschichtet. Bei einem Herstellungsverfahren werden insbesondere Bögen aus Papierwerkstoff und/oder Folie, welche die Schichten 18p, 20p, 80p ausbilden, vorgestanzt, insbesondere vollständig gestanzt. Anschließend werden insbesondere die vorgestanzten Bögen, welche die Schichten 18p, 20p, 80p bilden, bereitgelegt und verbunden, wie beispielsweise durch Laminieren, Schweißen und/oder Kleben.

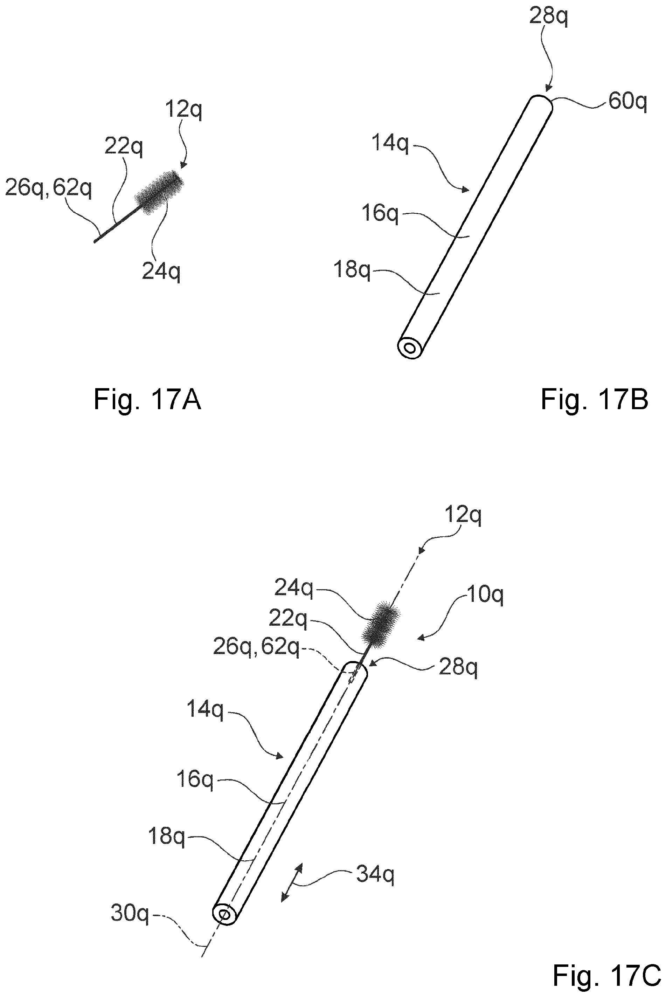

Die Figur 17A zeigt ein Körperpflegeprodukt 10q in einer schematischen perspektivischen Darstellung. Das Körperpflegeprodukt 10q ist im vorliegenden Fall als eine Interdentalbürste ausgebildet.

Das Körperpflegeprodukt 10q weist eine Anwendungseinheit 12q auf. Ferner weist das Körperpflegeprodukt 10q zumindest eine mit der Anwendungseinheit 12q verbundene Griffeinheit 14q auf.

Die Griffeinheit 14q ist zu einem Greifen durch den Bediener vorgesehen. Die Griffeinheit 14q weist eine zylindrische Grundform auf. Die Griffeinheit 14q weist ferner einen materiellen Griffkörper 16q auf. Der materielle Griffkörper 16q bildet ein materielles Volumen der Griffeinheit 14q. Der materielle Griffkörper 16q weist eine zylindrische Form auf. Der materielle Griffkörper 16q der Griffeinheit 14q besteht zumindest teilweise, insbesondere zumindest zu einem Großteil, aus einem Papierwerkstoff. Der materielle Griffkörper 16q besteht vollständig aus einem Papierwerkstoff. Der materielle Griffkörper 16q der Griffeinheit 14q weist zumindest eine Schicht 18q aus einem Papierwerkstoff auf, welche gerollt ausgebildet ist. Der materielle Griffkörper 16q weist beispielhaft genau eine Schicht 18q auf, es wäre jedoch auch ein mehrschichtiger Aufbau denkbar. Die Schicht 18q aus dem Papierwerkstoff ist um eine definierte Rollachse aufgerollt. Die Rollachse verläuft parallel, insbesondere koaxial, zu einer Längsachse 30q des Körperpflegeprodukts 10q. Die Schicht 18q wird bei einer Herstellung des materiellen Griffkörpers 16q aufgerollt. Die Schicht 18q bildet in einem Endzustand, insbesondere nach einer Herstellung, eine Rolle, insbesondere eine zylindrische Rolle, aus. Die Schicht 18q des materiellen Griffkörpers 16q ist in eine zylindrische, insbesondere hohlzylindrische, Form gerollt. Die Schicht 18q des materiellen Griffkörpers 16q ist mittels Kleben, Schweißen und/oder Laminieren in der Rolle mit sich selbst verbunden. Der Papierwerkstoff ist zu einer hohlzylindrischen Rolle gerollt. Der materielle Griffkörper 16q kann insbesondere gerade oder spiralförmig gerollt sein.

Ferner weist die Anwendungseinheit 12q einen Reinigungselementträger 22q und mehrere mit dem Reinigungselementträger 22q verbundene, insbesondere eingedrehte, Reinigungselemente 24q auf. Der Reinigungselementträger 22q ist beispielhaft von einem Draht gebildet, wobei die Reinigungselemente 24q von in den Draht eingedrehten Borsten gebildet sind. Es wäre jedoch auch eine andere, einem Fachmann als sinnvoll erscheinende Ausgestaltung des Reinigungselementträgers 22q und/oder der Reinigungselemente 24q denkbar. Der Reinigungselementträger 22q ist zu einer Verbindung mit der Griffeinheit 14q mit einem den Reinigungselementen 24q abgewandten Verbindungsabschnitt 26q mit dem materiellen Griffkörper 16q verbunden. Der Verbindungsabschnitt 26q ist insbesondere von einem den Reinigungselementen 24q abgewandten Drahtende gebildet.

Ferner weist das Körperpflegeprodukt 10q eine Steckverbindungseinheit 28q auf, welche zu einer lösbaren Steckverbindung der Anwendungseinheit 12q, welche insbesondere als eine Interdentalbürste ausgebildet ist, mit der Griffeinheit 14q vorgesehen ist. Die Steckverbindungseinheit 28q ist zumindest teilweise einstückig mit der Anwendungseinheit 12q und zumindest teilweise einstückig mit der Griffeinheit 14q ausgebildet. Die Steckverbindungseinheit 28q weist ein erstes Steckverbindungselement 60q auf, welches einstückig mit der Griffeinheit 14q verbunden ist, und ein zu dem ersten Steckverbindungselement 60q korrespondierendes zweites Steckverbindungselement 62q auf, welches fest mit der Anwendungseinheit 12q verbunden ist. Das zweite Steckverbindungselement 62q ist von dem Verbindungsabschnitt 26q gebildet. Das erste Steckverbindungselement 60q ist von einer zylindrischen Ausnehmung in dem materiellen Griffkörper 16q gebildet. Die Ausnehmung kann dabei bereits durch eine hohlzylindrische Herstellung des Griffkörpers 16q oder durch eine nachträgliche Bohrung hergestellt werden. Über die Steckverbindungseinheit 28q kann insbesondere die Anwendungseinheit 12q von dem materiellen Griffkörper 16q getrennt werden. Die Steckverbindungseinheit 28q ist insbesondere zu einer Bereitstellung einer lösbaren oder unlösbaren Rastverbindung vorgesehen.

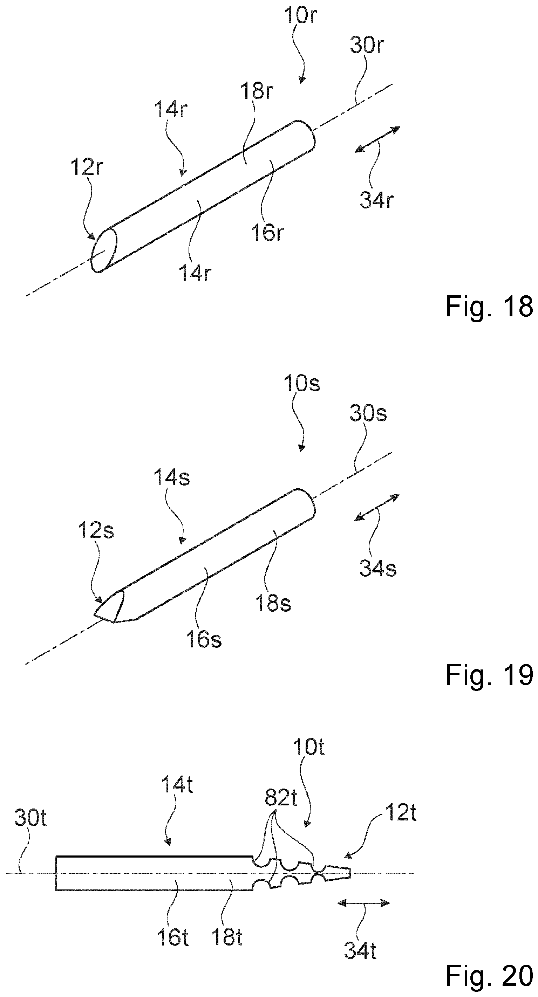

Die Figur 18 zeigt ein Körperpflegeprodukt 10r in einer schematischen perspektivischen Darstellung. Diese Ausführungsform ist nicht Teil der beanspruchten Erfindung. Das Körperpflegeprodukt 10r ist im vorliegenden Fall als ein Zahnstocher ausgebildet.

Das Körperpflegeprodukt 10r weist eine Anwendungseinheit 12r auf. Ferner weist das Körperpflegeprodukt 10r zumindest eine mit der Anwendungseinheit 12r verbundene Griffeinheit 14r auf.

Die Griffeinheit 14r ist zu einem Greifen durch den Bediener vorgesehen. Die Griffeinheit 14r weist eine zylindrische, insbesondere vollzylindrische, Grundform auf. Die Griffeinheit 14r weist ferner einen materiellen Griffkörper 16r auf. Der materielle Griffkörper 16r bildet ein materielles Volumen der Griffeinheit 14r. Der materielle Griffkörper 16r weist eine zylindrische Form auf. Der materielle Griffkörper 16r der Griffeinheit 14r besteht zumindest teilweise, insbesondere zumindest zu einem Großteil, aus einem Papierwerkstoff. Der materielle Griffkörper 16r besteht vollständig aus einem Papierwerkstoff. Der materielle Griffkörper 16r der Griffeinheit 14r weist zumindest eine Schicht 18r aus einem Papierwerkstoff auf, welche gerollt ausgebildet ist. Die Schicht 18r aus dem Papierwerkstoff ist um eine definierte Rollachse aufgerollt. Die Rollachse verläuft parallel, insbesondere koaxial, zu einer Längsachse 30r des Körperpflegeprodukts 10r. Die Schicht 18r wird bei einer Herstellung des materiellen Griffkörpers 16r aufgerollt. Die Schicht 18r bildet in einem Endzustand, insbesondere nach einer Herstellung, eine Rolle, insbesondere eine zylindrische Rolle, aus. Die Schicht 18r des materiellen Griffkörpers 16r ist in eine vollzylindrische Form gerollt. Die Schicht 18r des materiellen Griffkörpers 16r ist mittels Kleben, Schweißen und/oder Laminieren in der Rolle mit sich selbst verbunden. Der Papierwerkstoff ist zu einer vollzylindrischen Rolle gerollt. Der materielle Griffkörper 16r kann insbesondere gerade oder spiralförmig gerollt sein.

Die Anwendungseinheit 12r ist einstückig mit der Griffeinheit 14r ausgebildet. Die Anwendungseinheit 12r ist durch ein schräges Abschneiden eines Endes des materiellen Griffkörpers 16r realisiert.

Die Figur 19 zeigt ein Körperpflegeprodukt 10s in einer schematischen perspektivischen Darstellung. Diese Ausführungsform ist nicht Teil der beanspruchten Erfindung.

Das Körperpflegeprodukt 10s ist im vorliegenden Fall als ein Zahnstocher ausgebildet.

Das Körperpflegeprodukt 10s weist eine Anwendungseinheit 12s auf. Ferner weist das Körperpflegeprodukt 10s zumindest eine mit der Anwendungseinheit 12s verbundene Griffeinheit 14s auf.

Die Griffeinheit 14s ist zu einem Greifen durch den Bediener vorgesehen. Die Griffeinheit 14s weist eine zylindrische, insbesondere vollzylindrische, Grundform auf. Die Griffeinheit 14s weist ferner einen materiellen Griffkörper 16s auf. Der materielle Griffkörper 16s bildet ein materielles Volumen der Griffeinheit 14s. Der materielle Griffkörper 16s weist eine zylindrische Form auf. Der materielle Griffkörper 16s der Griffeinheit 14s besteht zumindest teilweise, insbesondere zumindest zu einem Großteil, aus einem Papierwerkstoff. Der materielle Griffkörper 16s besteht vollständig aus einem Papierwerkstoff. Der materielle Griffkörper 16s der Griffeinheit 14s weist zumindest eine Schicht 18s aus einem Papierwerkstoff auf, welche gerollt ausgebildet ist. Die Schicht 18s aus dem Papierwerkstoff ist um eine definierte Rollachse aufgerollt. Die Rollachse verläuft parallel, insbesondere koaxial, zu einer Längsachse 30s des Körperpflegeprodukts 10s. Die Schicht 18s wird bei einer Herstellung des materiellen Griffkörpers 16s aufgerollt. Die Schicht 18s bildet in einem Endzustand, insbesondere nach einer Herstellung, eine Rolle, insbesondere eine zylindrische Rolle, aus. Die Schicht 18s des materiellen Griffkörpers 16s ist in eine vollzylindrische Form gerollt. Die Schicht 18s des materiellen Griffkörpers 16s ist mittels Kleben, Schweißen und/oder Laminieren in der Rolle mit sich selbst verbunden. Der Papierwerkstoff ist zu einer vollzylindrischen Rolle gerollt. Der materielle Griffkörper 16s kann insbesondere gerade oder spiralförmig gerollt sein.

Die Anwendungseinheit 12s ist einstückig mit der Griffeinheit 14s ausgebildet. Die Anwendungseinheit 12s ist durch ein von zwei Seiten schräges Abschneiden eines Endes des materiellen Griffkörpers 16s realisiert. Ein Ende des materiellen Griffkörpers 16s, welches die Anwendungseinheit 12s ausbildet, weist zumindest teilweise eine Satteldachform auf.

Die Figur 20 zeigt ein Körperpflegeprodukt 10t in einer schematischen perspektivischen Darstellung. Diese Ausführungsform ist nicht Teil der beanspruchten Erfindung. Das Körperpflegeprodukt 10t ist im vorliegenden Fall als ein Zahnstocher ausgebildet.

Das Körperpflegeprodukt 10t weist eine Anwendungseinheit 12t auf. Ferner weist das Körperpflegeprodukt 10t zumindest eine mit der Anwendungseinheit 12t verbundene Griffeinheit 14t auf.

Die Griffeinheit 14t ist zu einem Greifen durch den Bediener vorgesehen. Die Griffeinheit 14t weist eine zylindrische, insbesondere vollzylindrische, Grundform auf. Die Griffeinheit 14t weist ferner einen materiellen Griffkörper 16t auf. Der materielle Griffkörper 16t bildet ein materielles Volumen der Griffeinheit 14t. Der materielle Griffkörper 16t weist eine zylindrische Form auf. Der materielle Griffkörper 16t der Griffeinheit 14t besteht zumindest teilweise, insbesondere zumindest zu einem Großteil, aus einem Papierwerkstoff. Der materielle Griffkörper 16t besteht vollständig aus einem Papierwerkstoff. Der materielle Griffkörper 16t der Griffeinheit 14t weist zumindest eine Schicht 18t aus einem Papierwerkstoff auf, welche gerollt ausgebildet ist. Die Schicht 18t aus dem Papierwerkstoff ist um eine definierte Rollachse aufgerollt. Die Rollachse verläuft parallel, insbesondere koaxial, zu einer Längsachse 30t des Körperpflegeprodukts 10t. Die Schicht 18t wird bei einer Herstellung des materiellen Griffkörpers 16t aufgerollt. Die Schicht 18t bildet in einem Endzustand, insbesondere nach einer Herstellung, eine Rolle, insbesondere eine zylindrische Rolle, aus. Die Schicht 18t des materiellen Griffkörpers 16t ist in eine vollzylindrische Form gerollt. Die Schicht 18t des materiellen Griffkörpers 16t ist mittels Kleben, Schweißen und/oder Laminieren in der Rolle mit sich selbst verbunden. Der Papierwerkstoff ist zu einer vollzylindrischen Rolle gerollt. Der materielle Griffkörper 16t kann insbesondere gerade oder spiralförmig gerollt sein.

Die Anwendungseinheit 12t ist einstückig mit der Griffeinheit 14t ausgebildet. Die Anwendungseinheit 12t ist durch ein von zwei Seiten schräges Abschneiden eines Endes des materiellen Griffkörpers 16t realisiert. Ein Ende des materiellen Griffkörpers 16t, welches die Anwendungseinheit 12t ausbildet, weist zumindest teilweise eine Satteldachform auf. Zusätzlich sind in dem Bereich der Anwendungseinheit 12t Lochungen 82t vorgesehen, welche eine Reinigung unterstützen sollen.

Die gemachten Beschreibungen für spezifische Figuren lassen sich selbstverständlich auch auf andere Figuren übertragen, die gleiche oder ähnliche Ausprägungen zeigen und in welchen die Ausprägungen nicht im gleichen Detail beschrieben sind.

### Bezugszeichen

- 10: Körperpflegeprodukt
- 12: Anwendungseinheit
- 14: Griffeinheit
- 16: Griffkörper
- 18: Schicht
- 20: Schicht
- 22: Reinigungselementträger
- 24: Reinigungselement
- 26: Verbindungsabschnitt
- 28: Steckverbindungseinheit
- 30: Längsachse
- 34: Haupterstreckungsrichtung
- 36: Arm
- 36': Arm
- 38: Faltachse
- 40: Faltkante
- 42: Teilschicht
- 42': Teilschicht
- 44: Spannungslasche
- 44': Spannungslasche
- 46: Faden
- 48: Kopfbereich
- 50: Halsbereich
- 52: Grundkörper
- 54: Teilelement
- 54': Teilelement
- 56: Teilelement
- 56': Teilelement
- 56": Teilelement
- 56‴: Teilelement
- 58: Formschlussausnehmung
- 58': Formschlussausnehmung
- 60: Steckverbindungselement
- 62: Steckverbindungselement
- 64: Kernfortsatz
- 66: Verformung
- 68: Teilschicht
- 68': Teilschicht
- 68": Teilschicht
- 70: Bedruckung
- 72: Standfuß
- 74: Faltkante
- 76: Fortsatz
- 78: Pfeil
- 80: Schicht
- 82: Lochung

## Patentansprüche

1. Mundhygienemittel mit zumindest einer Anwendungseinheit (12e; 12n; 12o) und mit zumindest einer mit der Anwendungseinheit (12e; 12n; 12o) verbundenen Griffeinheit (14e; 14n; 14o), welche zumindest einen materiellen Griffkörper (16e; 16n; 16o) aufweist,
wobei
der materielle Griffkörper (16e; 16n; 16o) der Griffeinheit (14e; 14n; 14o) oder der materielle Griffkörper (16e; 16n; 16o) der Griffeinheit (14e; 14n; 14o) und die Anwendungseinheit (12e; 12n; 12o) zumindest teilweise, insbesondere zumindest zu einem Großteil, aus einem Papierwerkstoff besteht,
**dadurch gekennzeichnet, dass**
die Anwendungseinheit (12e; 12n; 12o) zumindest einen, von einem Draht gebildeten Reinigungselementträger (22e; 22n; 22o), und mehrere mit dem Reinigungselementträger (22e; 22n; 22o) verbundene, eingedrehte, Reinigungselemente (24e; 24n; 24o) aufweist, wobei der Reinigungselementträger (22e; 22n; 22o) zu einer Verbindung mit der Griffeinheit (14e; 14n; 14o) mit einem den Reinigungselementen (24e; 24n; 24o) abgewandten Verbindungsabschnitt (26e; 26n; 26o) mit dem materiellen Griffkörper (16e; 16n; 16o) verbunden ist, wobei der zumindest eine Reinigungselementträger (22e; 22n; 22o) der Anwendungseinheit (12e; 12n; 12o) zu einer zumindest teilweise formschlüssigen Verbindung mit der Griffeinheit (14e; 14n; 14o) in dem Verbindungsabschnitt (26e; 26n; 26o) gebogen ausgebildet ist.

2. Mundhygienemittel nach Anspruch 1,
**dadurch gekennzeichnet, dass**
der materielle Griffkörper der Griffeinheit zumindest eine Schicht aus einem Papierwerkstoff aufweist, welche gerollt ausgebildet ist.

3. Mundhygienemittel nach Anspruch 2,
**dadurch gekennzeichnet, dass**
die zumindest eine Schicht des materiellen Griffkörpers in eine zylindrische Form gerollt ist.

4. Mundhygienemittel zumindest nach Anspruch 1,
**dadurch gekennzeichnet, dass**
der materielle Griffkörper (16e; 16n; 16o) der Griffeinheit (14e; 14n; 14o) zumindest zwei Schichten (18e, 20e; 18n, 20n; 18o, 20o) aus einem Papierwerkstoff aufweist, die geschichtet miteinander verbunden sind.

5. Mundhygienemittel zumindest nach Anspruch 1,
**dadurch gekennzeichnet, dass**
der materielle Griffkörper (16n; 16o) der Griffeinheit (14n; 14o) zumindest eine Schicht (18n; 18o) aus einem Papierwerkstoff aufweist, die zumindest teilweise gefaltet ausgebildet ist.

6. Mundhygienemittel nach Anspruch 1,
**dadurch gekennzeichnet, dass**
der Verbindungsabschnitt (26e; 26n; 26o) des Reinigungselementträgers (22e; 22n; 22o) der Anwendungseinheit (12e; 12n; 12o) in den materiellen Griffkörper (16e; 16n; 16o) ragt und von diesem umschlossen ist.

7. Mundhygienemittel nach Anspruch 1,
**dadurch gekennzeichnet, dass**
der zumindest eine Reinigungselementträger der Anwendungseinheit an einem den Reinigungselementen abgewandten Verbindungsabschnitt in zumindest eine Schicht des materiellen Griffkörpers eingerollt ausgebildet ist.

8. Mundhygienemittel zumindest nach Anspruch 1,
**dadurch gekennzeichnet, dass**
der zumindest eine Reinigungselementträger (22e; 22n; 22o) der Anwendungseinheit (12e; 12n; 12o) zumindest teilweise stoffschlüssig, insbesondere mittels Klebung und/oder Verschweißung, mit dem materiellen Griffkörper (16e; 16n; 16o) verbunden ist.

9. Mundhygienemittel nach einem der vorhergehenden Ansprüche,
**dadurch gekennzeichnet, dass**
die Anwendungseinheit zumindest ein einen Papierwerkstoff umfassendes Reinigungselement aufweist.

10. Mundhygienemittel nach Anspruch 8,
**dadurch gekennzeichnet, dass**
das zumindest eine Reinigungselement zumindest teilweise von einem Zahnzwischenraumreinigungspapier gebildet ist.

11. Mundhygienemittel zumindest nach Anspruch 5,
**dadurch gekennzeichnet, dass**
die Anwendungseinheit (12e; 12n; 12o) zwischen den zumindest zwei Schichten (18e, 20e; 20n; 20o) des materiellen Griffkörpers (16e; 16n; 16o) an der Griffeinheit (14e; 14n; 14o) angeordnet ist.

12. Mundhygienemittel nach einem der vorhergehenden Ansprüche,
**gekennzeichnet durch**
eine Steckverbindungseinheit welche zu einer lösbaren Steckverbindung der Anwendungseinheit, welche insbesondere als ein Zahnbürstenkopf ausgebildet ist, mit der Griffeinheit vorgesehen ist.

13. Mundhygienemittel nach einem der vorhergehenden Ansprüche,
**dadurch gekennzeichnet, dass**
der Papierwerkstoff eine wasserabweisende Beschichtung und/oder Imprägnierung aufweist.

14. Mundhygienemittel nach einem der vorhergehenden Ansprüche,
**dadurch gekennzeichnet, dass**
der Papierwerkstoff lackiert und/oder bedruckt ausgebildet ist.

15. Verfahren zur Herstellung eines Mundhygienemittels (10e; 10n; 10o) nach einem der vorhergehenden Ansprüche.

16. Verfahren nach Anspruch 15,
**dadurch gekennzeichnet, dass**
der materielle Griffkörper der Griffeinheit auf zumindest eine Schicht aus einem Papierwerkstoff gerollt wird.

17. Verfahren nach Anspruch 16,
**dadurch gekennzeichnet, dass**
die Anwendungseinheit in zumindest einem Verbindungsabschnitt in den materiellen Griffkörper eingerollt wird.

18. Verfahren zumindest nach Anspruch 15,
**dadurch gekennzeichnet, dass**
der materielle Griffkörper (16e) der Griffeinheit (14e) aus zumindest zwei miteinander verbundenen Schichten (18e, 20e) aus einem Papierwerkstoff geschichtet wird.

19. Verfahren nach Anspruch 18,
**dadurch gekennzeichnet, dass**
die Anwendungseinheit (12e) in zumindest einem Verbindungsabschnitt (26e) der zumindest zwei Schichten (18e, 20e) in dem materiellen Griffkörper (16e) eingeklebt und/oder einlaminiert wird.

## Claims

1. Oral hygiene product
with at least one application unit (12e; 12n; 12o)
and with at least one handle unit (14e; 14n; 14o) that is connected with the application unit (12e; 12n; 12o) and comprises at least one material handle body (16e; 16n; 16o),
wherein
the material handle body (16e; 16n; 16o) of the handle unit (14e; 14n; 14o) or the material handle body (16e; 16n; 16o) of the handle unit (14e; 14n; 14o) and the application unit (12e; 12n; 12o) is made at least partially, in particular at least to a large extent, of a paper material,
**characterized in that**
the application unit (12e; 12n; 12o) comprises at least one cleaning element carrier (22e; 22n; 22o), which is realized as a wire, and comprises several cleaning elements (24e; 24n; 24o) which are connected with the cleaning element carrier 22e; 22n; 22o) and twisted-in,
wherein for a connection to the handle unit (14e; 14n; 14o), the cleaning element carrier (22e; 22n; 22o) is connected to the material handle body (16e; 16n; 16o) via a connection section (26e; 26n; 26o) that faces away from the cleaning elements (24e; 24n; 24o),
wherein for an at least partially form-fit connection to the handle unit (14e; 14n; 14o), the at least one cleaning element carrier (22e; 22n; 22o) of the application unit (12e; 12n; 12o) is realized so as to be bent in the connection section (26e; 26n; 26o).

2. Oral hygiene product according to claim 1,
**characterized in that**
the material handle body of the handle unit comprises at least one layer of a paper material, which is realized in a rolled-up fashion.

3. Oral hygiene product according to claim 2,
**characterized in that**
the at least one layer of the material handle body is rolled in a cylindrical shape.

4. Oral hygiene product at least according to claim 1,
**characterized in that**
the material handle body (16e; 16n; 16o) of the handle unit (14e; 14n; 14o) comprises at least two layers (18e, 20e; 18n, 20n; 18o, 20o) of a paper material, which are connected to one another in a layered fashion.

5. Oral hygiene product at least according to claim 1,
**characterized in that**
the material handle body (16n; 16o) of the handle unit (14n; 14o) comprises at least one layer (18n; 18o) of a paper material, which is realized so as to be at least partially folded.

6. Oral hygiene product according to claim 1,
**characterized in that**
the connection section (26e; 26n; 26o) of the cleaning element carrier (22e; 22n; 22o) of the application unit (12e; 12n; 12o) projects into the material handle body (16e; 16n; 16o) and is enclosed by the material handle body (16e; 16n; 16o).

7. Oral hygiene product according to claim 1,
**characterized in that**
the at least one cleaning element carrier of the application unit is implemented so as to be rolled into at least one layer of the material handle body in a connection section that faces away from the cleaning elements.

8. Oral hygiene product at least according to claim 1,
**characterized in that**
the at least one cleaning element carrier (22e; 22n; 22o) of the application unit (12e; 12n; 12o) is connected to the material handle body (16e; 16n; 16o) at least partially by substance-to-substance bond, in particular by gluing and/or welding.

9. Oral hygiene product according to any one of the preceding claims, **characterized in that**
the application unit has at least one cleaning element comprising a paper material.

10. Oral hygiene product according to claim 8,
**characterized in that**
the at least one cleaning element is embodied at least partially as an interdental cleaning paper.

11. Oral hygiene product at least according to claim 5,
**characterized in that**
the application unit (12e; 12n; 12o) is arranged on the handle unit (14e; 14n; 14o) between the at least two layers (18e, 20e; 20n; 20o) of the material handle body (16e; 16n; 16o).

12. Oral hygiene product according to any one of the preceding claims, **characterized by**
a plug connection unit configured for a releasable plug connection of the application unit, which is in particular embodied as a toothbrush head, to the handle unit.

13. Oral hygiene product according to any one of the preceding claims, **characterized in that**
the paper material has a water-repellent coating and/or impregnation.

14. Oral hygiene product according to any one of the preceding claims, **characterized in that**
the paper material is realized so as to be varnished and/or printed.

15. Method for producing an oral hygiene product (10e; 10n; 10o) according to any one of the preceding claims.

16. Method according to claim 15,
**characterized in that**
the material handle body of the handle unit is rolled onto at least one layer of a paper material.

17. Method according to claim 16,
**characterized in that**
the application unit is in at least one connection section rolled into the material handle body.

18. Method at least according to claim 15,
**characterized in that**
the material handle body (16e) of the handle unit (14e) is layered from at least two interconnected layers (18e, 20e) of a paper material.

19. Method according to claim 18,
**characterized in that**
the application unit (12e) is in at least one connection section (26e) of the at least two layers (18e, 20e) glued and/or laminated into the material handle body (16e).

## Revendications

1. Produit d'hygiène buccale
avec au moins une unité d'application (12e ; 12n ; 12o)
et avec au moins une unité de poignée (14e ; 14n ; 14o) reliée à l'unité d'application (12e ; 12n ; 12o) et comprenant au moins un corps de poignée matériel (16e ; 16n ; 16o),
où
le corps de poignée matériel (16e ; 16n ; 16o) de l'unité de poignée (14e ; 14n ; 14o) ou le corps de poignée matériel (16e ; 16n ; 16o) de l'unité de poignée (14e ; 14n ; 14o) et l'unité d'application (12e ; 12n ; 12o) est constitué au moins partiellement, en particulier au moins en grande partie, d'un matériau papier, **caractérisé en ce que**
l'unité d'application (12e ; 12n ; 12o) comprend au moins un porteur d'éléments à nettoyage (22e ; 22n ; 22o) formé d'un fil métallique, et comprend plusieurs éléments à nettoyage (24e ; 24n ; 24o) qui sont reliés au porteur d'éléments à nettoyage (22e ; 22n ; 22o) et sont tordus là-dedans,
où pour une liaison avec l'unité de poignée (14e ; 14n ; 14o), le porteur d'éléments à nettoyage (22e ; 22n ; 22o) est relié au corps de poignée matériel (16e ; 16n ; 16o) moyennant une section de liaison (26e ; 26n ; 26o) détournée des éléments de nettoyage (24e ; 24n ; 24o),
où pour une liaison au moins partiellement par forme avec l'unité de poignée (14e ; 14n ; 14o), l'au moins un porteur d'éléments à nettoyage (22e ; 22n ; 22o) de l'unité d'application (12e ; 12n ; 12o) est réalisé en forme courbée dans la section de liaison (26e ; 26n ; 26o) .

2. Produit d'hygiène buccale selon la revendication 1,
**caractérisé en ce que**
le corps de poignée matériel de l'unité de poignée comprend au moins une couche d'un matériau papier, qui est réalisée de manière enroulée.

3. Produit d'hygiène buccale selon la revendication 2,
**caractérisé en ce que**
l'au moins une couche du corps de poignée matériel est enroulée en forme cylindrique.

4. Produit d'hygiène buccale au moins selon la revendication 1, **caractérisé en ce que**
le corps de poignée matériel (16e ; 16n ; 16o) de l'unité de poignée (14e ; 14n ; 14o) comprend au moins deux couches (18e, 20e ; 18n, 20n ; 18o, 20o) d'un matériau papier, qui sont reliées l'une à l'autre en couches.

5. Produit d'hygiène buccale au moins selon la revendication 1, **caractérisé en ce que**
le corps de poignée matériel (16n ; 16o) de l'unité de poignée (14n ; 14o) comprend au moins une couche (18n ; 18o) d'un matériau papier, qui est réalisée de manière au moins partiellement pliée.

6. Produit d'hygiène buccale selon la revendication 1,
**caractérisé en ce que**
la section de liaison (26e ; 26n ; 26o) du porteur d'éléments à nettoyage (22e ; 22n ; 22o) de l'unité d'application (12e ; 12n ; 12o) fait saillie dans le corps de poignée matériel (16e ; 16n ; 16o) et est entourée par celui-ci.

7. Produit d'hygiène buccale selon la revendication 1,
**caractérisé en ce que**
l'au moins un porteur d'éléments à nettoyage de l'unité d'application est réalisé de manière enroulée dans au moins une couche du corps de poignée matériel sur une section de liaison détournée des éléments à nettoyage.

8. Produit d'hygiène buccale au moins selon la revendication 1, **caractérisé en ce que**
l'au moins un porteur d'éléments à nettoyage (22e ; 22n ; 22o) de l'unité d'application (12e ; 12n ; 12o) est relié au corps de poignée matériel (16e ; 16n ; 16o) au moins partiellement par liaison matérielle, en particulier par collage et/ou soudage.

9. Produit d'hygiène buccale selon l'une quelconque des revendications précédentes,
**caractérisé en ce que**
l'unité d'application inclut au moins un élément à nettoyage comprenant un matériau papier.

10. Produit d'hygiène buccale selon la revendication 8,
**caractérisé en ce que**
l'au moins un élément à nettoyage est formé au moins partiellement d'un papier à nettoyage d'espace interdentaire.

11. Produit d'hygiène buccale au moins selon la revendication 5,
**caractérisé en ce que**
l'unité d'application (12e ; 12n ; 12o) est disposée sur l'unité de poignée (14e ; 14n ; 14o) entre les au moins deux couches (18e, 20e ; 20n ; 20o) du corps de poignée matériel (16e ; 16n ; 16o).

12. Produit d'hygiène buccale selon l'une quelconque des revendications précédentes,
**caractérisé par**
une unité de liaison par enfichage prévue pour une liaison par enfichage séparable de l'unité d'application, laquelle est réalisée en particulier comme tête de brosse à dents, avec l'unité de poignée.

13. Produit d'hygiène buccale selon l'une quelconque des revendications précédentes,
**caractérisé en ce que**
le matériau papier comprend un revêtement et/ou une imprégnation hydrofuge.

14. Produit d'hygiène buccale selon l'une quelconque des revendications précédentes,
**caractérisé en ce que**
le matériau papier est réalisé de manière vernie et/ou imprimée.

15. Procédé de fabrication d'un produit d'hygiène buccale (10e ; 10n ; 10o) selon l'une quelconque des revendications précédentes.

16. Procédé selon la revendication 15,
**caractérisé en ce que**
le corps de poignée matériel de l'unité de poignée est enroulé sur au moins une couche d'un matériau papier.

17. Procédé selon la revendication 16,
**caractérisé en ce que**
l'unité d'application est enroulée dans le corps de poignée matériel dans au moins une section de liaison.

18. Procédé au moins selon la revendication 15,
**caractérisé en ce que**
le corps de poignée matériel (16e) de l'unité de poignée (14e) est gerbé d'au moins deux couches (18e, 20e) d'un matériau papier, qui sont reliées l'une à l'autre.

19. Procédé selon la revendication 18,
**caractérisé en ce que**
l'unité d'application (12e) est collée et/ou laminée dans le corps de poignée matériel (16e) dans au moins une section de liaison (26e) des au moins deux couches (18e, 20e).
